# EUROPEAN PATENT APPLICATION

(11) **EP 1 605 044 A2**
(43) Date of publication of application: **14.12.2005**
(21) Application number: 04715524.7
(22) Date of filing: 27.02.2004
(51) Int. Cl.: C12N 15/11, C12N 15/00, C12N 5/10, C12Q 1/68, G01N 33/53

(54) **MARKER FOR DETECTING MESENCHYMAL STEM CELL AND METHOD OF DISTINGUISHING MESENCHYMAL STEM CELL USING THE MARKER**

(30) Priority: 10.03.2003 JP 2003063077
(71) Applicant: Japan Science and Technology Agency, Kawaguchi-shi, Saitama 332-0012 (JP); Kato, Yukio, Hiroshima-shi 732-0062 (JP); Two Cells Co. Ltd, Hiroshima-shi, Hiroshioma 732-0811 (JP)
(72) Inventor: KATO, Yukio, Hiroshima-shi, Hiroshima 732-0062 (JP); TSUJI, Koichiro, Hiroshima-shi, Hiroshima 7320811 (JP); KOIKE, Chika, Hiroshima-shi, Hiroshima 734-0001 (JP)
(74) Representative: Harding, Charles Thomas
(86) International application number: PCT/JP2004/002457
(87) International publication number: WO 2004/081174

(57) **Abstract**

The present invention provides a marker for detecting, separating/distinguishing a mesenchymal stem cell and a method for detecting, separating/distinguishing a mesenchymal stem cell by using the marker. A gene shown in the sequence listing is expressed specifically in a mesenchymal stem cell. The present invention comprises use of the gene as a marker for detecting mesenchymal stem cells. In addition, the present invention comprises a probe for detecting the mesenchymal stem cell marker gene and a PCR primer for amplifying the gene in a test cell in detecting the mesenchymal stem cell gene marker, and further comprises a polypeptide marker for detecting mesenchymal stem cells comprising a polypeptide wherein the mesenchymal stem cell marker gene of the present invention is expressed, an antibody for detecting the polypeptide marker which specifically binds to the polypeptide marker, and still further, a method for distinguishing and separating a mesenchymal stem cell by using a probe for detecting the mesenchymal stem cell marker gene, and an antibody which specifically binds to a polypeptide marker.

SEQUENCE LISTING

<110> Japan Science and Technology Agency
Yukio KATO
Two Cells Co., Ltd.

<120> Markers for mesenchymal stem cells and the method to identify

<130> V320P006US(PCT)

<150> JP2003-63077
<151> 2000-03-10

<160> 116

<170> PatentIn version 3.1

<210> 1
<211> 652
<212> DNA
<213> Homo sapiens

<400> 1
gaattgccaa ttccttgttt gtgcaaaatg gatttcatgt caatgaggag tttttgcaaa 60

tgatgaaaaa atattttaat gcagcagtaa atcatgtgga cttcagtcaa aatgtagccg 120

tggccaacta catcaataag tgggtggaga ataacacaaa caatctggtg aaagatttgg 180

tatccccaag ggattttgat gctgccactt atctggccct cattaatgct gtctatttca 240

aggggaactg gaagtcgcag tttaggcctg aaaatactag aaccttttct ttcactaaag 300

atgatgaaag tgaagtccaa attccaatga tgtatcagca aggagaattt tattatgggg 360

aatttagtga tggctccaat gaagctggtg gtatctacca agtcctagaa ataccatatg 420

aaggagatga aataagcatg atgctggtgc tgtccagaca ggaagttcct cttgctactc 480

tggagccatt agtcaaagca cagctggttg aagaatgggc aaactctgtg aagaagcaaa 540

aagtagaagt atacctgccc aggttcacag tggaacagga aattgattta aaagatgttt 600

tgaaggctct tggaataact gaaaattttc atcaaagatg caaattttga ca 652

<210> 2
<211> 1450
<212> DNA
<213> Homo sapiens

<400> 2

ctggatagaa cagctcaagc cttgccactt cgggcttctc actgcagctg ggcttggact 60

tcggagtttt gccattgcca gtgggacgtc tgagactttc tccttcaagt acttggcaga 120

tcactctctt agcagggtct gcgcttcgca gccgggatga agctggtttc cgtcgccctg 180

atgtacctgg gttcgctcgc cttcctaggc gctgacaccg ctcggttgga tgtcgcgtcg 240

gagtttcgaa agaagtggaa taagtgggct ctgagtcgtg ggaagaggga actgcggatg 300

tccagcagct accccaccgg gctcgctgac gtgaaggccg ggcctgccca gacccttatt 360

cggccccagg acatgaaggg tgcctctcga agccccgaag acagcagtcc ggatgccgcc 420

cgcatccgag tcaagcgcta ccgccagagc atgaacaact tccagggcct ccggagcttt 480

ggctgccgct tcgggacgtg cacggtgcag aagctggcac accagatcta ccagttcaca 540

gataaggaca aggacaacgt cgcccccagg agcaagatca gcccccaggg ctacggccgc 600

cggcgccggc gctccctgcc cgaggccggc ccgggtcgga ctctggtgtc ttctaagcca 660

caagcacacg gggctccagc ccccccgagt ggaagtgctc cccactttct ttaggattta 720

ggcgcccatg gtacaaggaa tagtcgcgca agcatcccgc tggtgcctcc cgggacgaag 780

gacttcccga gcggtgtggg gaccgggctc tgacagccct gcggagaccc tgagtccggg 840

aggcaccgtc cggcggcgag ctctggcttt gcaagggccc ctccttctgg gggcttcgct 900

tccttagcct tgctcaggtg caagtgcccc agggggcggg gtgcagaaga atccgagtgt 960

ttgccaggct taaggagagg agaaactgag aaatgaatgc tgagaccccc ggagcagggg 1020

tctgagccac agccgtgctc gcccacaaac tgatttctca cggcgtgtca ccccaccagg 1080

gcgcaagcct cactattact tgaactttcc aaaacctaaa gaggaaaagt gcaatgcgtg 1140

ttgtacatac agaggtaact atcaatattt aagtttgttg ctgtcaagat tttttttgta 1200

acttcaaata tagagatatt tttgtacgtt atatattgta ttaagggcat tttaaaagca 1260

attatattgt cctcccccta ttttaagacg tgaatgtctc agcgaggtgt aaagttgttc 1320

gccgcgtgga atgtgagtgt gtttgtgtgc atgaaagaga aagactgatt acctcctgtg 1380

tggaagaagg aaacaccgag tctctgtata atctatttac ataaaatggg tgatatgcga 1440

acagcaaacc 1450

<210> 3

<211> 185
<212> PRT
<213> Homo sapiens

<400> 3
Met Lys Leu Val Ser Val Ala Leu Met Tyr Leu Gly Ser Leu Ala Phe
1               5                   10                  15
Leu Gly Ala Asp Thr Ala Arg Leu Asp Val Ala Ser Glu Phe Arg Lys
            20                  25                  30
Lys Trp Asn Lys Trp Ala Leu Ser Arg Gly Lys Arg Glu Leu Arg Met
        35                  40                  45
Ser Ser Ser Tyr Pro Thr Gly Leu Ala Asp Val Lys Ala Gly Pro Ala
    50                  55                  60
Gln Thr Leu Ile Arg Pro Gln Asp Met Lys Gly Ala Ser Arg Ser Pro
65                  70                  75                  80
Glu Asp Ser Ser Pro Asp Ala Ala Arg Ile Arg Val Lys Arg Tyr Arg
                85                  90                  95
Gln Ser Met Asn Asn Phe Gln Gly Leu Arg Ser Phe Gly Cys Arg Phe
            100                 105                 110
Gly Thr Cys Thr Val Gln Lys Leu Ala His Gln Ile Tyr Gln Phe Thr
        115                 120                 125
Asp Lys Asp Lys Asp Asn Val Ala Pro Arg Ser Lys Ile Ser Pro Gln
    130                 135                 140
Gly Tyr Gly Arg Arg Arg Arg Arg Ser Leu Pro Glu Ala Gly Pro Gly
145                 150                 155                 160
Arg Thr Leu Val Ser Ser Lys Pro Gln Ala His Gly Ala Pro Ala Pro
                165                 170                 175
Pro Ser Gly Ser Ala Pro His Phe Leu
            180                 185


<210> 4
<211> 809
<212> DNA
<213> Homo sapiens

<400> 4
atgcctgtct tcatcttgaa agaaaagctc caggtccctt ctccagccac ccagccccaa     60

gatggtgatg ctgctgctgc tgctttccgc actggctggc ctcttcggtg cggcagaggg    120

acaagcattt catcttggga agtgccccaa tcctccggtg caggagaatt ttgacgtgaa    180

taagtatctc ggaagatggt acgaaattga gaagatccca acaacctttg agaatggacg    240

ctgcatccag gccaactact cactaatgga aaacggaaag atcaaagtgt taaaccagga    300

gttgagagct gatggaactg tgaatcaaat cgaaggtgaa gccaccccag ttaacctcac    360

agagcctgcc aagctggaag ttaagttttc ctggtttatg ccatcggcac cgtactggat    420

cctggccacc gactatgaga actatgccct cgtgtattcc tgtacctgca tcatccaact    480

ttttcacgtg gattttgctt ggatcttggc aagaaaccct aatctccctc cagaaacagt 540

ggactctcta aaaaatatcc tgacttctaa taacattgat gtcaagaaaa tgacggtcac 600

agaccaggtg aactgcccca agctctcgta accaggttct acagggaggc tgcacccact 660

ccatgttact tctgcttcgc tttcccctac cccacccccc cccataaaga caaaccaatc 720

aaccacgaca aaggaagttg acctaaacat gtaaccatgc cctaccctgt taccttgcta 780

gctgcaaaat aaacttgttg ctgacctgc 809

<210> 5
<211> 189
<212> PRT
<213> Homo sapiens

<400> 5
Met Val Met Leu Leu Leu Leu Leu Ser Ala Leu Ala Gly Leu Phe Gly
1               5                   10                  15
Ala Ala Glu Gly Gln Ala Phe His Leu Gly Lys Cys Pro Asn Pro Pro
            20                  25                  30
Val Gln Glu Asn Phe Asp Val Asn Lys Tyr Leu Gly Arg Trp Tyr Glu
        35                  40                  45
Ile Glu Lys Ile Pro Thr Thr Phe Glu Asn Gly Arg Cys Ile Gln Ala
    50                  55                  60
Asn Tyr Ser Leu Met Glu Asn Gly Lys Ile Lys Val Leu Asn Gln Glu
65                  70                  75                  80
Leu Arg Ala Asp Gly Thr Val Asn Gln Ile Glu Gly Glu Ala Thr Pro
                85                  90                  95
Val Asn Leu Thr Glu Pro Ala Lys Leu Glu Val Lys Phe Ser Trp Phe
            100                 105                 110
Met Pro Ser Ala Pro Tyr Trp Ile Leu Ala Thr Asp Tyr Glu Asn Tyr
        115                 120                 125
Ala Leu Val Tyr Ser Cys Thr Cys Ile Ile Gln Leu Phe His Val Asp
    130                 135                 140
Phe Ala Trp Ile Leu Ala Arg Asn Pro Asn Leu Pro Pro Glu Thr Val
145                 150                 155                 160
Asp Ser Leu Lys Asn Ile Leu Thr Ser Asn Asn Ile Asp Val Lys Lys
                165                 170                 175
Met Thr Val Thr Asp Gln Val Asn Cys Pro Lys Leu Ser
            180                 185

<210> 6
<211> 2127
<212> DNA
<213> Homo sapiens

<400> 6

gaaagatgga tcactccagc tcaaagagaa catgtgggaa tgaaaggaca ggctgggccc 60

aaaggagaaa agggtgatgc tggggaggag cttcctggcc ctcctgaacc ttctgggcct 120

gttggaccca cggcaggagc agaagcagag ggctctggcc taggctgggg ctcggacgtc 180

ggctctggct ctggtgacct ggtgggcagt gagcagctgc tgagaggtcc tccaggaccc 240

ccagggccac ctggcttacc tgggattcca ggaaaaccag gaactgatgt tttcatggga 300

ccccctggat ctcctggaga ggatggacct gctggtgaac ctgggccccc gggccctgag 360

ggacagcctg gagttgatgg agccaccggc cttcccggga tgaaagggga gaagggagca 420

agagggccta atggctcagt tggtgaaaag ggtgaccctg gcaacagagg cttacctgga 480

cccccgggga aaaagggaca agctggccct cctggggtca tgggaccccc agggcctcct 540

ggaccccctg ggcccccagg ccctggatgc acaatgggac ttggattcga ggataccgaa 600

ggctctggaa gcacccagct attgaatgaa cccaaactct ccagaccaac ggctgcaatt 660

ggtctcaaag gagagaaagg agaccgggga cccaagggag aaagggggat ggatggagcc 720

agtattgtgg gaccccctgg gccgagaggg ccacctgggc acatcaaggt cttgtctaat 780

tccttgatca atatcaccca tggattcatg aatttctcgg acattcctga gctggtgggg 840

cctccggggc cggacgggtt gcctgggctg ccaggatttc caggtcctag aggaccaaaa 900

ggtgacactg gtttacctgg ctttccagga ctaaaaggag aacagggcga gaagggagag 960

ccgggtgcca tcctgacaga ggacattcct ctggaaaggc tgatggggaa aaagggtgaa 1020

cctggaatgc atggagcccc aggaccaatg gggcccaaag gaccaccagg acataaagga 1080

gaatttggcc ttcccgggcg acctggtcgc ccaggactga atggcctcaa gggtaccaaa 1140

ggagatccag gggtcattat gcagggccca cctggcttac ctggccctcc aggcccccct 1200

gggccacctg gagctgtgat taacatcaaa ggagccattt tcccaatacc cgtccgacca 1260

cactgcaaaa tgccagttga tactgctcat cctgggagtc cagagctcat cacttttcac 1320

ggtgttaaag gagagaaagg atcctggggt cttcctggct caaagggaga aaaaggcgac 1380

cagggagccc agggaccacc aggtcctcca cttgatctag cttacctgag acactttctg 1440

aacaacttga agggggagaa tggagacaag gggttcaaag gtgaaaaagg agaaaaagga 1500

gacattaatg gcagcttcct tatgtctggg cctccaggcc tgcccggaaa tccaggcccg 1560

gctggccaaa aaggggagac agtcgttggg ccccaaggac ccccaggtgc tcctggtctg 1620

cctgggccac ctggctttgg aagacctggt gatcctgggc caccggggcc cccggggcca 1680

ccaggacctc cagctatcct gggagcagct gtggcccttc caggtccccc tggccctcca 1740

ggacagccag ggcttcccgg atccagaaac ctggtcacag cattcagcaa catggatgac 1800

atgctgcaga aagcgcattt ggttatagaa ggaacattca tctacctgag ggacagcact 1860

gagtttttca ttcgtgttag agatggctgg aaaaaattac agctgggaga actgatcccc 1920

attcctgccg acagccctcc accccctgcg ctttccagca acccacatca gcttctgcct 1980

ccaccaaacc ctatttcaag tgccaattat gagaagcctg ctctgcattt ggctgctctg 2040

aacatgccat tttctgggga cattcgagct gattttcagt gcttcaagca ggccagagct 2100

gcaggactgt tgtccaccta ccgagca 2127

<210> 7
<211> 2384
<212> DNA
<213> Homo sapiens

<400> 7
cctgctcgtt cctgacagaa ttaacggcac agccaacaag atgaacggag ctttggatca 60

ctcagaccaa ccagacccag atgccattaa gatgtttgtc ggacagatcc cccggtcatg 120

gtcggaaaag gagctgaaag aactttttga gccttacgga gccgtctacc agatcaacgt 180

cctccgggac cggagtcaga accctccgca gagtaaaggt tgttgtttcg taacatttta 240

tacaagaaaa gctgcacttg aggcccagaa tgcactgcac aatattaaaa ctttacctgg 300

gatgcatcat cccattcaga tgaaacctgc agatagtgaa aagtccaacg ctgtggaaga 360

cagaaaattg ttcataggaa tggtttcgaa gaaatgtaat gagaacgaca tcagggtgat 420

gttctctcca tttggccaga tagaagaatg ccggatcctc cggggacctg atgggctgag 480

tcgaggctgt gcgtttgtca cattttctac aagggcaatg gcacagaatg caatcaaagc 540

catgcatcag tctcagacca tggagggctg ctcttcacct atcgtggtga agtttgctga 600

cactcagaag gacaaagagc aaaggcgcct ccagcagcag ctcgctcagc agatgcagca 660

gctcaacact gccacctggg ggaacctgac agggctgggc ggactgaccc cacagtatct 720

ggcgctcctg cagcaggcca cctcctccag caacctgggt gcgttcagcg gcattcaaca 780

aatggcaggc atgaatgctt tacagttgca gaacctggcg acgctggctg ctgctgcagc 840
tgcggcccag acctcagcca ccagcaccaa tgcaaaccct ctctctacca cgagcagcgc 900
cctgggagcc ctcacgagtc ccgtggctgc ttcaaccccc aactccactg ctggtgcagc 960
catgaactcc ttgacctctc tcgggactct gcaaggactg gctggagcca ctgttggact 1020
gaataatatt aatgcactag cagttgctca aatgctctca ggtatggcgg ctctgaatgg 1080
aggacttggc gccacaggct tgacgaatgg cacggctggc accatggacg ccctcaccca 1140
ggcctactca ggaattcaac agtacgcagc cgccgcgctg cccactctgt acagccagag 1200
cctgctgcag cagcagagcg ctgcaggcag ccagaaggaa ggtccagagg gggcaaacct 1260
ctttatttac caccttccac aggaatttgg agaccagcac attctgcaga tgttcatgcc 1320
ttttggaaat gttatctctg ctaaagtctt cattgacaaa cagaccaatc tgagcaagtg 1380
ctttggtttt gttagctacg acaatccagt ctctgcacaa gctgctatcc aagctatgaa 1440
tggctttcag atcggcatga aacgcttgaa ggtgcagctg aagcgttcca aaaacgacag 1500
caaaccttac tgatcctaac cccagaggct ccctgctctc attttagctt tcttaggaca 1560
tcttcatgcc cgttagttca tcgtttgcct agcatgtccc tgtggcgtct caaaaaaaag 1620
tttcatcgtc ccgtcattgt ttctgatgtc tttctgacct cacatcatat ttggttctcc 1680
tactgacctt tgatctagtt tgacctttga aatttgcatg tgacctcatc tagctatgaa 1740
ttctgggaag tcaatgtgaa aaacattgct gcattcatgc aagactgaaa tttattatta 1800
gacaaattca ttatagaaaa aacctgtggc aaaaacgttt ctttcttatt ttttttcttt 1860
tcctaaaaca gacttgaaag tattatacag ggattggcat tcttcccggt cactggtaac 1920
aatagcaata tgtgtccagg gacacagaat gttggtttct aacagactac ttccaaaaac 1980
agtttgagaa aaaaactgtc tgattttaag tctctagagg tctgtaatag tttttacatt 2040
tttcaggcag tgtaaagttt tttgataagg ccattttagg tggctcactt tctcattaag 2100
atatatatat agaaccactt tttgtagatt agtataagaa aaatatttac cctgttttgg 2160
ggcaaatgct acctatttgt gtcacctttt gctgaactca cagttagaca atccatggtt 2220
taatgcacat gaaattacct atattttata ctgtttcaat gtacaggaga aaggttactg 2280
taaactgtgt tatgttggtg cttctgtgaa ttaagttgtg gtttcatcat gagtcttaat 2340

gttctttgtt gataagacaa gtttagaatt ggtttactta atac 2384

<210> 8
<211> 490
<212> PRT
<213> Homo sapiens

<400> 8
Met Asn Gly Ala Leu Asp His Ser Asp Gln Pro Asp Pro Asp Ala Ile
1               5                   10                  15
Lys Met Phe Val Gly Gln Ile Pro Arg Ser Trp Ser Glu Lys Glu Leu
            20                  25                  30
Lys Glu Leu Phe Glu Pro Tyr Gly Ala Val Tyr Gln Ile Asn Val Leu
        35                  40                  45
Arg Asp Arg Ser Gln Asn Pro Pro Gln Ser Lys Gly Cys Cys Phe Val
    50                  55                  60
Thr Phe Tyr Thr Arg Lys Ala Ala Leu Glu Ala Gln Asn Ala Leu His
65                  70                  75                  80
Asn Ile Lys Thr Leu Pro Gly Met His His Pro Ile Gln Met Lys Pro
                85                  90                  95
Ala Asp Ser Glu Lys Ser Asn Ala Val Glu Asp Arg Lys Leu Phe Ile
            100                 105                 110
Gly Met Val Ser Lys Lys Cys Asn Glu Asn Asp Ile Arg Val Met Phe
        115                 120                 125
Ser Pro Phe Gly Gln Ile Glu Glu Cys Arg Ile Leu Arg Gly Pro Asp
    130                 135                 140
Gly Leu Ser Arg Gly Cys Ala Phe Val Thr Phe Ser Thr Arg Ala Met
145                 150                 155                 160
Ala Gln Asn Ala Ile Lys Ala Met His Gln Ser Gln Thr Met Glu Gly
                165                 170                 175
Cys Ser Ser Pro Ile Val Val Lys Phe Ala Asp Thr Gln Lys Asp Lys
            180                 185                 190
Glu Gln Arg Arg Leu Gln Gln Gln Leu Ala Gln Gln Met Gln Gln Leu
        195                 200                 205
Asn Thr Ala Thr Trp Gly Asn Leu Thr Gly Leu Gly Gly Leu Thr Pro
    210                 215                 220
Gln Tyr Leu Ala Leu Leu Gln Gln Ala Thr Ser Ser Ser Asn Leu Gly
225                 230                 235                 240
Ala Phe Ser Gly Ile Gln Gln Met Ala Gly Met Asn Ala Leu Gln Leu
                245                 250                 255
Gln Asn Leu Ala Thr Leu Ala Ala Ala Ala Ala Ala Ala Gln Thr Ser
            260                 265                 270
Ala Thr Ser Thr Asn Ala Asn Pro Leu Ser Thr Thr Ser Ser Ala Leu
        275                 280                 285
Gly Ala Leu Thr Ser Pro Val Ala Ala Ser Thr Pro Asn Ser Thr Ala
    290                 295                 300
Gly Ala Ala Met Asn Ser Leu Thr Ser Leu Gly Thr Leu Gln Gly Leu
305                 310                 315                 320
Ala Gly Ala Thr Val Gly Leu Asn Asn Ile Asn Ala Leu Ala Val Ala
                325                 330                 335

Gln Met Leu Ser Gly Met Ala Ala Leu Asn Gly Gly Leu Gly Ala Thr
        340                 345                 350
Gly Leu Thr Asn Gly Thr Ala Gly Thr Met Asp Ala Leu Thr Gln Ala
    355                 360                 365
Tyr Ser Gly Ile Gln Gln Tyr Ala Ala Ala Ala Leu Pro Thr Leu Tyr
370                 375                 380
Ser Gln Ser Leu Leu Gln Gln Gln Ser Ala Ala Gly Ser Gln Lys Glu
385                 390                 395                 400
Gly Pro Glu Gly Ala Asn Leu Phe Ile Tyr His Leu Pro Gln Glu Phe
                405                 410                 415
Gly Asp Gln His Ile Leu Gln Met Phe Met Pro Phe Gly Asn Val Ile
        420                 425                 430
Ser Ala Lys Val Phe Ile Asp Lys Gln Thr Asn Leu Ser Lys Cys Phe
    435                 440                 445
Gly Phe Val Ser Tyr Asp Asn Pro Val Ser Ala Gln Ala Ala Ile Gln
450                 455                 460
Ala Met Asn Gly Phe Gln Ile Gly Met Lys Arg Leu Lys Val Gln Leu
465                 470                 475                 480
Lys Arg Ser Lys Asn Asp Ser Lys Pro Tyr
                485                 490

<210> 9
<211> 419
<212> DNA
<213> Homo sapiens

<400> 9
tttttttttt tttttttcag catagtgaac gttaatattt ttatttcttt tgtaatgaag     60

agtacaaatg gttgggagca ggacatcaca ggaggaggaa agatagcgcc atctctgcag    120

aagaactcct gagccacaca cagaaggaaa gttgatcccc agggcagcct ttcccaccaa    180

aaaaatcagg cccaatccag gagagtttgc cagtagctcc ccagggttcc agggtgtctg    240

ccagccttcc taggaatcgt gggcaggctt ctaggtgcca gtgactcaaa ctcctttttc    300

cacttcccag ttcaacctgg tcactctcat ccccacaagt tcccaatctg aatcccattc    360

tctgaccatt ctctgcttcc ttgtttttaa tctcatttga gagtgatcct cacgggttc     419


<210> 10
<211> 762
<212> DNA
<213> Homo sapiens

<400> 10
gccgggagga gcggcgcgcg ctcggacctc tcccgccctg ctcgttcgct ctccagcttg     60

ggatggccgg ctacctgcgg gtcgtgcgct cgctctgcag agcctcaggc tcgcggccgg    120

cctgggcgcc ggcggccctg acagccccca cctcgcaaga gcagccgcgg cgccactatg 180

ccgacaaaag gatcaaggtg gcgaagccgt ggtggagatg gatggtgatg agatgacccg 240

tattatctgg cagttcatca aggagaagct catcctgccc cacgtggaca tccagctaaa 300

gtattttgac ctcgggctcc caaaccgtga ccagactgat gaccaggtca ccattgactc 360

tgcactggcc acccagaagt acagtgtggc tgtcaagtgt gccaccatca cccctgatga 420

ggcccgtgtg gaagagttca agctgaagaa gatgtggaaa agtcccaatg gaactatccg 480

gaacatcctg ggggggactg tcttccggga gcccatcatc tgcaaaaaca tcccacgcct 540

agtccctggc tggaccaagc ccatcaccat tggcaggcac gcccatggcg accagtacaa 600

ggccacagac tttgtggcag accgggccgg cactttcaaa atggtcttca ccccaaaaga 660

tggcagtggt gtcaaggagt gggaagtgta caacttcccc gcaggcgcgc gtgggcatgg 720

gcatgtacma caccgacgag tccatctcag gttttgcgca ca 762

<210> 11
<211> 689
<212> DNA
<213> Homo sapiens

<400> 11
aactccatct ttgaaaaaca tttaataatg taatgtgttt gtggtacagg gtgagtacag 60

atgcacagga ggccataggg tttaggcaaa ggggagcaca aaagttgaag atgaggcgct 120

gccatcaatg ctgggacttc aggccaaggg caggaactga ggaagccaca agggaggaca 180

ttttctgcag ttgctgaacc agtagcaact aggtcctgag aaagccctct ctcgtggaag 240

aataacagcc aggcgggaaa gcttttcatc ctgcaaagct ggggaagaag attcttcctt 300

aaattgtcat ctgcacttca gctcaggaat cctgttggct gaagtccaga gtgtcctttc 360

tgattcctga agtagatgaa cagcccggcc ccaaggaaga gcaggcccag cacaaagccc 420

ccgactccac tcagcatctt gctctgtgca gattcagacc gtgcactcca ttccactgtg 480

agagggctcg ttacgcttgg gtgctccact tggcaggtgt aaacctctcc actccgagga 540

actgtttcta gcatcaccag ggtctggaat gtccagtctc cattctggat caggcgcgtg 600

gacaccaccc cagccttctc ttcctgcccg ttccggaacc agctgacttc aatgctgcct 660

ggatagaaac cactcacaga gcagaccag 689

<210> 12
<211> 1959
<212> DNA
<213> Homo sapiens

<400> 12
gggagcacgc gggagtccgg ctgcccaccc atggcagggt ctaccagaag ggccacgagc 60

tggtgttggc caatattgct gaaagtgatg ctggtgtcta cacctgccac gcggccaacc 120

tggctggtca gcggagacag gatgtcaaca tcactgtggc cactgtgccc tcctggctga 180

agaagcccca agacagccag ctggaggagg gcaaacccgg ctacttggat tgcctgaccc 240

aggccacacc aaaacctaca gttgtctggt acagaaacca gatgctcatc tcagaggact 300

cacggttcga ggtgttcctg gcaaaggctc agggcttgga ggagggagtg gcagagaccc 360

tggtacttgt gaagagcctg cagagcaagg atgagcagca gcagctggac ttccggaggg 420

agttggagat gtttgggaag ctgaaccacg ccaacgtggt gcggctcctg gggctgtgcc 480

gggaggctga gccccactac atggtgctgg aatatgtgga tctgggagac ctcaagcagt 540

tcctgaggat ttccaagagc aaggatgaaa aattgaagtc acagcccctc agcaccaagc 600

agaaggtggc cctatgcacc caggtagccc tgggcatgga gcacctgtcc aacaaccgct 660

ttgtgcataa ggacttggct gcgcgtaact gcctggtcag tgcccagaga caagtgaagg 720

tgtctgccct gggcctcagc aaggatgtgt acaacagtga gtactaccac ttccgccagg 780

cctgggtgcc gctgcgctgg atgtcccccg aggccatcct ggagggtgac ttctctacca 840

agtctgatgt ctgggccttc ggtgtgctga tgtgggaagt gtttacacat ggagagatgc 900

cccatggtgg gcaggcagat gatgaagtac tggcagattt gcaggctggg aaggctagac 960

ttcctcagcc cgagggctgc ccttccaaac tctatcggct gatgcagcgc tgctgggccc 1020

tcagccccaa ggaccggccc tccttcagtg agattgccag cgccctggga gacagcaccg 1080

tggacagcaa gccgtgagga gggagcccgc tcaggatggc ctgggcaggg gaggacatct 1140

ctagagggaa gctcacagca tgatgggcaa gatccctgtc ctcctgggcc ctgaggcccc 1200

tgccctagtg caacaggcat tgctgaggtc tgagcagggc ctggcctttc ctcctcttcc 1260

tcaccctcat cctttgggag gctgacttgg acccaaactg ggcgactagg gctttgagct 1320

gggcagtttt ccctgccacc tcttcctcta tcagggacag tgtgggtgcc acaggtaacc 1380

ccaatttctg gccttcaact tctccccttg accgggtcca actctgccac tcatctgcca 1440

actttgcctg gggagggcta ggcttgggat gagctgggtt tgtggggagt tccttaatat 1500

tctcaagttc tgggcacaca gggttaatga gtctcttggc ccactggtcc cacttggggg 1560

tctagaccag gattatagag gacacagcaa gtgagtcctc cccactctgg gcttgtgcac 1620

actgacccag acccacgtct tccccaccct tctctccttt cctcatccta agtgcctggc 1680

agatgaagga gttttcagga gcttttgaca ctatataaac cgcccttttt gtatgcacca 1740

cgggcggctt ttatatgtaa ttgcagcgtg gggtgggtgg gcatgggagg taggggtggg 1800

ccctggagat gaggagggtg ggccatcctt accccacact tttattgttg tcgttttttg 1860

tttgttttgt ttttttgttt ttgtttttgt ttttacactc gctgctctca ataaataagc 1920

cttttttaca aaaaaaaaaa aaaaaaaaaa aaaaaaaaa 1959

<210> 13
<211> 364
<212> PRT
<213> Homo sapiens

<400> 13
Glu His Ala Gly Val Arg Leu Pro Thr His Gly Arg Val Tyr Gln Lys
1               5                   10                  15

Gly His Glu Leu Val Leu Ala Asn Ile Ala Glu Ser Asp Ala Gly Val
            20                  25                  30

Tyr Thr Cys His Ala Ala Asn Leu Ala Gly Gln Arg Arg Gln Asp Val
        35                  40                  45

Asn Ile Thr Val Ala Thr Val Pro Ser Trp Leu Lys Lys Pro Gln Asp
    50                  55                  60

Ser Gln Leu Glu Glu Gly Lys Pro Gly Tyr Leu Asp Cys Leu Thr Gln
65                  70                  75                  80

Ala Thr Pro Lys Pro Thr Val Val Trp Tyr Arg Asn Gln Met Leu Ile
                85                  90                  95

Ser Glu Asp Ser Arg Phe Glu Val Phe Leu Ala Lys Ala Gln Gly Leu
            100                 105                 110

Glu Glu Gly Val Ala Glu Thr Leu Val Leu Val Lys Ser Leu Gln Ser
        115                 120                 125

Lys Asp Glu Gln Gln Gln Leu Asp Phe Arg Arg Glu Leu Glu Met Phe
    130                 135                 140

Gly Lys Leu Asn His Ala Asn Val Val Arg Leu Leu Gly Leu Cys Arg
145                 150                 155                 160

Glu Ala Glu Pro His Tyr Met Val Leu Glu Tyr Val Asp Leu Gly Asp
                165                 170                 175

Leu Lys Gln Phe Leu Arg Ile Ser Lys Ser Lys Asp Glu Lys Leu Lys
            180                 185                 190

Ser Gln Pro Leu Ser Thr Lys Gln Lys Val Ala Leu Cys Thr Gln Val
        195                 200                 205

Ala Leu Gly Met Glu His Leu Ser Asn Asn Arg Phe Val His Lys Asp
    210                 215                 220

Leu Ala Ala Arg Asn Cys Leu Val Ser Ala Gln Arg Gln Val Lys Val
225                 230                 235                 240

Ser Ala Leu Gly Leu Ser Lys Asp Val Tyr Asn Ser Glu Tyr Tyr His
                245                 250                 255

Phe Arg Gln Ala Trp Val Pro Leu Arg Trp Met Ser Pro Glu Ala Ile
            260                 265                 270

Leu Glu Gly Asp Phe Ser Thr Lys Ser Asp Val Trp Ala Phe Gly Val
        275                 280                 285

Leu Met Trp Glu Val Phe Thr His Gly Glu Met Pro His Gly Gly Gln
    290                 295                 300

Ala Asp Asp Glu Val Leu Ala Asp Leu Gln Ala Gly Lys Ala Arg Leu
305                 310                 315                 320

Pro Gln Pro Glu Gly Cys Pro Ser Lys Leu Tyr Arg Leu Met Gln Arg
                325                 330                 335

Cys Trp Ala Leu Ser Pro Lys Asp Arg Pro Ser Phe Ser Glu Ile Ala
            340                 345                 350

Ser Ala Leu Gly Asp Ser Thr Val Asp Ser Lys Pro
        355                 360

<210> 14
<211> 430
<212> DNA
<213> Homo sapiens

<400> 14
ggttgatgtt tcaacaaagt agttttaagt tccatccatt cagattttaa gcattttgat 60

ttatttaaaa agggattgtt catagaaaaa aattactttg aacagtatac aggactggtg 120

gagattggct atttcacaac atcagacagt acaatcagta tctgccatat ggctggtctc 180

tgtagacgcc ctttgctgtc ctcgctgaag gtgccttgtg tcttgagtta gtccactctt 240

cttgcccgta ggaatcataa gtctcctcac tgagtccatg tccgtaatca tagtaatcag 300

caccactttg ggctggggtg ctatagctgt tatcatagga atcataactc tgttcatcat 360

aagcagtgcc atatccatca tcatagtcat attctccata agtctcttgg tgtcgggggt 420

ggcggtggaa 430

<210> 15
<211> 616
<212> DNA
<213> Homo sapiens

<400> 15
ggtatgatga ccaaacataa aaagtgtttt ataattgttg gtgttttaat aacaactaat 60

attattactc tgatagttaa actaactcga gattctcaga gtttatgccc ctatgattgg 120

attggtttcc aaaacaaatg ctattatttc tctaaagaag aaggagattg gaattcaagt 180

aaatacaact gttccactca acatgccgac ctaactataa ttgacaacat agaagaaatg 240

aattttctta ggcggtataa atgcagttct gatcactgga ttggactgaa gatggcaaaa 300

aatcgaacag gacaatgggt agatggagct acatttacca aatcgtttgg catgagaggg 360

agtgaaggat gtgcctacct cagcgatgat ggtgcagcaa cagctagatg ttacaccgaa 420

agaaaatgga tttgcaggaa aagaatacac taagttaatg tctaagataa tggggaaaat 480

agaaaataac attattaagt gtaaaaccag caaagtactt ttttaattaa acaaagttcg 540

agttttgtac ctgtctggtt aattctgctt acgtgtcagg ctacacataa aagccacttc 600

aaagattggc aaaaaa 616

<400> 16
Met Met Thr Lys His Lys Lys Cys Phe Ile Ile Val Gly Val Leu Ile
1               5                   10                  15
Thr Thr Asn Ile Ile Thr Leu Ile Val Lys Leu Thr Arg Asp Ser Gln
            20                  25                  30

Ser Leu Cys Pro Tyr Asp Trp Ile Gly Phe Gln Asn Lys Cys Tyr Tyr
        35                  40                  45
Phe Ser Lys Glu Glu Gly Asp Trp Asn Ser Ser Lys Tyr Asn Cys Ser
    50                  55                  60
Thr Gln His Ala Asp Leu Thr Ile Ile Asp Asn Ile Glu Glu Met Asn
65                  70                  75                  80
Phe Leu Arg Arg Tyr Lys Cys Ser Ser Asp His Trp Ile Gly Leu Lys
                85                  90                  95
Met Ala Lys Asn Arg Thr Gly Gln Trp Val Asp Gly Ala Thr Phe Thr
            100                 105                 110
Lys Ser Phe Gly Met Arg Gly Ser Glu Gly Cys Ala Tyr Leu Ser Asp
        115                 120                 125
Asp Gly Ala Ala Thr Ala Arg Cys Tyr Thr Glu Arg Lys Trp Ile Cys
    130                 135                 140
Arg Lys Arg Ile His
145

<210> 17
<211> 1973
<212> DNA
<213> Homo sapiens

<400> 17
gggatattgg agtagcaaga ggctgggaag ccatcactta ccttgcactg agaaagaaga 60

caaaggccag tatgcacagc tttcctccac tgctgctgct gctgttctgg ggtgtggtgt 120

ctcacagctt cccagcgact ctagaaacac aagagcaaga tgtggactta gtccagaaat 180

acctggaaaa atactacaac ctgaagaatg atgggaggca agttgaaaag cggagaaata 240

gtggcccagt ggttgaaaaa ttgaagcaaa tgcaggaatt ctttgggctg aaagtgactg 300

ggaaaccaga tgctgaaacc ctgaaggtga tgaagcagcc cagatgtgga gtgcctgatg 360

tggctcagtt tgtcctcact gaggggaacc ctcgctggga gcaaacacat ctgacctaca 420

ggattgaaaa ttacacgcca gatttgccaa gagcagatgt ggaccatgcc attgagaaag 480

ccttccaact ctggagtaat gtcacacctc tgacattcac caaggtctct gagggtcaag 540

cagacatcat gatatctttt gtcaggggag atcatcggga caactctcct tttgatggac 600

ctggaggaaa tcttgctcat gcttttcaac caggcccagg tattggaggg gatgctcatt 660

ttgatgaaga tgaaaggtgg accaacaatt tcagagagta caacttacat cgtgttgcgg 720

ctcatgaact cggccattct cttggactct cccattctac tgatatcggg gctttgatgt 780

accctagcta caccttcagt ggtgatgttc agctagctca ggatgacatt gatggcatcc 840

aagccatata tggacgttcc caaaatcctg tccagcccat cggcccacaa accccaaaag 900

cgtgtgacag taagctaacc tttgatgcta taactacgat tcggggagaa gtgatgttct 960

ttaaagacag attctacatg cgcacaaatc ccttctaccc ggaagttgag ctcaatttca 1020

tttctgtttt ctggccacaa ctgccaaatg ggcttgaagc tgcttacgaa tttgccgaca 1080

gagatgaagt ccggtttttc aaagggaata agtactgggc tgttcaggga cagaatgtgc 1140

tacacggata ccccaaggac atctacagct cctttggctt ccctagaact gtgaagcata 1200

tcgatgctgc tctttctgag gaaaacactg gaaaaaccta cttctttgtt gctaacaaat 1260

actggaggta tgatgaatat aaacgatcta tggatccagg ttatcccaaa atgatagcac 1320

atgactttcc tggaattggc cacaaagttg atgcagtttt catgaaagat ggatttttct 1380

atttctttca tggaacaaga caatacaaat ttgatcctaa aacgaagaga attttgactc 1440

tccagaaagc taatagctgg ttcaactgca ggaaaaattg aacattacta atttgaatgg 1500

aaaacacatg gtgtgagtcc aaagaaggtg ttttcctgaa gaactgtcta ttttctcagt 1560

catttttaac ctctagagtc actgatacac agaatataat cttatttata cctcagtttg 1620

catatttttt tactatttag aatgtagccc tttttgtact gatataattt agttccacaa 1680

atggtgggta caaaaagtca agtttgtggc ttatggattc atataggcca gagttgcaaa 1740

gatcttttcc agagtatgca actctgacgt tgatcccaga gagcagcttc agtgacaaac 1800

atatcctttc aagacagaaa gagacaggag acatgagtct ttgccggagg aaaagcagct 1860

caagaacaca tgtgcagtca ctggtgtcac cctggatagg caagggataa ctcttctaac 1920

acaaaataag tgttttatgt ttggaataaa gtcaaccttg tttctactgt ttt 1973

<210> 18
<211> 469
<212> PRT
<213> Homo sapiens

<400> 18

Met His Ser Phe Pro Pro Leu Leu Leu Leu Leu Phe Trp Gly Val Val
1               5                   10                  15

Ser His Ser Phe Pro Ala Thr Leu Glu Thr Gln Glu Gln Asp Val Asp
            20                  25                  30

Leu Val Gln Lys Tyr Leu Glu Lys Tyr Tyr Asn Leu Lys Asn Asp Gly
        35                  40                  45

Arg Gln Val Glu Lys Arg Arg Asn Ser Gly Pro Val Val Glu Lys Leu
    50                  55                  60

Lys Gln Met Gln Glu Phe Phe Gly Leu Lys Val Thr Gly Lys Pro Asp
65                  70                  75                  80

Ala Glu Thr Leu Lys Val Met Lys Gln Pro Arg Cys Gly Val Pro Asp
                85                  90                  95

Val Ala Gln Phe Val Leu Thr Glu Gly Asn Pro Arg Trp Glu Gln Thr
            100                 105                 110

His Leu Thr Tyr Arg Ile Glu Asn Tyr Thr Pro Asp Leu Pro Arg Ala
        115                 120                 125

Asp Val Asp His Ala Ile Glu Lys Ala Phe Gln Leu Trp Ser Asn Val
    130                 135                 140

Thr Pro Leu Thr Phe Thr Lys Val Ser Glu Gly Gln Ala Asp Ile Met
145                 150                 155                 160

Ile Ser Phe Val Arg Gly Asp His Arg Asp Asn Ser Pro Phe Asp Gly
                165                 170                 175

Pro Gly Gly Asn Leu Ala His Ala Phe Gln Pro Gly Pro Gly Ile Gly
            180                 185                 190

Gly Asp Ala His Phe Asp Glu Asp Glu Arg Trp Thr Asn Asn Phe Arg
        195                 200                 205

Glu Tyr Asn Leu His Arg Val Ala Ala His Glu Leu Gly His Ser Leu
    210                 215                 220

Gly Leu Ser His Ser Thr Asp Ile Gly Ala Leu Met Tyr Pro Ser Tyr
225                 230                 235                 240

Thr Phe Ser Gly Asp Val Gln Leu Ala Gln Asp Asp Ile Asp Gly Ile
                245                 250                 255

Gln Ala Ile Tyr Gly Arg Ser Gln Asn Pro Val Gln Pro Ile Gly Pro
            260                 265                 270

Gln Thr Pro Lys Ala Cys Asp Ser Lys Leu Thr Phe Asp Ala Ile Thr
        275                 280                 285

Thr Ile Arg Gly Glu Val Met Phe Phe Lys Asp Arg Phe Tyr Met Arg
    290                 295                 300

Thr Asn Pro Phe Tyr Pro Glu Val Glu Leu Asn Phe Ile Ser Val Phe
305                 310                 315                 320

Trp Pro Gln Leu Pro Asn Gly Leu Glu Ala Ala Tyr Glu Phe Ala Asp
                325                 330                 335

Arg Asp Glu Val Arg Phe Phe Lys Gly Asn Lys Tyr Trp Ala Val Gln
            340                 345                 350

Gly Gln Asn Val Leu His Gly Tyr Pro Lys Asp Ile Tyr Ser Ser Phe
        355                 360                 365

Gly Phe Pro Arg Thr Val Lys His Ile Asp Ala Ala Leu Ser Glu Glu
    370                 375                 380

Asn Thr Gly Lys Thr Tyr Phe Phe Val Ala Asn Lys Tyr Trp Arg Tyr
385                 390                 395                 400

Asp Glu Tyr Lys Arg Ser Met Asp Pro Gly Tyr Pro Lys Met Ile Ala
                405                 410                 415

His Asp Phe Pro Gly Ile Gly His Lys Val Asp Ala Val Phe Met Lys
            420                 425                 430

Asp Gly Phe Phe Tyr Phe Phe His Gly Thr Arg Gln Tyr Lys Phe Asp
        435                 440                 445

Pro Lys Thr Lys Arg Ile Leu Thr Leu Gln Lys Ala Asn Ser Trp Phe
450                 455                 460

Asn Cys Arg Lys Asn
465

<210> 19
<211> 1141
<212> DNA
<213> Homo sapiens

<400> 19
ccgccaggcg ctttctcgga cgccttgccc agcgggccgc ccgaccccct gcaccatgga    60

ccccgctcgc cccctggggc tgtcgattct gctgcttttc ctgacggagg ctgcactggg    120

cgatgctgct caggagccaa caggaaataa cgcggagatc tgtctcctgc ccctagacta    180

cggaccctgc cgggccctac ttctccgtta ctactacgac aggtacacgc agagctgccg    240

ccagttcctg tacgggggct gcgagggcaa cgccaacaat ttctacacct gggaggcttg    300

cgacgatgct tgctggagga tagaaaaagt tcccaaagtt tgccggctgc aagtgagtgt    360

ggacgaccag tgtgaggggt ccacagaaaa gtatttcttt aatctaagtt ccatgacatg    420

tgaaaaattc ttttccggtg ggtgtcaccg gaaccggatt gagaacaggt ttccagatga    480

agctacttgt atgggcttct gcgcaccaaa gaaaattcca tcattttgct acagtccaaa    540

agatgaggga ctgtgctctg ccaatgtgac tcgctattat tttaatccaa gatacagaac    600

ctgtgatgct ttcacctata ctggctgtgg agggaatgac aataactttg ttagcaggga    660

ggattgcaaa cgtgcatgtg caaaagcttt gaaaaagaaa aagaagatgc caaagcttcg    720

ctttgccagt agaatccgga aaattcggaa gaagcaattt taaacattct taatatgtca    780

tcttgtttgt ctttatggct tatttgcctt tatggttgta tctgaagaat aatatgacag    840

catgaggaaa caaatcattg gtgatttatt caccagtttt tattaataca agtcactttt    900

taaaaaattt ggattttttt atatataact agctgctatt caaatgtgag tctaccattt    960

ttaatttatg gttcaactgt ttgtgagact gaattcttgc aatgcataag atataaaagc    1020

aaatatgact cactcatttc ttggggtcgt attcctgatt tcagaagagg atcataactg    1080

aaacaacata agacaatata atcatgtgct tttaacatat ttgagaataa aaaggactag 1140

c 1141

<210> 20
<211> 22
<212> DNA
<213> Artificial

<220>
<223> Sequence:serine (or cysteine) proteinase inhibitor sense primer

<400> 20
ccaaactttg aaagccaagg tg 22

<210> 21
<211> 21
<212> DNA
<213> Artificial

<220>
<223> serine (or cysteine) proteinase inhibitor antisense primer

<400> 21
tgcacttcaa agaccagcag g 21

<210> 22
<211> 21
<212> DNA
<213> Artificial

<220>
<223> adrenomedullin sense primer

<400> 22
aggaatagtc gcgcaagcat c 21

<210> 23
<211> 22
<212> DNA
<213> Artificial

<220>
<223> adrenomedullin antisense primer

<400> 23
cacgcattgc acttttcctc tt 22

<210> 24
<211> 21
<212> DNA
<213> Artificial

<220>
<223> apolipoprotein D sense primer

<400> 24
tgaagccacc ccagttaacc t 21


<210> 25
<211> 23
<212> DNA
<213> Artificial

<220>
<223> apolipoprotein D antisense primer

<400> 25
ggtcaacttc ctttgtgcgt ggt 23


<210> 26
<211> 22
<212> DNA
<213> Artificial

<220>
<223> collagen type 15 alpha 1 sense primer

<400> 26
accaaaccct atttcaagtg cc 22


<210> 27
<211> 21
<212> DNA
<213> Artificial

<220>
<223> collagen type 15 alpha 1 antisense primer

<400> 27
tagttatcca caaggcggac g 21


<210> 28
<211> 21
<212> DNA

<213> Artificial

<220>
<223> CUG triplet repeat RNA-binding protein 2 sense primer

<400> 28
atgtttgtcg gacagatccc c 21

<210> 29
<211> 21
<212> DNA
<213> Artificial

<220>
<223> CUG triplet repeat RNA-binding protein 2 antisense primer

<400> 29
atgtgacaaa cgcacagcct c 21

<210> 30
<211> 20
<212> DNA
<213> Artificial

<220>
<223> dermatopontin sense primer

<400> 30
atttgaaccg gcaaggcttc 20

<210> 31
<211> 21
<212> DNA
<213> Artificial

<220>
<223> dermatopontinanti sense primer

<400> 31
aagtggttgt tgctcctcgg a 21

<210> 32
<211> 20
<212> DNA
<213> Artificial

<220>
<223> isocitrate dehydrogenase sense primer

<400> 32
tgccatccag aagaaatggc 20

<210> 33
<211> 19
<212> DNA
<213> Artificial

<220>
<223> isocitrate dehydrogenase antisense primer

<400> 33
aggcaaagat gctggcgat 19

<210> 34
<211> 22
<212> DNA
<213> Artificial

<220>
<223> MHC Class 2 DR beta3 sense primer

<400> 34
tagctgtgga caaagccaac ct 22

<210> 35
<211> 22
<212> DNA
<213> Artificial

<220>
<223> MHC Class 2 DR beta3 antisense primer

<400> 35
ggcacacacc acgttctctg ta 22

<210> 36
<211> 22
<212> DNA
<213> Artificial

<220>
<223> protein tyrosine kinase 7 sense primer

<400> 36
tggaaccaga gcgtacgact gt 22

<210> 37
<211> 19
<212> DNA
<213> Artificial

<220>
<223> protein tyrosine kinase 7 antisense primer

<400> 37
tggctttgca gcgcttctt 19

<210> 38
<211> 22
<212> DNA
<213> Artificial

<220>
<223> Sam-68 like phosphotyrosine protein, T-STAR sense primer

<400> 38
aggagaaggc aaggatgaag aa 22

<210> 39
<211> 22
<212> DNA
<213> Artificial

<220>
<223> Sam-68 like phosphotyrosine protein, T-STAR antisense primer

<400> 39
tgttaactcc tggagctgtg ct 22

<210> 40
<211> 22
<212> DNA
<213> Artificial

<220>
<223> C-type lectin superfamily member 2 sense primer

<400> 40
gcccctatga ttggattggt tt 22

<210> 41
<211> 22
<212> DNA

<213> Artificial

<220>
<223> C-type lectin superfamily member 2 antisense primer

<400> 41
catccttcac tccctctcat gc 22


<210> 42
<211> 25
<212> DNA
<213> Artificial

<220>
<223> MMP (martix metalloproteinase) 1 sense primer

<400> 42
cgactctaga aacacaagag caaga 25


<210> 43
<211> 25
<212> DNA
<213> Artificial

<220>
<223> MMP (martix metalloproteinase) 1 antisense primer

<400> 43
aaggttagct tactgtcaca cgctt 25


<210> 44
<211> 17
<212> DNA
<213> Artificial

<220>
<223> TFPI (tissue factor pathway inhibitor) 2 sense primer

<400> 44
gtcgattctg cttttcc 17


<210> 45
<211> 20
<212> DNA
<213> Artificial

<220>
<223> TFPI (tissue factor pathway inhibitor) 2 antisense primer

<400> 45
atggaatttt ctttggtgcg                                    20


<210> 46
<211> 1045
<212> DNA
<213> Homo sapiens

<400> 46
ctcccgagct ctactgactc ccaacagagc gcccaagcaa gaaaatggcc ataagtggag     60

tccctgtgct aggatttttc atcatagctg tgctgatgag cgctcaggaa tcatgggcta    120

tcaaagaaga acatgtgatc atccaggccg agttctatct gaatcctgac caatcaggcg    180

agtttatgtt tgactttgat ggtgatgaga ttttccatgt ggatatggca aagaaggaga    240

cggtctggcg gcttgaagaa tttggacgat ttgccagctt tgaggctcaa ggtgcattgg    300

ccaacatagc tgtggacaaa gccaacctgg aaatcatgac aaagcgctcc aactatactc    360

cgatcaccaa tgtacctcca gaggtaactg tgctcacgaa cagccctgtg gaactgagag    420

agcccaacgt cctcatctgt ttcatcgaca agttcacccc accagtggtc aatgtcacgt    480

ggcttcgaaa tggaaaacct gtcaccacag gagtgtcaga gacagtcttc ctgcccaggg    540

aagaccacct tttccgcaag ttccactatc tccccttcct gccctcaact gaggacgttt    600

acgactgcag ggtggagcac tggggcttgg actgagcctc ttctcaagca ctgggaggtt    660

gatgctccaa gcccgtctcc agagacttac gagacgtggt gtgtgccctg gggctgactg    720

tgggtctggt cgggcttcct ttgggaccct ctttcatcat ccacggcgag tgcgccaaaa    780

gcattgccca gaaacccagg gggccctctg taaggcctgt gcgggggcag aacggtccct    840

taaaaagaag aacctcgtgg gcaaaacctg gcgcaagacg tcatacaaag ggggggcata    900

taacacgcgt gccccagaac gaccgcactc agggctcgcc gcctataaca ccccggtgtg    960

gtacccccaa ggcgcgctaa aactaaacag cgcgcacata gtagctcagg cgatacacca   1020

ctcacatata taatagcacc gctaa                              1045


<210> 47
<211> 18
<212> DNA
<213> Artificial

<220>
<223> adrenomedullin primer for RT-PCR

<400> 47
ggtttccgtc gccctgat 18


<210> 48
<211> 24
<212> DNA
<213> Artificial

<220>
<223> adrenomedullin primer for RT-PCR

<400> 48
gagcccactt attccacttc tttc 24


<210> 49
<211> 22
<212> DNA
<213> Artificial

<220>
<223> matrix metaroproteinase 1 primer for RT-PCR

<400> 49
gatggacctg gaggaaatct tg 22


<210> 50
<211> 24
<212> DNA
<213> Artificial

<220>
<223> matrix metaroproteinase 1 primer for RT-PCR

<400> 50
ccgcaacacg atgtaagttg tact 24


<210> 51
<211> 21
<212> DNA
<213> Artificial

<220>
<223> tissue factor pathway inhibitor 2 primer for RT-PCR

<400> 51
ggcaacgcca acaatttcta c                                    21

<210> 52
<211> 26
<212> DNA
<213> Artificial

<220>
<223> tissue factor pathway inhibitor 2 primer for RT-PCR

<400> 52
caaactttgg gaactttttc tatcct                               26

<210> 53
<211> 23
<212> DNA
<213> Artificial

<220>
<223> serine (or cysteine) proteinase inhibitor primer for PT-PCR

<400> 53
tgggtggaga ataacacaaa caa                                  23

<210> 54
<211> 22
<212> DNA
<213> Artificial

<220>
<223> serine (or cysteine) proteinase inhibitor primer for RT-PCR

<400> 54
ccagataagt ggcagcatca aa                                   22

<210> 55
<211> 28
<212> DNA
<213> Artificial

<220>
<223> protein tyrosine kinase 7 primer for RT-PCR

<400> 55
gggagttcct taatattctc aagttctg                             28

<210> 56
<211> 27
<212> DNA
<213> Artificial

<220>
<223> protein tyrosine kinase 7

<400> 56
gctgtgtcct ctataatcct ggtctag 27


<210> 57
<211> 20
<212> DNA
<213> Artificial

<220>
<223> collagen, type XV, alpha 1 primer for RT-PCR

<400> 57
ccagcaaccc acatcagctt 20


<210> 58
<211> 23
<212> DNA
<213> Artificial

<220>
<223> collagen, type XV, alpha 1 primer for RT-PCR

<400> 58
atgcagagca ggcttctcat aat 23


<210> 59
<211> 29
<212> DNA
<213> Artificial

<220>
<223> Sam68-like phosphotyrosine protein primer for RT-PCR

<400> 59
tcacaacatc agacagtaca atcagtatc 29


<210> 60
<211> 22
<212> DNA
<213> Artificial

<220>
<223> Sam68-like phosphotyrosine protein primer for RT-PCR

<400> 60
acgggcaaga agagtggact aa 22

<210> 61
<211> 23
<212> DNA
<213> Artificial

<220>
<223> apolipoprotein D primer for RT-PCR

<400> 61
tgagaagatc ccaacaacct ttg 23

<210> 62
<211> 24
<212> DNA
<213> Artificial

<220>
<223> apolipoprotein D primer for PT-PCR

<400> 62
tgatctttcc gttttccatt agtg 24

<210> 63
<211> 22
<212> DNA
<213> Artificial

<220>
<223> major histocompatibility complex DR beta

<400> 63
ggctgaagtc cagagtgtcc tt 22

<210> 64
<211> 18
<212> DNA
<213> Artificial

<220>
<223> major histocompatibility complex DR beta primer for RT-PCR

<400> 64
gctgggcctg ctcttcct 18

<210> 65
<211> 19
<212> DNA
<213> Artificial

<220>
<223> major histocompatibility complex DR alpha primer for RT-PCR

<400> 65
gcccagggaa gaccacctt 19

<210> 66
<211> 23
<212> DNA
<213> Artificial

<220>
<223> major histocompatibility complex DR alpha primer for RT-PCR

<400> 66
cagtcgtaaa cgtcctcagt tga 23

<210> 67
<211> 27
<212> DNA
<213> Artificial

<220>
<223> C-type lectin primer for RT-PCR

<400> 67
atccattttc tttcggtgta acatcta 27

<210> 68
<211> 23
<212> DNA
<213> Artificial

<220>
<223> C-type lectin primer for RT-PCR

<400> 68
catgagaggg agtgaaggat gtg 23

<210> 69
<211> 24
<212> DNA
<213> Artificial

<220>
<223> CUG triplet repeat RNA-binding protein 2 primer for RT-PCR

<400> 69
catgaatgct ttacagttgc agaa 24


<210> 70
<211> 19
<212> DNA
<213> Artificial

<220>
<223> CUG triplet repeat RNA-binding protein 2 primer for RT-PCR

<400> 70
gcgctgctcg tggtagaga 19


<210> 71
<211> 22
<212> DNA
<213> Artificial

<220>
<223> INTEGRIN, ALPHA 6 (ITGA6), MRNA

<400> 71
gatgacagtg ttccccgata cc 22


<210> 72
<211> 20
<212> DNA
<213> Artificial

<220>
<223> INTEGRIN, ALPHA 6 (ITGA6), MRNA

<400> 72
tgtaagtcag ccacgccaaa 20


<210> 73
<211> 21
<212> DNA
<213> Artificial

<220>
<223>  SOLUTE CARRIER FAMILY 20 (PHOSPHATE TRANSPORTER), MEMBER 1

<400>  73
aatggagaag ctgacatggc c                                    21


<210>  74
<211>  21
<212>  DNA
<213>  Artificial

<220>
<223>  SOLUTE CARRIER FAMILY 20 (PHOSPHATE TRANSPORTER), MEMBER 1

<400>  74
ccaaacccac agaccaacac a                                    21


<210>  75
<211>  21
<212>  DNA
<213>  Artificial

<220>
<223>  RIBONUCLEOTIDE REDUCTASE M2 POLYPEPTIDE (RRM2)

<400>  75
tccaaggaca ttcagcactg g                                    21


<210>  76
<211>  19
<212>  DNA
<213>  Artificial

<220>
<223>  RIBONUCLEOTIDE REDUCTASE M2 POLYPEPTIDE (RRM2), MRNA

<400>  76
atcgacgcaa aagaaccgg                                    19


<210>  77
<211>  22
<212>  DNA
<213>  Artificial

<220>
<223>  FOLLISTATIN (FST), TRANSCRIPT VARIANT FST317, MRNA

<400> 77
ggaagtccag taccaaggca ga                                22

<210> 78
<211> 19
<212> DNA
<213> Artificial

<220>
<223> FOLLISTATIN (FST), TRANSCRIPT VARIANT FST317, MRNA

<400> 78
tggcattgtc actggcaca                                19

<210> 79
<211> 20
<212> DNA
<213> Artificial

<220>
<223> SPROUTY (DROSOPHILA) HOMOLOG 2 (SPRY2), MRNA

<400> 79
gccatccgaa acaccaatga                                20

<210> 80
<211> 22
<212> DNA
<213> Artificial

<220>
<223> SPROUTY (DROSOPHILA) HOMOLOG 2 (SPRY2), MRNA

<400> 80
tgccacagtc ctcacacctg ta                                22

<210> 81
<211> 21
<212> DNA
<213> Artificial

<220>
<223> RAB3B, MEMBER RAS ONCOGENE FAMILY (RAB3B), MRNA

<400> 81
atgctgatga cacgttcacc c                                21

<210> 82
<211> 22
<212> DNA
<213> Artificial

<220>
<223> RAB3B, MEMBER RAS ONCOGENE FAMILY (RAB3B), MRNA

<400> 82
ggcctgcctt acactgatgt tc                                22


<210> 83
<211> 21
<212> DNA
<213> Artificial

<220>
<223> SOLUTE CARRIER FAMILY 2 (FACILITATED GLUCOSE TRANSPORTER)

<400> 83
gccttttcgt taaccgcttt g                                21


<210> 84
<211> 19
<212> DNA
<213> Artificial

<220>
<223> SOLUTE CARRIER FAMILY 2 (FACILITATED GLUCOSE TRANSPORTER)

<400> 84
tcctcgttgc ggttgatga                                19


<210> 85
<211> 21
<212> DNA
<213> Artificial

<220>
<223> INTERLEUKIN 13 RECEPTOR, ALPHA 2 (IL13RA2), MRNA

<400> 85
tgaaggctat ttgaagtcgc c                                21


<210> 86
<211> 19
<212> DNA
<213> Artificial

<220>
<223> INTERLEUKIN 13 RECEPTOR, ALPHA 2 (IL13RA2), MRNA

<400> 86
cttcgcttca atgcccttg                                        19


<210> 87
<211> 21
<212> DNA
<213> Artificial

<220>
<223> SERINE/THREONINE KINASE 12 (STK12), MRNA

<400> 87
tggaaacgtg tacttggctc g                                     21


<210> 88
<211> 21
<212> DNA
<213> Artificial

<220>
<223> SERINE/THREONINE KINASE 12 (STK12), MRNA

<400> 88
accagccgaa gtcagcaatc t                                     21


<210> 89
<211> 22
<212> DNA
<213> Artificial

<220>
<223> MINICHROMOSOME MAINTENANCE DEFICIENT (S. CEREVISIAE) 5

<400> 89
aagtttggcc tgactaccag ca                                    22


<210> 90
<211> 22
<212> DNA
<213> Artificial

<220>
<223> MINICHROMOSOME MAINTENANCE DEFICIENT (S. CEREVISIAE)

<400> 90
ccagacgtgt ataccccaat gg                                    22

<210> 91
<211> 22
<212> DNA
<213> Artificial

<220>
<223> THYROID HORMONE RECEPTOR INTERACTOR 13 (TRIP13), MRNA

<400> 91
cctgtgtaaa gcgttagccc ag                                    22

<210> 92
<211> 22
<212> DNA
<213> Artificial

<220>
<223> THYROID HORMONE RECEPTOR INTERACTOR 13 (TRIP13), MRNA

<400> 92
gtggcccaat gtactgcttg at                                    22

<210> 93
<211> 22
<212> DNA
<213> Artificial

<220>
<223> KINESIN-LIKE 6 (MITOTIC CENTROMERE-ASSOCIATED KINESIN)

<400> 93
ccagctttaa cgaagccatg ac                                    22

<210> 94
<211> 22
<212> DNA
<213> Artificial

<220>
<223> KINESIN-LIKE 6 (MITOTIC CENTROMERE-ASSOCIATED KINESIN)

<400> 94
gcatttacca gaacctgtcc ca                                    22

<210> 95
<211> 22
<212> DNA
<213> Artificial

<220>
<223> CYCLIN-DEPENDENT KINASE INHIBITOR 3

<400> 95
tcggtttatg tgctcttcca gg 22


<210> 96
<211> 22
<212> DNA
<213> Artificial

<220>
<223> CYCLIN-DEPENDENT KINASE INHIBITOR 3

<400> 96
atcctcttag gtctcgcagg ct 22


<210> 97
<211> 22
<212> DNA
<213> Artificial

<220>
<223> CHROMOSOME CONDENSATION PROTEIN G (HCAP-G), MRNA

<400> 97
gctcgccaga aattcgagtc ta 22


<210> 98
<211> 22
<212> DNA
<213> Artificial

<220>
<223> CHROMOSOME CONDENSATION PROTEIN G (HCAP-G), MRNA

<400> 98
catgatcact ttcagagtcg gc 22


<210> 99
<211> 21
<212> DNA
<213> Artificial

<220>
<223>  CDC28 PROTEIN KINASE 1 (CKS1), MRNA

<400>  99
gcccactacc caagaaacca a                                    21


<210>  100
<211>  22
<212>  DNA
<213>  Artificial

<220>
<223>  CDC28 PROTEIN KINASE 1 (CKS1), MRNA

<400>  100
cataaatccg caagtcacca ca                                   22


<210>  101
<211>  20
<212>  DNA
<213>  Artificial

<220>
<223>  PROTEIN REGULATOR OF CYTOKINESIS 1 (PRC1), MRNA

<400>  101
cccaagtgga attgatgcga                                      20


<210>  102
<211>  22
<212>  DNA
<213>  Artificial

<220>
<223>  PROTEIN REGULATOR OF CYTOKINESIS 1 (PRC1), MRNA

<400>  102
cccctcacac actgcttcat tt                                   22


<210>  103
<211>  21
<212>  DNA
<213>  Artificial

<220>
<223>  CELL DIVISION CYCLE 2, G1 TO S AND G2 TO M (CDC2), MRNA

<400> 103
ctgattttgg ccttgccaga g                                    21


<210> 104
<211> 22
<212> DNA
<213> Artificial

<220>
<223> CELL DIVISION CYCLE 2, G1 TO S AND G2 TO M (CDC2), MRNA

<400> 104
ggatgattca gtgccatttt gc                                   22


<210> 105
<211> 21
<212> DNA
<213> Artificial

<220>
<223> CDC20 (CELL DIVISION CYCLE 20, S. CEREVISIAE, HOMOLOG)

<400> 105
acagttcgcg ttcgagagtg a                                    21


<210> 106
<211> 21
<212> DNA
<213> Artificial

<220>
<223> CDC20 (CELL DIVISION CYCLE 20, S. CEREVISIAE, HOMOLOG)

<400> 106
ttccactgag ccgaaggatc t                                    21


<210> 107
<211> 22
<212> DNA
<213> Artificial

<220>
<223> LIKELY ORTHOLOG OF MATERNAL EMBRYONIC LEUCINE ZIPPER KINAS

<400> 107
tggctctctc ccagtagcat tc                                   22

<210> 108
<211> 21
<212> DNA
<213> Artificial

<220>
<223> LIKELY ORTHOLOG OF MATERNAL EMBRYONIC LEUCINE ZIPPER KINAS

<400> 108
tcacttgcgg tcacatcttc c 21

<210> 109
<211> 22
<212> DNA
<213> Artificial

<220>
<223> MINICHROMOSOME MAINTENANCE DEFICIENT (S. CEREVISIAE) 7

<400> 109
agagaacaca aggattgccc ag 22

<210> 110
<211> 22
<212> DNA
<213> Artificial

<220>
<223> MINICHROMOSOME MAINTENANCE DEFICIENT (S. CEREVISIAE) 7

<400> 110
atgacaggag ctgagacttg gc 22

<210> 111
<211> 22
<212> DNA
<213> Artificial

<220>
<223> CYCLIN A2 (CCNA2), MRNA

<400> 111
ggcactgctg ctatgctgtt ag 22

<210> 112
<211> 21
<212> DNA
<213> Artificial

<220>
<223> CYCLIN A2 (CCNA2), MRNA

<400> 112
tgaaggtcca tgagacaagg c                                    21


<210> 113
<211> 19
<212> DNA
<213> Artificial

<220>
<223> THYMIDINE KINASE 1, SOLUBLE (TK1), MRNA

<400> 113
ggcgcaatga gctgcatta                                    19


<210> 114
<211> 22
<212> DNA
<213> Artificial

<220>
<223> THYMIDINE KINASE 1, SOLUBLE (TK1), MRNA

<400> 114
aaatggcttc ctctggaagg tc                                    22


<210> 115
<211> 21
<212> DNA
<213> Artificial

<220>
<223> cyclin-dependent kinase inhibitor 2A (melanoma, p16, inhibits CDK
     4) (CDKN2A), mRNA

<400> 115
tccccgattg aaagaaccag a                                    21


<210> 116
<211> 22
<212> DNA
<213> Artificial

<220>
<223> cyclin-dependent kinase inhibitor 2A (melanoma, p16, inhibits CDK

4) (CDKN2A), mRNA

<400> 116
aagagaagcc agtaaccccc ct 22

## Description

### Technical Field

The present invention relates to detection and separation/distinguishment of mesenchymal stem cells; particularly, a gene marker for detecting mesenchymal stem cells and a polypeptide marker for detecting mesenchymal stem cells used for detecting, separating/distinguishing mesenchymal stem cells; and a method for detecting and distinguishing mesenchymal stem cells by using the markers.

### Background Art

Mesenchymal stem cells exist in the bone marrow, etc., of mammals, and are known as multipotent stem cells which differentiate into adipocytes, chondrocytes, and osteocytes. Due to their multipotency in differentiation, mesenchymal stem cells draw attention as xenograft, homograft and autograft materials for regenerative medicine of many tissues, such as bone, cartilage, tendon, muscle, fat, and periodontal tissue (Gene & Medicine, Vol. 4, No. 2 (2000) p58-61). A general statement as to the present condition and prospect of study of mesenchymal stem cells has been issued recently, and there is a report concerning collection and culture of mesenchymal stem cells (Experimental Medicine, Vol. 19, No. 3 (February issue) 2001, p350-356). Further, it has been reported that mesenchymal stem cells also exist in an adipose tissue (Tissue Engineering, P. A. Zuk et al., Multilineage cells from human adipose tissue: implications for cell-based therapies. 7: 211-228, 2001).

In recent years, some patent applications relating to culture, differentiation, etc. , of mesenchymal stem cells have been published. For instance, Published Japanese Translation of PCT International Publication No. 11-506610 discloses a composition and method for maintaining the viability of human mesenchymal precursor cells in a serum-free environment; Published Japanese Translation of PCT International Publication No. 10-512756 discloses a method comprising contacting the mesenchymal stem cells with a bioactive factor, such as osteoinductive factor, adjunct factor for differentiation, chondroinductive factor which comprise prostaglandin, ascorbic acid, collagenous extracellular matrix, etc. , in order to induce differentiation of mesenchymal stem cells; Japanese Laid-Open Patent Application No. 2000-217576 discloses a method for differentiating a multipotential mesenchymal stem cell into adipocytes, which includes incubation of the mulitpotential mesenchymal stem cell in the presence of prolactin or a substance with an equivalent effect, respectively.

The present inventors previously found that it is possible to proliferate mesenchymal stem cells remarkably rapidly while maintaining their differentiation capacity, by culturing mesenchymal stem cells in the presence of extracellular matrix of basement membrane, or in a medium containing a fibroblast growth factor (FGF), etc., and disclosed a culturing method capable of obtaining significantly larger amount of mesenchymal stem cells than a conventional culturing method (Japanese Laid-Open Patent Application No. 2003-52360). In addition, the present inventors disclosed a method for separating and collecting mesenchymal stem cells from oral tissues, in order to conduct separation and collection being safe for a collected parent body and easy for collecting, upon collecting mesenchymal stem cells (Japanese Laid-Open Patent Application No. 2003-52365).

Recently, with the development of regenerative medicine, mesenchymal stem cells draw attention as xenograft, homograft and autograft materials for regenerative medicine of many tissues, such as bone, cartilage, tendon, muscle, fat, periodontaltissue, because of their multipotency in differentiation. In order to use mesenchymal stem cells for regenerative medicine of tissues, it is necessary to collect the stem cells from body tissues first, to proliferate them, and to further differentiate and proliferate them to prepare a tissue. Mesenchymal stem cells exist in the bone marrow and periosteum, and it is necessary to develop a method for collecting mesenchymal stem cells from these tissues safely and easily for the practical use of these cells in tissue regenerative medicine. In addition, for the practical use of mesenchymal stem cells in tissue regenerative medicine, it is necessary to develop a technique for securing a sufficient amount of mesenchymal stem cells. In order to do this, it is important to develop a technique for culturing and proliferating the collected mesenchymal stem cells while maintaining their differentiation capacity.

As stated above, the present inventors have developed the method for conducting separation and collection being safe for a collected parent body and easy for collecting, upon collecting mesenchymal stem cells. Further, the present inventors have developed a method for explosively proliferating mesenchymal stem cells while maintaining their differentiation capacity by culturingmesenchymal stem cells in the presence of extracellular matrix of basement membrane, or in a medium containing a fibroblast growth factor (FGF), etc. However, for the purpose of practical use of the cultured and proliferated mesenchymal stem cells in regenerative medicine, it is necessary to confirm that the cultured cells are mesenchymal stem cells, and to develop a method for detecting and distinguishing the mesenchymal stem cells. As for mesenchymal stem cells, marker genes that characterize the cells have not been identified conventionally. Therefore, for the purpose of practical use of mesenchymal stem cells in regenerative medicine, the identification of marker genes that characterize mesenchymal stem cells, and the development of a method for detecting, and separating/distinguishing mesenchymal stem cells are important problems to be solved.

The present invention provides a gene marker for detecting mesenchymal stem cells and a polypeptide marker for detecting mesenchymal stem cells used for detecting and distinguishing mesenchymal stem cells; a primer for PCR amplification for detecting the gene marker; and a method for detecting and distinguishing mesenchymal stem cells by using the markers and the primer.

In order to find a gene which can serve as a marker for detecting mesenchymal stem cells, the present inventors compared the expression of various genes in mesenchymal stem cells, and searched for a gene which can serve as a marker. As a result, the present inventors found that there was a difference between fibroblasts and mesenchymal stem cells in the expression levels of 13 genes shown in the sequence listing, and the genes were expressed specifically in mesenchymal stem cells, and that the genes could serve as a marker for detecting mesenchymal stem cells. The present invention has been thus completed. The present invention comprises a probe for detecting mesenchymal stem cell marker genes, and a PCR primer for amplifying the genes in test cell upon detecting the mesenchymal stem cell gene markers. Further, the present invention comprises a polypeptide marker for detecting mesenchymal stem cells comprising a polypeptide wherein the mesenchymal stem cell marker gene of the present invention is expressed, and an antibody for detecting the polypeptide marker, which specifically binds to the polypeptide marker. Still further, the present invention comprises a method for distinguishing and separating mesenchymal stem cells using the probe for detecting mesenchymal stem cell marker genes, and the antibody which specifically binds to the polypeptide marker.

The process of the completion of the present invention is as follows: among various stem cells, mesenchymal stem cells are capable of differentiating into many tissues such as bone, cartilage, tendon, fat, skeletal muscle, cardiac muscle, blood vessel, nerve, etc., and are significantly expected as cells for regenerative medicine. However, marker genes that characterize mesenchymal stem cells have not been identified. Therefore, the present inventors have conducted a keen search for identifying marker genes that characterize mesenchymal stem cells. In brief, the search comprises the following steps: proliferating mesenchymal stem cells with the method, previously developed by the present inventors, wherein mesenchymal stem cells derived from bone marrow are cultured in the presence of FGF, etc., thereby explosively proliferating the cells while maintaining their differentiation capacity; culturing the mesenchymal stem cells obtained by this method and human fibroblasts in the presence of FGF; extracting mRNA from the cultured cells; by using the mRNA as a template and a DNA chip (Incyte) as a probe, comparing the genes expressed in human-derived mesenchymal stem cells and fibroblasts by DNA microarray technology.

Among 9400 genes, 63 genes exhibited significantly high expression, and 141 genes exhibited significantly low expression in mesenchymal stem cells. Among them, 87 genes (29 genes exhibiting high expression and 58 genes exhibiting low expression in mesenchymal stem cells) exhibited a difference, of three-fold or more, in expression levels. Total RNAs were extracted from mesenchymal stem cells derived from 3 individuals and fibroblasts derived from 3 individuals, and by using them as a template, the expression levels of the above-mentioned 87 genes were examined by semiquantitative RT-PCR. As a result, most genes exhibited no difference in the expression levels. In addition, there were many cases wherein differences in the expression were considered to be caused by individual differences, because differences in the expression levels were observed between mesenchymal stem cells, or fibroblasts, from different individuals.

There were 3 genes (tissue factor pathway inhibitor 2, major histocompatibility complex class2 DR beta 3, serine (or cysteine) proteinase inhibitor) whose high expression in mesenchymal stem cells was confirmed by RT-PCR, and 10 genes (matrixmetalloprotease1, collagenase type XV alpha, CUG triplet repeat RNA binding protein, dermatopontin, protein tyrosine kinase 7, isocitrate dehydrogenase 2, Sam68-like phosphotyrosine protein, C-type lectin superfamily member 2, adrenomedullin, apolopoprotein D) whose low expression in mesenchymal stem cells was confirmed by RT-PCR. By measuring the expression levels of these 13 genes, it has become possible to identify mesenchymal stem cells, which are difficult to be determined, in a short time and at a low cost. In addition, as a result of gene search in the present invention, many genes exhibiting differences in the expression levels between mesenchymal stem cells, or fibroblasts, have been confirmed. These genes serve as a gene marker for detecting mesenchymal stem cells, and can be used for detecting mesenchymal stem cells as microarray, DNA chip or the like by constructing a probe for detecting genes.

### Disclosure of the Invention

The present invention specifically comprises: a gene marker for detecting a mesenchymal stem cell which is a gene having a base sequence shown in SEQ ID NO: 1, 2, 4, 6, 7, 9, 10, 11, 12, 14, 15, 17, 19 or 46 in the sequence listing ("1"), a gene marker for detecting a mesenchymal stem cell which is a gene of INTEGRIN, ALPHA 6 (ITGA6), MRNA (NM_000210); a gene of SOLUTE CARRIER FAMILY 20 (PHOSPHATE TRANSPORTER), MEMBER 1 (NM_005415); a gene of RIBONUCLEOTIDE REDUCTASE M2 POLYPEPTIDE (RRM2), MRNA (NM_001034); a gene of FOLLISTATIN (FST), TRANSCRIPT VARIANT FST317, MRNA (NM_006350); a gene of SPROUTY (DROSOPHILA) HOMOLOG 2 (SPRY2), MRNA (NM_005842); a gene of RAB3B, MEMBER RAS ONCOGENE FAMILY (RAB3B), MRNA (NM_002867); a gene of SOLUTE CARRIER FAMILY 2 (FACILITATED GLUCOSE TRANSPORTER) (NM_006516); a gene of INTERLEUKIN 13 RECEPTOR, ALPHA 2 (IL13RA2), MRNA (NM_000640); a gene of SERINE/THREONINE KINASE 12 (STK12), MRNA (NM_004217) ; a gene of MINICHROMOSOME MAINTENANCE DEFICIENT (S. CEREVISIAE) 5 (CE) (NM_006739); a gene of THYROID HORMONE RECEPTOR INTERACTOR 13 (TRIP13), MRNA (NM_004237); a gene of KINESIN-LIKE 6 (MITOTIC CENTROMERE-ASSOCIATED KINESIN) (K) (NM_006845); a gene of CYCLIN-DEPENDENT KINASE INHIBITOR 3 (CDK2-ASSOCIATED DUAL (NM_005192); a gene of CHROMOSOME CONDENSATION PROTEIN G (HCAP-G), MRNA (NM_022346); a gene of CDC28 PROTEIN KINASE 1 (CKS1), MRNA (NM_001826); a gene of PROTEIN REGULATOR OF CYTOKINESIS 1 (PRC1), MRNA (NM_003981); a gene of CELL DIVISION CYCLE 2, G1 TO S AND G2 TO M (CDC2), MRNA (NM_001786); a gene of CDC20 (CELL DIVISION CYCLE 20, S. CEREVISIAE, HOMOLOG) (CD) (NM_001255); a gene of LIKELY ORTHOLOG OF MATERNAL EMBRYONIC LEUCINE ZIPPER KINAS (NM_014791); a gene of MINICHROMOSOME MAINTENANCE DEFICIENT (S. CEREVISIAE) 7 (M) (NM_005916); a gene of CYCLINA2 (CCNA2), MRNA (NM_001237); a gene of THYMIDINE KINASE 1, SOLUBLE (TK1), MRNA (NM_003258); or a gene of cyclin-dependent kinase inhibitor 2A (melanoma, p16, inhibits CDK4) (CDKN2A), mRNA (NM_000077) ("2"), a gene marker for detecting a mesenchymal stem cell which is a gene of EGF-containing fibulin-like extracellular matrix protein 1 (NM_018894); a gene of Human insulin-like growth factor binding protein 5 (IGFBP5) mRNA (AU132011); a gene of Homo sapiens clone 24775 mRNA sequence (AA402981); proteoglycan 1, secretory granule (AV734015); a gene of insulin-like growth factor binding protein 5 (AA374325); a gene of solute carrier family 21 (organic anion transporter), member 3 (AF085224) ; a gene of transglutaminase 2 (C polypeptide, protein-glutamine-gamma-glutamyl transferase) (AL552373); a gene of coagulation factor II (thrombin) receptor (M62424); a gene of plasminogen activator, urokinase (NM_002658); a gene of tissue inhibitor of metalloproteinase 3 (Sorsby fundus dystrophy, pseudoinflammatory) (W96324); a gene of matrix Gla protein (BF668572); a gene of B-cell CLL/lymphoma 1 (Z23022); a gene of CD74 antigen (invariant polypeptide of major histocompatibility complex, class II antigen-associated) (BG333618); a gene of adducin 3 (gamma) (AL135243); a gene of solute carrier family 16 (monocarboxylic acid transporters), member 4 (NM_004696); a gene of protein S (alpha) (NM_000313); a gene of phosphatidylinositol (4,5) bisphosphate 5-phosphatase homolog; phosphatidylinositol polyphosphate 5-phosphatase type IV (AF187891); a gene of progesterone membrane binding protein (BE858855); a gene of brain-derived neurotrophic factor (X60201); or a gene of apolipoprotein E (BF967316) ("3"), a gene marker for detecting a mesenchymal stem cell which is a gene of HOMO SAPIENS WNT INHIBITORY FACTOR-1 (WIF-1), MRNA(NM_007191); a gene of HOMO SAPIENS HYPOTHETICAL PROTEIN FLJ11175 (FLJ11175), MRNA (NM_018349); a gene of HOMO SAPIENS RHO GDP DISSOCIATION INHIBITOR (GDI) BETA (ARHGDIB), MRNA (NM_001175); a gene of HOMO SAPIENS POTASSIUM INTERMEDIATE/SMALL CONDUCTANCE CALCIUM-ACTIVATE (NM_021614); a gene of HOMO SAPIENS NUCLEAR FACTOR (ERYTHROID-DERIVED 2)-LIKE 3 (NFE2L3), MRNA (NM_004289); a gene of HOMO SAPIENS GS3955 PROTEIN (GS3955), MRNA (NM_021643); a gene of HOMO SAPIENS CDNA 3' END SIMILAR TO SIMILAR TO GLYCOPROTEIN MUC18 (AA302605); a gene of HOMO SAPIENS HYPOTHETICAL PROTEIN FLJ21841 (FLJ21841), MRNA (NN_024609); a gene of HOMO SAPIENS ZINC FINGER PROTEIN 185 (LIM DOMAIN) (ZNF185), MRNA (NK_007150); a gene of SC1 = PUTATIVE TRANS-ACTING FACTOR INVOLVED IN CELL CYCLE CONTROL [HUMAN, MRNA, 24] (S53374); a gene of HOMO SAPIENS NADH DEHYDROGENASE (UBIQUINONE) 1 ALPHA SUBCOMPLEX, 8 (19K) (NM_014222); a gene of HOMO SAPIENS HOMOLOG OF YEAST MCM10; HYPOTHETICAL PROTEIN PR02249 (PRO2) (NK_018518); a gene of HOMO SAPIENS COLLAGEN, TYPE VII, ALPHA 1 (EPIDERMOLYSIS BULLOSA, DYSTRO (NM_000094); a gene of HOMO SAPIENS AUTOCRINE MOTILITY FACTOR RECEPTOR (AMFR), MRNA (NM_001144); a gene of HOMO SAPIENS CLONE 24421 MRNA SEQUENCE /CDS = UNKNOWN /GB = AF070641 /GI = 3283914 /UG = (AF070641); a gene of HOMO SAPIENS MRNA; a gene of CDNA DKFZP586E1621 (FROM CLONE DKFZP586E1621) /CDS = UNKNOWN /GB (AL080235) ("4"), a gene marker for detecting a mesenchymal stem cell which is a gene of HOMO SAPIENS RESERVED (KCNK12), MRNA (NK_022055); a gene of HOMO SAPIENS UBIQUITIN-CONJUGATING ENZYME E2C (UBE2C), MRNA (NM_007019); a gene of HOMO SAPIENS CDNA FLJ10517 FIS, CLONE NT2RP2000812 /CDS = (61,918) /GB = AK001379 /G (AK001379); a gene of HOMO SAPIENS CARTILAGE LINKING PROTEIN 1 (CRTL1), MRNA (NM_001884); a gene of HOMO SAPIENS MRNA; CDNA DKFZP434F2322 (FROM CLONE DKFZP434F2322) /CDS = UNKNOWN /GB (AL133105); a gene of HOMO SAPIENS, CLONE MGC:9549 IMAGE:3857382, MRNA, COMPLETE CDS /CDS = (92,1285) /G (BC012453); a gene of HOMO SAPIENS HYPOTHETICAL PROTEIN DKFZP586J1119 (DKFZP586J1119), MRNA (NK_032270); a gene of HOMO SAPIENS GLIA MATURATION FACTOR, GAMMA (GMFG), MRNA (NM_004877); a gene of HOMO SAPIENS CDNA: FLJ23095 FIS, CLONE LNG07413 /CDS = UNKNOWN /GB = AK026748 /GI = 10 (AK026748); a gene of HOMO SAPIENS HYPOTHETICAL PROTEIN DKFZP762E1312 (DKFZP762E1312), MRNA (NM_018410); a gene of HOMO SAPIENS IROQUOIS-CLASS HOMEODOMAIN PROTEIN (IRX-2A), MRNA (NM_005853); or a gene of HOMO SAPIENS HYPOTHETICAL PROTEIN FLJ10540 (FLJ10540), MRNA (NK_018131) ("5"), a gene marker for detecting a mesenchymal stem cell which is a gene of HOMO SAPIENS MRNA; CDNA DKFZP434C1915 (FROM CLONE DKFZP434C1915); PARTIAL CDS /C (AL137698); a gene of HOMO SAPIENS MINICHROMOSOME MAINTENANCE DEFICIENT (S. CEREVISIAE) 7 (M) (NM_005916); a gene of HOMO SAPIENS DYNEIN, CYTOPLASMIC, INTERMEDIATE POLYPEPTIDE 1 (DNCI1), M (NM_004411); a gene of HUMAN MRNA FOR PROTEIN GENE PRODUCT (PGP) 9.5. (X04741); a gene of HOMO SAPIENS RAD51-INTERACTING PROTEIN (PIR51), MRNA (NM_006479); a gene of HOMO SAPIENS BACULOVIRAL IAP REPEAT-CONTAINING 5 (SURVIVIN) (BIRC5), MR (NM_001168); a gene of HOMO SAPIENS UDP-N-ACETYL-ALPHA-D-GALACTOSAMINE:POLYPEPTIDE N-ACETYLGAL (NM_004482); a gene of HOMO SAPIENS CYCLIN B1 (CCNB1), MRNA (NM_031966); a gene of HOMO SAPIENS FLAP STRUCTURE-SPECIFIC ENDONUCLEASE 1 (FEN1), MRNA (NM_004111); a gene of HOMO SAPIENS SERINE/THREONINE KINASE 15 (STK15), MRNA (NM_003600); a gene of HOMO SAPIENS MAD2 (MITOTIC ARREST DEFICIENT, YEAST, HOMOLOG)-LIKE 1 (MA) (NM_002358); a gene of HOMO SAPIENS NUCLEOLAR PROTEIN ANKT (ANKT), MRNA (NM_018454); a gene of HOMO SAPIENS HSPC150 PROTEIN SIMILAR TO UBIQUITIN-CONJUGATING ENZYME (H) (NM_014176); a gene of HOMO SAPIENS CYTOCHROME P450 RETINOID METABOLIZING PROTEIN (P450RAI-2), (NM_019885); a gene of HOMO SAPIENS NIMA (NEVER IN MITOSIS GENE A)-RELATED KINASE 2 (NEK2), MR (NM_002497); a gene of HOMO SAPIENS BONE MORPHOGENETIC PROTEIN 4 (BMP4), MRNA (NM_001202); a gene of HOMO SAPIENS CDNA FLJ10674 FIS, CLONE NT2RP2006436 /CDS = UNKNOWN /GB = AK001536 /GI (AK001536); or a gene of HOMO SAPIENS CENTROMERE PROTEIN A (17 KD) (CENPA), MRNA (NM_001809) ("6"), a gene marker for detecting a mesenchymal stem cell which is a gene of HOMO SAPIENS MRNA; CDNA DKFZP564P116 (FROM CLONE DKFZP564P116) /CDS = UNKNOWN /GB = A (AL049338); a gene of HOMO SAPIENS TTK PROTEIN KINASE (TTK), MRNA (NM_003318); a gene of HOMO SAPIENS KARYOPHERIN ALPHA 2 (RAG COHORT 1, IMPORTIN ALPHA 1) (KPNA (NM_002266); a gene of HOMO SAPIENS POLYMERASE (DNA DIRECTED), THETA (POLQ), MRNA (NM_006596); a gene of HOMO SAPIENS ETS VARIANT GENE 5 (ETS-RELATED MOLECULE) (ETV5), MRNA (NM_004454); a gene of HOMO SAPIENS TRANSFORMING, ACIDIC COILED-COIL CONTAINING PROTEIN 3 (TAG) (NM_006342); a gene of HOMO SAPIENS KINESIN-LIKE 1 (KNSL1), MRNA (NM_004523); a gene of HOMO SAPIENS CENTROMERE PROTEIN E (312 KD) (CENPE), MRNA (NM_001813); a gene of HOMO SAPIENS CDNA, 3' END /CLONE = IMAGE:1651289 /CLONE_END = 3' /GB = AI12 (AI123815); a gene of HOMO SAPIENS DISTAL-LESS HOMEO BOX 5 (DLX5), MRNA (NM_005221); a gene of HOMO SAPIENS V-MYB AVIAN MYELOBLASTOSIS VIRAL ONCOGENE HOMOLOG-LIKE 2 (NM_002466); or a gene of HOMO SAPIENS SERUM DEPRIVATION RESPONSE (PHOSPHATIDYLSERINE-BINDING PRO (NM_004657) ("7"), a gene marker for detecting a mesenchymal stem cell which is a gene of HOMO SAPIENS HYPOTHETICAL PROTEIN FLJ10604 (FLJ10604), MRNA (NM_018154); a gene of HOMO SAPIENS RIBONUCLEASE HI, LARGE SUBUNIT (RNASEHI), MRNA (NM_006397); a gene of HOMO SAPIENS ANILLIN (DROSOPHILA SCRAPS HOMOLOG), ACTIN BINDING PROTEIN (NM_018685); a gene of HOMO SAPIENS H4 HISTONE FAMILY, MEMBER G (H4FG), MRNA (NM_003542); a gene of HOMO SAPIENS G PROTEIN-COUPLED RECEPTOR 37 (ENDOTHELIN RECEPTOR TYPE B-(NM_005302) HOMO SAPIENS UBIQUITIN CARRIER PROTEIN (E2-EPF), MRNA (NM_014501); a gene of HOMO SAPIENS, SIMILAR TO TUMOR DIFFERENTIALLY EXPRESSED 1, CLONE IMAGE:3639252, (BC007375); a gene of HOMO SAPIENS HYPOTHETICAL PROTEIN MGC2577 (MGC2577), MRNA (NM_031299); a gene of HOMO SAPIENS HYPOTHETICAL PROTEIN FLJ13912 (FLJ13912), MRNA (NM_022770); a gene of HOMO SAPIENS ANNEXIN A3 (ANXA3), MRNA (NM_005139); a gene of HOMO SAPIENS STATHMIN 1/ONCOPROTEIN 18 (STMN1), MRNA (NM_005563) HOMO SAPIENS, SIMILAR TO RIBOSOMAL PROTEIN L39, CLONE MGC:20168 IMAGE:4555759, M (BC012328); or a gene of HOMO SAPIENS POLO (DROSOPHIA)-LIKE KINASE (PLK), MRNA (NM_005030) ("8"), a gene marker for detecting a mesenchymal stem cell which is a gene of HOMO SAPIENS PROTEASOME (PROSOME, MACROPAIN) 26S SUBUNIT, ATPASE, 5 (PS) (NM_002805); a gene of HOMO SAPIENS EUKARYOTIC TRANSLATION INITIATION FACTOR 4 GAMMA, 2 (EIF4G) (NM_001418); a gene of HOMO SAPIENS DIHYDROPYRIMIDINASE-LIKE 3 (DPYSL3), MRNA) (NM_001387); a gene of HOMO SAPIENS ADAPTOR-RELATED PROTEIN COMPLEX 1, SIGMA 2 SUBUNIT (AP1S2) (NM_003916); a gene of HOMO SAPIENS PLECTIN 1, INTERMEDIATE FILAMENT BINDING PROTEIN, 500 KD (P) (NM_000445); a gene of HOMO SAPIENS HETEROGENEOUS NUCLEAR RIBONUCLEOPROTEIN U (SCAFFOLD ATTAC (NM_031844); a gene of HOMO SAPIENS HYPOTHETICAL PROTEIN MGC5306 (MGC5306), MRNA (NM_024116); a gene of HOMO SAPIENS MHC CLASS I POLYPEPTIDE-RELATED SEQUENCE A (MICA), MRNA (NM_000247); a gene of HOMO SAPIENS PROTEIN ASSOCIATED WITH PRK1 (AWP1), MRNA (NM_019006); a gene of HOMO SAPIENS PROGESTERONE RECEPTOR MEMBRANE COMPONENT 2 (PGRMC2), MRNA (NM_006320); a gene of HOMO SAPIENS POLYGLUTAMINE BINDING PROTEIN 1 (PQBP1), MRNA (NM_005710); a gene of HOMO SAPIENS S-ADENOSYLMETHIONINE DECARBOXYLASE 1 (AMD1), MRNA (NM_001634); HOMO SAPIENS INTERFERON-INDUCED, HEPATITIS C-ASSOCIATED MICROTUBULAR AG (NM_006417); a gene of HOMO SAPIENS PROTEIN KINASE, AMP-ACTIVATED, GAMMA 1 NON-CATALYTIC SUBUN (NM_002733); HOMO SAPIENS PROCOLLAGEN-PROLINE, 2-OXOGLUTARATE 4-DIOXYGENASE (PROLINE (NM_004199); a gene of HOMO SAPIENS ENDOTHELIAL DIFFERENTIATION, LYSOPHOSPHATIDICACIDG-PROTE (NM_012152); or a gene of HOMO SAPIENS KIAA0127 GENE PRODUCT (KIAA0127), MRNA (NM_014755) ("9"), a probe for detecting a mesenchymal stem cell marker gene having a DNA sequence which hybridizes with the marker gene for detecting a mesenchymal stem cell according to any one of "1" to "9" under a stringent condition ("10"), the probe for detecting a mesenchymal stem cell marker gene according to "10" , which comprises whole or part of an antisense strand of the base sequence according to any one of "1" to "9" ("11"), a microarray or a DNA chip for detecting a mesenchymal stem cell marker gene, wherein at least one of the DNA according to "10" or "11" is immobilized ("12"), the microarray or the DNA chip for detecting a mesenchymal stem cell marker gene according to "12", wherein a probe for detecting two or more genes in a group of genes having a base sequence shown in SEQ ID NO: 1, 2, 4, 6, 7, 9, 10, 11, 12, 14, 15, 17, 19 or 46 in the sequence listing and a group of the genes according to any one of "2" to "9" is immobilized ("13"), a polypeptide marker for detecting a mesenchymal stem cell which is a polypeptide having an amino acid sequence shown in SEQ ID NO: 3, 5, 8, 13, 16 or 18 in the sequence listing ("14"), an antibody which is induced by using the polypeptide according to "14" and which specifically binds to the polypeptide ("15"), the antibody according to "15", which is a monoclonal antibody ("16"), and the antibody according to "15" , which is a polyclonal antibody ("17").

The present invention also comprises: a method for distinguishing a mesenchymal stem cell wherein expression of the mesenchymal stem cell marker gene according to any one of "1" to "9" in a test cell is detected ("18"), the method for distinguishing a mesenchymal stem cell according to "18", wherein the expression of the mesenchymal stem cell marker gene is detected by using Northern blotting ("19"), the method for distinguishingamesenchymal stem cell according to "18", wherein the expression of the mesenchymal stem cell marker gene is detected by using the probe for detecting a mesenchymal stem cell marker gene according to "10" or "11" ("20"), the method for distinguishing a mesenchymal stem cell according to "18", wherein the expression of the mesenchymal stem cell marker gene is detected by using the microarray or the DNA chip for detecting a mesenchymal stem cell marker gene according to "12" or "13" ("21"), a method for distinguishing a mesenchymal stem cell, wherein the detection of the expression of the mesenchymal stem cell marker gene according to any one of "18" to "21" comprises use of quantitative or semiquantitative PCR ("22"), and the method for distinguishing a mesenchymal stem cell according to "22", wherein the use of quantitative or semiquantitative PCR is RT-PCR method ("23").

The present invention further comprises: an RT-PCR primer for amplifying a mesenchymal stem cell marker gene which comprises a sense primer having a base sequence shown in SEQ ID NO: 20 in the sequence listing, and an antisense primer having a base sequence shown in SEQ ID NO: 21 in the sequence listing; a sense primer having a base sequence shown in SEQ ID NO: 22 in the sequence listing, and an antisense primer having a base sequence shown in SEQ ID NO: 23 in the sequence listing; a sense primer having a base sequence shown in SEQ ID NO: 24 in the sequence listing, and an antisense primer having a base sequence shown in SEQ ID NO: 25 in the sequence listing; a sense primer having a base sequence shown in SEQ ID NO: 26 in the sequence listing, and an antisense primer having a base sequence shown in SEQ ID NO: 27 in the sequence listing; a sense primer having a base sequence shown in SEQ ID NO: 28 in the sequence listing, and an antisense primer having a base sequence shown in SEQ ID NO: 29 in the sequence listing; a sense primer having a base sequence shown in SEQ ID NO: 30 in the sequence listing, and an antisense primer having a base sequence shown in SEQ ID NO: 31 in the sequence listing; a sense primer having a base sequence shown in SEQ ID NO: 32 in the sequence listing, and an antisense primer having a base sequence shown in SEQ ID NO: 33 in the sequence listing; a sense primer having a base sequence shown in SEQ ID NO: 34 in the sequence listing, and an antisense primer having a base sequence shown in SEQ ID NO: 35 in the sequence listing; a sense primer having a base sequence shown in SEQ ID NO: 36 in the sequence listing, and an antisense primer having a base sequence shown in SEQ ID NO: 37 in the sequence listing; a sense primer having a base sequence shown in SEQ ID NO: 38 in the sequence listing, and an antisense primer having a base sequence shown in SEQ ID NO: 39 in the sequence listing; a sense primer having a base sequence shown in SEQ ID NO: 40 in the sequence listing, and an antisense primer having a base sequence shown in SEQ ID NO: 41 in the sequence listing; a sense primer having a base sequence shown in SEQ ID NO: 42 in the sequence listing, and an antisense primer having a base sequence shown in SEQ ID NO: 43 in the sequence listing; or a sense primer having a base sequence shown in SEQ ID NO: 44 in the sequence listing, and an antisense primer having a base sequence shown in SEQ ID NO: 45 in the sequence listing ("24"), a real time PCR primer for amplifying a mesenchymal stem cell marker gene which comprises a sense primer having a base sequence shown in SEQ ID NO: 47 in the sequence listing, and an antisense primer having a base sequence shown in SEQ ID NO: 48 in the sequence listing; a sense primer having a base sequence shown in SEQ ID NO: 49 in the sequence listing, and an antisense primer having a base sequence shown in SEQ ID NO: 50 in the sequence listing; a sense primer having a base sequence shown in SEQ ID NO: 51 in the sequence listing, and an antisense primer having a base sequence shown in SEQ ID NO: 52 in the sequence listing; a sense primer having a base sequence shown in SEQ ID NO: 53 in the sequence listing, and an antisense primer having a base sequence shown in SEQ ID NO: 54 in the sequence listing; a sense primer having a base sequence shown in SEQ ID NO: 55 in the sequence listing, and an antisense primer having a base sequence shown in SEQ ID NO: 56 in the sequence listing; a sense primer having a base sequence shown in SEQ ID NO: 57 in the sequence listing, and an antisense primer having a base sequence shown in SEQ ID NO: 58 in the sequence listing; a sense primer having a base sequence shown in SEQ ID NO: 59 in the sequence listing, and an antisense primer having a base sequence shown in SEQ ID NO: 60 in the sequence listing; a sense primer having a base sequence shown in SEQ ID NO: 61 in the sequence listing, and an antisense primer having a base sequence shown in SEQ ID NO: 62 in the sequence listing; a sense primer having a base sequence shown in SEQ ID NO: 63 in the sequence listing, and an antisense primer having a base sequence shown in SEQ ID NO: 64 in the sequence listing; a sense primer having a base sequence shown in SEQ ID NO: 65 in the sequence listing, and an antisense primer having a base sequence shown in SEQ ID NO: 66 in the sequence listing; a sense primer having a base sequence shown in SEQ ID NO: 67 in the sequence listing, and an antisense primer having a base sequence shown in SEQ ID NO: 68 in the sequence listing; or a sense primer having a base sequence shown in SEQ ID NO: 69 in the sequence listing, and an antisense primer having a base sequence shown in SEQ ID NO: 70 in the sequence listing ("25"), and an RT-PCR primer for amplifying a mesenchymal stem cell marker gene which comprises a sense primer having a base sequence shown in SEQ ID NO: 71 in the sequence listing, and an antisense primer having a base sequence shown in SEQ ID NO: 72 in the sequence listing; a sense primer having a base sequence shown in SEQ ID NO: 73 in the sequence listing, and an antisense primer having a base sequence shown in SEQ ID NO: 74 in the sequence listing; a sense primer having a base sequence shown in SEQ ID NO: 75 in the sequence listing, and an antisense primer having a base sequence shown in SEQ ID NO: 76 in the sequence listing; a sense primer having a base sequence shown in SEQ ID NO: 77 in the sequence listing, and an antisense primer having a base sequence shown in SEQ ID NO: 78 in the sequence listing; a sense primer having a base sequence shown in SEQ ID NO: 79 in the sequence listing, and an antisense primer having a base sequence shown in SEQ ID NO: 80 in the sequence listing; a sense primer having a base sequence shown in SEQ ID NO: 81 in the sequence listing, and an antisense primer having a base sequence shown in SEQ ID NO: 82 in the sequence listing; a sense primer having a base sequence shown in SEQ ID NO: 83 in the sequence listing, and an antisense primer having a base sequence shown in SEQ ID NO: 84 in the sequence listing; a sense primer having a base sequence shown in SEQ ID NO: 85 in the sequence listing, and an antisense primer having a base sequence shown in SEQ ID NO: 86 in the sequence listing; a sense primer having a base sequence shown in SEQ ID NO: 87 in the sequence listing, and an antisense primer having a base sequence shown in SEQ ID NO: 88 in the sequence listing; a sense primer having a base sequence shown in SEQ ID NO: 89 in the sequence listing, and an antisense primer having a base sequence shown in SEQ ID NO: 90 in the sequence listing; a sense primer having a base sequence shown in SEQ ID NO: 91 in the sequence listing, and an antisense primer having a base sequence shown in SEQ ID NO: 92 in the sequence listing; a sense primer having a base sequence shown in SEQ ID NO: 93 in the sequence listing, and an antisense primer having a base sequence shown in SEQ ID NO: 94 in the sequence listing; a sense primer having a base sequence shown in SEQ ID NO: 95 in the sequence listing, and an antisense primer having a base sequence shown in SEQ ID NO: 96 in the sequence listing; a sense primer having a base sequence shown in SEQ ID NO: 97 in the sequence listing, and an antisense primer having a base sequence shown in SEQ ID NO: 98 in the sequence listing; a sense primer having a base sequence shown in SEQ ID NO: 99 in the sequence listing, and an antisense primer having a base sequence shown in SEQ ID NO: 100 in the sequence listing; a sense primer having a base sequence shown in SEQ ID NO: 101 in the sequence listing, and an antisense primer having a base sequence shown in SEQ ID NO: 102 in the sequence listing; a sense primer having a base sequence shown in SEQ ID NO: 103 in the sequence listing, and an antisense primer having a base sequence shown in SEQ ID NO: 104 in the sequence listing; a sense primer having a base sequence shown in SEQ ID NO: 105 in the sequence listing, and an antisense primer having a base sequence shown in SEQ ID NO: 106 in the sequence listing; a sense primer having a base sequence shown in SEQ ID NO: 107 in the sequence listing, and an antisense primer having a base sequence shown in SEQ ID NO: 108 in the sequence listing; a sense primer having a base sequence shown in SEQ ID NO: 109 in the sequence listing, and an antisense primer having a base sequence shown in SEQ ID NO: 110 in the sequence listing; a sense primer having a base sequence shown in SEQ ID NO: 111 in the sequence listing, and an antisense primer having a base sequence shown in SEQ ID NO: 112 in the sequence listing; a sense primer having a base sequence shown in SEQ ID NO: 113 in the sequence listing, and an antisense primer having a base sequence shown in SEQ ID NO: 114 in the sequence listing; or a sense primer having a base sequence shown in SEQ ID NO: 115 in the sequence listing, and an antisense primer having a base sequence shown in SEQ ID NO: 116 in the sequence listing ("26"). The present invention still further comprises: the method for distinguishing a mesenchymal stem cell according to "18", wherein a gene in a test cell is amplified by using at least one pair of primers comprising the sense primer and the antisense primer according to any one of "24" to "26", and the amplified gene is detected by using the probe for detecting a mesenchymal stem cell marker gene according to "10" or "11" ("27"), a method for distinguishing a mesenchymal stem cell, wherein expression of a mesenchymal stem cell marker polypeptide in a test cell is detected by using the antibody according to any one of "15" to "17" ("28"), a method for distinguishing a mesenchymal stem cell, wherein expression of one or more genes in a group of mesenchymal stem cell marker genes comprising SEQ ID NOs: 1, 11 and 19 in the sequence listing, and expression of one or more genes in a group of mesenchymal stem cell marker genes comprising SEQ ID NOs: 2, 4, 6, 7, 9, 10, 12, 14, 15 and 17 in the sequence listing, in a test cell, are evaluated in combination ("29"), a method for distinguishing and separating a mesenchymal stem cell, wherein expression of a marker gene for detecting a mesenchymal stem cell and/or a marker polypeptide for detecting a mesenchymal stem cell in a test cell is detected by using the probe for detecting a mesenchymal stem cell marker gene according to "10" or "11" and/or the antibody according to any one of "15" to "17", and the distinguished mesenchymal stem cell is separated ("30"), the method for distinguishing and separating a mesenchymal stem cell according to "30" , wherein a mesenchymal stem cell in a test cell is labeled by a fluorescent antibody method using the antibody according to any one of "15" to "17", and the labeled mesenchymal stem cell is separated ("31"), and a kit for distinguishing a mesenchymal stem cell which comprises at least one of the probe for detecting a mesenchymal stem cell marker gene according to "10" or "11", the microarray or the DNA chip for detecting a mesenchymal stem cell marker gene according to "12" or "13" in which the probe is immobilized, and the antibody according to any one of "15" to "17" ("32").

### Brief Description of Drawings

Fig. 1 shows genes exhibiting considerable individual differences between human mesenchymal stem cells or human fibroblasts, according to the semiquantitative RT-PCR method in the Examples of the present invention.
Fig. 2 shows genes exhibiting high expression in MSC, according to the semiquantitative RT-PCR in the Examples of the present invention.
Fig. 3 shows genes exhibiting low expression in MSC, according to the semiquantitative RT-PCR in the Examples of the present invention.
Fig. 4 shows the result of confirmation of the expression levels of 13 genes confirmed to exhibit high/low expression in mesenchymal stem cells, by using semiquantitative RT-PCR with total RNA extracted from mesenchymal stem cells derived from 7 individuals and fibroblasts derived from 4 individuals as a template.
Fig. 5 shows the test result of the expression state of the marker gene of the present invention in mesenchymal stem cells and fibroblasts in the Examples of the present invention.
Fig. 6 shows the expression state of differential gene of each marker gene in mesenchymal stem cells and osteoblasts in the Examples of the present invention.
Fig. 7 shows the expression state of differential gene of each marker gene in mesenchymal stem cells and osteoblasts in the Examples of the present invention.
Fig. 8 shows the expression state of differential gene of each marker gene in mesenchymal stem cells and osteoblasts in the Examples of the present invention.
Fig. 9 shows the expression state of each marker gene in mesenchymal stem cells and human fibroblasts in the Examples of the present invention.
Fig. 10 shows the expression state of each marker gene in mesenchymal stem cells and human fibroblasts in the Examples of the present invention.
Fig. 11 shows the result of flow cytometric measurement of the expression of mesenchymal stem cell DR in human cells in the Examples of the present invention.
Fig. 12 shows the measurement result of the expression state of each gene in inducing differentiation of mesenchymal stem cells into osteoblasts in the Examples of the present invention.
Fig. 13 shows the changes in gene expression of each marker gene from day 4 to day 28 in the induction of osteogenic differentiation in the Examples of the present invention.
Fig. 14 shows the changes in gene expression of each marker gene from day 4 to day 28 in the induction of osteogenic differentiation in the Examples of the present invention.
Fig. 15 shows the changes in gene expression of each marker gene from day 4 to day 28 in the induction of osteogenic differentiation in the Examples of the present invention.
Fig. 16 shows the changes in gene expression of each marker gene from day 4 to day 28 in the induction of osteogenic differentiation in the Examples of the present invention.

### Best Mode of Carrying Out the Invention

The present invention comprises distinguishing mesenchymal stem cells by detecting the expression of a marker gene for detecting mesenchymal stem cells or a marker polypeptide for detecting mesenchymal stem cells, which are expressed specifically in mesenchymal stem cells. In the present invention, a gene which can serve as a gene marker for detecting mesenchymal stem cells is a gene having a base sequence shown in SEQ ID NO: 1, 2, 4, 6, 7, 9, 10, 11, 12, 14, 15, 17 or 19 in the sequence listing.

Further, in the present invention, a polypeptide which serves as a polypeptide marker for detecting mesenchymal stem cells is a polypeptide having an amino acid sequence shown in SEQ ID NO: 3, 5, 8, 13, 16 or 18 in the sequence listing.

The genes shown in SEQ ID NOs: 1, 2, 4, 6, 7, 9, 10, 11, 12, 14, 15, 17, 19 and 46 in the sequence listing are genes of [serine (or cysteine) proteinase inhibitor], [adrenomedullin], [apolopoprotein D], [collagenase type XV alpha 1], [CUG triplet repeat RNA binding protein2], [dermatopontin], [isocitrate dehydrogenase 2], [major histocompatibility complex class2, DR beta 3], [protein tyrosine kinase 7], [Sam68-like phosphotyrosine protein], [C-type lectin superfamily member 2], [matrix metalloprotease 1], [tissue factor pathway inhibitor 2], [major histocompatibility complex DR alpha], respectively.

The DNA sequence information of the marker genes for detecting mesenchymal stem cells of the present invention can be approached in NCBI gene database with Accession Nos. AI133613(SEQ ID NO: 1), NM_001124(SEQ ID NO: 2), NM_001647(SEQ IDNO: 4), L01697(SEQIDNO: 6), U69546 (SEQ ID NO: 7), AW016451(SEQ ID NO: 9), AL545953(SEQ ID NO: 10), BF732822(SEQ ID NO: 11), AL157486(SEQ ID NO: 12), AA112001(SEQ ID NO: 14), XM_006626(SEQ ID NO: 15), NM_002421(SEQ ID NO: 17), AL550357(SEQ ID NO: 19), and BF795929(SEQ ID NO: 46), respectively.

In addition, as genes which are confirmed in the present invention that they exhibit higher expression in mesenchymal stem cells than in fibroblasts, the following is exemplified:

A gene marker for detecting mesenchymal stem cells which is: a gene of INTEGRIN, ALPHA 6 (ITGA6), MRNA (NM_000210); a gene of SOLUTE CARRIER FAMILY 20 (PHOSPHATE TRANSPORTER), MEMBER 1 (NM_005415) ; a gene of RIBONUCLEOTIDE REDUCTASE M2 POLYPEPTIDE (RRM2), MRNA(NM_001034); a gene of FOLLISTATIN (FST), TRANSCRIPT VARIANT FST317, MRNA (NM_006350); a gene of SPROUTY (DROSOPHILA) HOMOLOG 2 (SPRY2), MRNA (NM_005842); a gene of RAB3B, MEMBER RAS ONCOGENE FAMILY (RAB3B), MRNA (NM_002867): a gene of SOLUTE CARRIER FAMILY 2 (FACILITATED GLUCOSE TRANSPORTER) (NM_006516); a gene of INTERLEUKIN 13 RECEPTOR, ALPHA 2 (IL13RA2), MRNA (NM_000640); a gene of SERINE/THREONINE KINASE 12 (STK12), MRNA (NM_004217); a gene of MINICHROMOSOME MAINTENANCE DEFICIENT (S. CEREVISIAE) 5 (CE) (NM_006739); a gene of THYROID HORMONE RECEPTOR INTERACTOR 13 (TRIP13), MRNA (NM_004237); a gene of KINESIN-LIKE 6 (MITOTIC CENTROMERE-ASSOCIATED KINESIN) (K) (NM_006845); a gene of CYCLIN-DEPENDENT KINASE INHIBITOR 3 (CDK2-ASSOCIATED DUAL (NM_005192); a gene of CHROMOSOME CONDENSATION PROTEIN G (HCAP-G), MRNA (NM_022346); a gene of CDC28 PROTEINKINASE 1 (CKS1), MRNA (NM_001826); agene of PROTEIN REGULATOR OF CYTOKINESIS 1 (PRC1), MRNA (NM_003981); a gene of CELL DIVISIONCYCLE 2, G1 TO SANDG2 TOM (CDC2), MRNA (NM_001786) ; a gene of CDC20 (CELL DIVISION CYCLE 20, S. CEREVISIAE, HOMOLOG) (CD) (NM_001255): a gene of LIKELY ORTHOLOG OF MATERNAL EMBRYONIC LEUCINE ZIPPER KINAS (NM_014791); a gene of MINICHROMOSOME MAINTENANCE DEFICIENT (S. CEREVISIAE) 7 (M) (NM_005916); a gene of CYCLINA2 (CCNA2), MRNA (NM_001237); a gene of THYMIDINE KINASE 1, SOLUBLE (TK1), MRNA (NM_003258); or a gene of cyclin-dependent kinase inhibitor 2A (melanoma, p16, inhibits CDK4) (CDKN2A), mRNA (NM_000077).

A gene of EGF-containing fibulin-like extracellular matrix protein 1 (NM_018894); a gene of Human insulin-like growth factor binding protein 5 (IGFBP5) mRNA (AU132011); a gene of Homo sapiens clone 24775 mRNA sequence (AA402981); proteoglycan 1, secretory granule (AV734015); a gene of insulin-like growth factor binding protein 5 (AA374325); a gene of solute carrier family 21 (organic anion transporter), member 3 (AF085224); a gene of transglutaminase 2 (C polypeptide, protein-glutamine-gamma-glutamyl transferase) (AL552373); a gene of coagulation factor II (thrombin) receptor (M62424); a gene of plasminogen activator, urokinase (NM_002658); a gene of tissue inhibitor of metalloproteinase 3 (Sorsby fundus dystrophy, pseudoinflammatory) (W96324); a gene of matrix Gla protein (BF668572); a gene of B-cell CLL/lymphoma 1(Z23022); a gene of CD74 antigen (invariant polypeptide of major histocompatibility complex, class II antigen-associated) (BG333618); a gene of adducin 3 (gamma) (AL135243); a gene of solute carrier family 16 (monocarboxylic acid transporters), member 4 (NM_004696); a gene of protein S (alpha)(NM_000313); a gene of phosphatidylinositol (4,5) bisphosphate 5-phosphatase homolog; phosphatidylinositol polyphosphate 5-phosphatase type IV(AF187891); a gene of progesterone membrane binding protein (BE858855); a gene of brain-derived neurotrophic factor (X60201); or a gene of apolipoprotein E (BF967316).

A gene of HOMO SAPIENS WNT INHIBITORY FACTOR-1 (WIF-1), MRNA (NM_007191); a gene of HOMO SAPIENS HYPOTHETICAL PROTEIN FLJ11175 (FLJ11175), MRNA(NM_018349); a gene of HOMO SAPIENS RHO GDP DISSOCIATION INHIBITOR (GDI) BETA (ARHGDIB), MRNA (NM_001175): a gene of HOMO SAPIENS POTASSIUM INTERMEDIATE/SMALL CONDUCTANCE CALCIUM-ACTIVATE(NM_021614); a gene of HOMO SAPIENS NUCLEAR FACTOR (ERYTHROID-DERIVED 2)-LIKE 3 (NFE2L3), MRNA (NM_004289); a gene of HOMO SAPIENS GS3955 PROTEIN (GS3955), MRNA (NM_021643); a gene of HOMO SAPIENS CDNA 3' END SIMILAR TO SIMILAR TO GLYCOPROTEIN MUC18 (AA302605); a gene of HOMO SAPIENS HYPOTHETICAL PROTEIN FLJ21841 (FLJ21841), MRNA (NM_024609); a gene of HOMO SAPIENS ZINC FINGER PROTEIN 185 (LIM DOMAIN) (ZNF185), MRNA (NM_007150); a gene of SC1 = PUTATIVE TRANS-ACTING FACTOR INVOLVED IN CELL CYCLE CONTROL [HUMAN, MRNA, 24] (S53374); a gene of HOMO SAPIENS NADH DEHYDROGENASE (UBIQUINONE) 1 ALPHA SUBCOMPLEX, 8 (19K) (NM_014222); a gene of HOMO SAPIENS HOMOLOG OF YEAST MCM10; HYPOTHETICAL PROTEIN PRO2249 (PRO2) (NM_018518); a gene of HOMO SAPIENS COLLAGEN, TYPE VII, ALPHA 1 (EPIDERMOLYSIS BULLOSA, DYSTRO (NM_000094); a gene of HOMO SAPIENS AUTOCRINE MOTILITY FACTOR RECEPTOR (AMFR), MRNA (NM_001144); a gene of HOMO SAPIENS CLONE 24421 MRNA SEQUENCE /CDS = UNKNOWN /GB = AF070641 /GI = 3283914 /UG = (AF070641); a gene of HOMO SAPIENS MRNA; a gene of CDNA DKFZP586E1621 (FROM CLONE DKFZP586E1621) /CDS = UNKNOWN /GB(AL080235).

Ageneof HOMO SAPIENS RESERVED (KCNK12), MRNA (NM_022055); a gene of HOMO SAPIENS UBIQUITIN-CONJUGATING ENZYME E2C (UBE2C), MRNA (NM_007019); a gene of HOMO SAPIENS CDNA FLJ10517 FIS, CLONE NT2RP2000812 /CDS = (61,918) /GB = AK001379 /G(AK001379); a gene of HOMO SAPIENS CARTILAGE LINKING PROTEIN 1 (CRTL1), MRNA (NM_001884); a gene of HOMO SAPIENS MRNA; CDNA DKFZP434F2322 (FROM CLONE DKFZP434F2322) /CDS = UNKNOWN /GB (AL133105); a gene of HOMO SAPIENS, CLONE MGC:9549 IMAGE:3857382, MRNA, COMPLETE CDS /CDS = (92,1285) /G (BC012453); a gene of HOMO SAPIENS HYPOTHETICAL PROTEIN DKFZP586J1119 (DKFZP586J1119), MRNA (NM_032270); a gene of HOMO SAPIENS GLIAMATURATION FACTOR, GAMMA (GMFG), MRNA (NM_004877); a gene of HOMO SAPIENS CDNA: FLJ23095 FIS, CLONE LNG07413 /CDS = UNKNOWN /GB = AK026748 /GI = 10 (AK026748); a gene of HOMO SAPIENS HYPOTHETICAL PROTEIN DKFZP762E1312 (DKFZP762E1312), MRNA (NM_018410); a gene of HOMO SAPIENS IROQUOIS-CLASS HOMEODOMAIN PROTEIN (IRX-2A), MRNA (NM_005853); or a gene of HOMO SAPIENS HYPOTHETICAL PROTEIN FLJ10540 (FLJ10540), MRNA (NM_018131).

A gene of HOMO SAPIENS MRNA; CDNA DKFZP434C1915 (FROM CLONE DKFZP434C1915); PARTIAL CDS /C (AL137698); a gene of HOMO SAPIENS MINICHROMOSOME MAINTENANCE DEFICIENT (S. CEREVISIAE) 7 (M) (NM_005916); a gene of HOMO SAPIENS DYNEIN, CYTOPLASMIC, INTERMEDIATE POLYPEPTIDE 1 (DNCI1), M (NM_004411); a gene of HUMAN MRNA FOR PROTEIN GENE PRODUCT (PGP) 9.5. (X04741); a gene of HOMO SAPIENS RAD51-INTERACTING PROTEIN (PIR51), MRNA (NM_006479); a gene of HOMO SAPIENS BACULOVIRAL IAP REPEAT-CONTAINING 5 (SURVIVIN) (BIRC5), MR (NM_001168); a gene of HOMO SAPIENS UDP-N-ACETYL-ALPHA-D-GALACTOSAMINE: POLYPEPTIDE N-ACETYLGAL (NM_004482); a gene of HOMO SAPIENS CYCLIN B1 (CCNB1), MRNA (NM_031966); a gene of HOMO SAPIENS FLAP STRUCTURE-SPECIFIC ENDONUCLEASE 1 (FEN1), MRNA (NM_004111); a gene of HOMO SAPIENS SERINE/THREONINE KINASE 15 (STK15), MRNA (NM_003600); a gene of HOMO SAPIENS MAD2 (MITOTIC ARREST DEFICIENT, YEAST, HOMOLOG)-LIKE 1 (MA) (NM_002358); a gene of HOMO SAPIENS NUCLEOLAR PROTEIN ANKT (ANKT), MRNA (NM_018454); a gene of HOMO SAPIENS HSPC150 PROTEIN SIMILAR TO UBIQUITIN-CONJUGATING ENZYME (H) (NM_014176); a gene of HOMO SAPIENS CYTOCHROME P450 RETINOID METABOLIZING PROTEIN (P450RAI-2), (NM_019885); a gene of HOMO SAPIENS NIMA (NEVER IN MITOSIS GENE A)-RELATED KINASE 2 (NEK2), MR (NM_002497); a gene of HOMO SAPIENS BONE MORPHOGENETIC PROTEIN 4 (BMP4), MRNA (NM_001202); a gene of HOMO SAPIENS CDNA FLJ10674 FIS, CLONE NT2RP2006436 /CDS = UNKNOWN /GB = AK001536 /GI (AK001536); or a gene of HOMO SAPIENS CENTROMERE PROTEIN A (17 KD) (CENPA), MRNA (NM_001809).

A gene of HOMO SAPIENS MRNA; CDNA DKFZP564P116 (FROM CLONE DKFZP564P116) /CDS = UNKNOWN /GB = A (AL049338); a gene of HOMO SAPIENS TTK PROTEIN KINASE (TTK), MRNA (NM_003318); a gene of HOMO SAPIENS KARYOPHERIN ALPHA 2 (RAG COHORT 1, IMPORTIN ALPHA 1) (KPNA (NM_002266); a gene of HOMO SAPIENS POLYMERASE (DNA DIRECTED), THETA (POLQ), MRNA (NM_006596); a gene of HOMO SAPIENS ETS VARIANT GENE 5 (ETS-RELATED MOLECULE) (ETV5), MRNA (NM_004454); a gene of HOMO SAPIENS TRANSFORMING, ACIDIC COILED-COIL CONTAINING PROTEIN 3 (TAC) (NM_006342); a gene of HOMO SAPIENS KINESIN-LIKE 1 (KNSL1), MRNA (NM_004523); a gene of HOMO SAPIENS CENTROMERE PROTEIN E (312 KD) (CENPE), MRNA (NM_001813); a gene of HOMO SAPIENS CDNA, 3' END /CLONE = IMAGE: 1651289 /CLONE_END = 3' /GB = AI12 (AI123815) ; a gene of HOMO SAPIENS DISTAL-LESS HOMEO BOX 5 (DLX5), MRNA (NM_005221); a gene of HOMO SAPIENS V-MYB AVIAN MYELOBLASTOSIS VIRAL ONCOGENE HOMOLOG-LIKE 2 (NM_002466); or a gene of HOMO SAPIENS SERUM DEPRIVATION RESPONSE (PHOSPHATIDYLSERINE-BINDING PRO (NM_004657).

A gene of HOMO SAPIENS HYPOTHETICAL PROTEIN FLJ10604 (FLJ10604), MRNA(NM_018154); a gene of HOMO SAPIENS RIBONUCLEASE HI, LARGE SUBUNIT (RNASEHI), MRNA (NM_006397); a gene of HOMO SAPIENS ANILLIN (DROSOPHILA SCRAPS HOMOLOG), ACTIN BINDING PROTEIN (NM_018685); a gene of HOMO SAPIENS H4 HISTONE FAMILY, MEMBER G (H4FG), MRNA (NM_003542); a gene of HOMO SAPIENS G PROTEIN-COUPLED RECEPTOR 37 (ENDOTHELIN RECEPTOR TYPE B-(NM_005302) HOMO SAPIENS UBIQUITIN CARRIER PROTEIN (E2-EPF), MRNA (NM_014501); a gene of HOMO SAPIENS, SIMILAR TO TUMOR DIFFERENTIALLY EXPRESSED 1, CLONE IMAGE:3639252, (BC007375); a gene of HOMO SAPIENS HYPOTHETICAL PROTEIN MGC2577 (MGC2577), MRNA(NM_031299); a gene of HOMO SAPIENS HYPOTHETICAL PROTEIN FLJ13912 (FLJ13912), MRNA (NM_022770); a gene of HOMO SAPIENS ANNEXIN A3 (ANXA3), MRNA (NM_005139); a gene of HOMO SAPIENS STATHMIN1/ONCOPROTEIN 18 (STMN1), MRNA (NM_005563) HOMO SAPIENS, SIMILAR TO RIBOSOMAL PROTEIN L39,CLONE MGC:20168IMAGE:4555759, M(BC012328); a gene of HOMO SAPIENS POLO (DROSOPHIA)-LIKE KINASE (PLK), MRNA (NM_005030).

Further, as genes which are confirmed in the present invention that they exhibit no or extremely lower expression in mesenchymal stem cells than in fibroblasts, the following is exemplified:

A gene marker for detecting mesenchymal stem cells which is: a gene of HOMO SAPIENS PROTEASOME (PROSOME, MACROPAIN) 26S SUBUNIT, ATPASE, 5 (PS) (NM_002805); a gene of HOMO SAPIENS EUKARYOTIC TRANSLATION INITIATION FACTOR 4 GAMMA, 2 (EIF4G) (NM_001418); a gene of HOMO SAPIENS DIHYDROPYRIMIDINASE-LIKE 3 (DPYSL3), MRNA) (NM_001387); a gene of HOMO SAPIENS ADAPTOR-RELATED PROTEIN COMPLEX 1, SIGMA 2 SUBUNIT (AP1S2) (NM_003916); a gene of HOMO SAPIENS PLECTIN 1, INTERMEDIATE FILAMENT BINDING PROTEIN, 500 KD (P) (NM_000445); a gene of HOMO SAPIENS HETEROGENEOUS NUCLEAR RIBONUCLEOPROTEIN U (SCAFFOLD ATTAC (NM_031844); a gene of HOMO SAPIENS HYPOTHETICAL PROTEIN MGC5306 (MGC5306), MRNA (NM_024116); a gene of HOMO SAPIENS MHC CLASS I POLYPEPTIDE-RELATED SEQUENCE A (MICA), MRNA (NM_000247); a gene of HOMO SAPIENS PROTEIN ASSOCIATED WITH PRK1 (AWP1), MRNA (NM_019006); a gene of HOMO SAPIENS PROGESTERONE RECEPTOR MEMBRANE COMPONENT 2 (PGRMC2), MRNA (NM_006320); a gene of HOMO SAPIENS POLYGLUTAMINE BINDING PROTEIN 1 (PQBP1), MRNA (NM_005710); a gene of HOMO SAPIENS S-ADENOSYLMETHIONINE DECARBOXYLASE 1 (AMD1), MRNA (NM_001634); HOMO SAPIENS INTERFERON-INDUCED, HEPATITIS C-ASSOCIATED MICROTUBULAR AG (NM_006417); a gene of HOMO SAPIENS PROTEINKINASE, AMP-ACTIVATED, GAMMA 1 NON-CATALYTIC SUBUN (NM_002733); HOMO SAPIENS PROCOLLAGEN-PROLINE, 2-OXOGLUTARATE 4-DIOXYGENASE (PROLINE (NM_004199); a gene of HOMO SAPIENS ENDOTHELIAL DIFFERENTIATION, LYSOPHOSPHATIDIC ACID G-PROTE (NM_012152);or a gene of HOMO SAPIENS KIAA0127 GENE PRODUCT (KIAA0127), MRNA(NM_014755).

In the present invention, known methods for detecting gene expression can be used to detect the expression of the mesenchymal stem cell marker gene of the present invention. For example, Northern blotting can be used to detect the expression of the mesenchymal stem cell marker gene of the present invention. In addition, it is possible to use a probe having a DNA sequence which hybridizes with a DNA sequence of the mesenchymal stem cell gene marker of the present invention under a stringent condition in order to detect and distinguish mesenchymal stem cells by the gene marker of the present invention. For the detection of mesenchymal stem cells by using the probe, known methods can be appropriately used. For instance, the detection of mesenchymal stem cells is conducted by the following process: constructing a DNA probe with appropriate length from a DNA sequence of a gene marker shown in the sequence listing; labeling the probe appropriately by fluorescent labeling, etc.; hybridizing the probe with a test substance. As the DNA probe, it is possible to use a probe for detecting mesenchymal stem cell marker genes comprising whole or part of an antisense strand of the base sequence of the gene marker of the present invention shown in the sequence listing. The probe can be used also in a form of a microarray or a DNA chip for detecting marker genes, wherein at least one of the probes is immobilized.

In the construction of the DNA probe mentioned above, the condition for the base sequence of the present invention "to hybridize with a DNA sequence of a marker gene for detecting mesenchymal stem cells under a stringent condition" is exemplified by hybridization at 42°C, and washing treatment at 42°C with buffer solution containing 1 × SSC (0.15 M NaCl, 0.015 M sodium citrate), and 0.1% SDS (sodium dodecyl sulfate), and more preferably exemplified by hybridization at 65°C, and washing treatment at 65°C with buffer solution containing 0.1 × SSC, and 0.1% SDS. There are various factors other than the temperature conditions mentioned above that affect the hybridization stringency and those skilled in the art can actualize the same stringency as that of the hybridization referred to in the above by appropriately combining various factors.

Further, when detecting the polypeptide marker for detecting mesenchymal stem cells of the present invention, an antibody induced by the polypeptide of the protein and specifically binds to the polypeptide can be used in the present invention. Examples of the antibody include monoclonal and polyclonal antibodies. The antibody can be constructed by ordinary methods with the polypeptide marker of the present invention as an antigen. In order to detect the expression of the marker polypeptide for detecting mesenchymal stem cells of the present invention in a test cell by using the antigen of the present invention, immunoassay methods using known antibodies can be used. As for such immunoassay methods, known immunoassay methods such as RIA, ELISA, and fluorescent antibody method are exemplified.

In the present invention, it is possible to detect and distinguish the expression of a marker gene for detecting mesenchymal stem cells and/or a marker polypeptide for detecting mesenchymal stem cells in a test cell by using the probe for detecting mesenchymal stem cells of the present invention and/or the antibody for detecting mesenchymal stem cells of the present invention, and to separate and obtain mesenchymal stem cells. When detecting a marker gene for detecting mesenchymal stem cells in a test cell, quantitative or semiquantitative PCR can be used to amplify the gene of the test cell. RT-PCR (reverse transcription PCR) can be used as the PCR. When conducting the PCR, primers comprising a sense primer and an antisense primer for amplifying a marker gene for detecting mesenchymal stem cells of the present invention are used.

Examples of the primers for amplifying a marker gene for detecting mesenchymal stem cells of the present invention include: a sense primer having a base sequence shown in SEQ ID NO: 20 in the sequence listing, and an antisense primer having a base sequence shown in SEQ ID NO: 21 in the sequence listing; a sense primer having a base sequence shown in SEQ ID NO: 22 in the sequence listing, and an antisense primer having a base sequence shown in SEQ ID NO: 23 in the sequence listing; a sense primer having a base sequence shown in SEQ ID NO: 24 in the sequence listing, and an antisense primer having a base sequence shown in SEQ ID NO: 25 in the sequence listing; a sense primer having a base sequence shown in SEQ ID NO: 26 in the sequence listing, and an antisense primer having a base sequence shown in SEQ ID NO: 27 in the sequence listing; a sense primer having a base sequence shown in SEQ ID NO: 28 in the sequence listing, and an antisense primer having a base sequence shown in SEQ ID NO: 29 in the sequence listing; a sense primer having a base sequence shown in SEQ ID NO: 30 in the sequence listing, and an antisense primer having a base sequence shown in SEQ ID NO: 31 in the sequence listing; a sense primer having a base sequence shown in SEQ ID NO: 32 in the sequence listing, and an antisense primer having a base sequence shown in SEQ ID NO: 33 in the sequence listing; a sense primer having a base sequence shown in SEQ ID NO: 34 in the sequence listing, and an antisense primer having a base sequence shown in SEQ ID NO: 35 in the sequence listing; a sense primer having a base sequence shown in SEQ ID NO: 36 in the sequence listing, and an antisense primer having a base sequence shown in SEQ ID NO: 37 in the sequence listing; a sense primer having a base sequence shown in SEQ ID NO: 38 in the sequence listing, and an antisense primer having a base sequence shown in SEQ ID NO: 39 in the sequence listing; a sense primer having a base sequence shown in SEQ ID NO: 40 in the sequence listing, and an antisense primer having a base sequence shown in SEQ ID NO: 41 in the sequence listing; a sense primer having a base sequence shown in SEQ ID NO: 42 in the sequence listing, and an antisense primer having a base sequence shown in SEQ ID NO: 43 in the sequence listing; or a sense primer having a base sequence shown in SEQ ID NO: 44 in the sequence listing, and an antisense primer having a base sequence shown in SEQ ID NO: 45 in the sequence listing.

Examples of the primers for amplifying a marker gene for detecting mesenchymal stem cells of the present invention further include: a real time PCR primer for amplifying a mesenchymal stem cell marker gene which comprises a sense primer having a base sequence shown in SEQ ID NO: 47 in the sequence listing, and an antisense primer having a base sequence shown in SEQ ID NO: 48 in the sequence listing; a sense primer having a base sequence shown in SEQ ID NO: 49 in the sequence listing, and an antisense primer having a base sequence shown in SEQ ID NO: 50 in the sequence listing; a sense primer having a base sequence shown in SEQ ID NO: 51 in the sequence listing, and an antisense primer having a base sequence shown in SEQ ID NO: 52 in the sequence listing; a sense primer having a base sequence shown in SEQ ID NO: 53 in the sequence listing, and an antisense primer having a base sequence shown in SEQ ID NO: 54 in the sequence listing; a sense primer having a base sequence shown in SEQ ID NO: 55 in the sequence listing, and an antisense primer having a base sequence shown in SEQ ID NO: 56 in the sequence listing; a sense primer having a base sequence shown in SEQ ID NO: 57 in the sequence listing, and an antisense primer having a base sequence shown in SEQ ID NO: 58 in the sequence listing; a sense primer having a base sequence shown in SEQ ID NO: 59 in the sequence listing, and an antisense primer having a base sequence shown in SEQ ID NO: 60 in the sequence listing; a sense primer having a base sequence shown in SEQ ID NO: 61 in the sequence listing, and an antisense primer having a base sequence shown in SEQ ID NO: 62 in the sequence listing; a sense primer having a base sequence shown in SEQ ID NO: 63 in the sequence listing, and an antisense primer having a base sequence shown in SEQ ID NO: 64 in the sequence listing; a sense primer having a base sequence shown in SEQ ID NO: 65 in the sequence listing, and an antisense primer having a base sequence shown in SEQ ID NO: 66 in the sequence listing; a sense primer having a base sequence shown in SEQ ID NO: 67 in the sequence listing, and an antisense primer having a base sequence shown in SEQ ID NO: 68 in the sequence listing; or a sense primer having a base sequence shown in SEQ ID NO: 69 in the sequence listing, and an antisense primer having a base sequence shown in SEQ ID NO: 70 in the sequence listing.

Examples of the primers for amplifying a marker gene for detectingmesenchymal stem cells of the present invention further include: an RT-PCR primer for amplifying a mesenchymal stem cell marker gene which comprises a sense primer having a base sequence shown in SEQ ID NO: 71 in the sequence listing, and an antisense primer having a base sequence shown in SEQ ID NO: 72 in the sequence listing; a sense primer having a base sequence shown in SEQ ID NO: 73 in the sequence listing, and an antisense primer having a base sequence shown in SEQ ID NO: 74 in the sequence listing; a sense primer having a base sequence shown in SEQ ID NO: 75 in the sequence listing, and an antisense primer having a base sequence shown in SEQ ID NO: 76 in the sequence listing; a sense primer having a base sequence shown in SEQ ID NO: 77 in the sequence listing, and an antisense primer having a base sequence shown in SEQ ID NO: 78 in the sequence listing; a sense primer having a base sequence shown in SEQ ID NO: 79 in the sequence listing, and an antisense primer having a base sequence shown in SEQ ID NO: 80 in the sequence listing; a sense primer having a base sequence shown in SEQ ID NO: 81 in the sequence listing, and an antisense primer having a base sequence shown in SEQ ID NO: 82 in the sequence listing; a sense primer having a base sequence shown in SEQ ID NO: 83 in the sequence listing, and an antisense primer having a base sequence shown in SEQ ID NO: 84 in the sequence listing; a sense primer having a base sequence shown in SEQ ID NO: 85 in the sequence listing, and an antisense primer having a base sequence shown in SEQ ID NO: 86 in the sequence listing; a sense primer having a base sequence shown in SEQ ID NO: 87 in the sequence listing, and an antisense primer having a base sequence shown in SEQ ID NO: 88 in the sequence listing; a sense primer having a base sequence shown in SEQ ID NO: 89 in the sequence listing, and an antisense primer having a base sequence shown in SEQ ID NO: 90 in the sequence listing; a sense primer having a base sequence shown in SEQ ID NO: 91 in the sequence listing, and an antisense primer having a base sequence shown in SEQ ID NO: 92 in the sequence listing; a sense primer having a base sequence shown in SEQ ID NO: 93 in the sequence listing, and an antisense primer having a base sequence shown in SEQ ID NO: 94 in the sequence listing; a sense primer having a base sequence shown in SEQ ID NO: 95 in the sequence listing, and an antisense primer having a base sequence shown in SEQ ID NO: 96 in the sequence listing; a sense primer having a base sequence shown in SEQ ID NO: 97 in the sequence listing, and an antisense primer having a base sequence shown in SEQ ID NO: 98 in the sequence listing; a sense primer having a base sequence shown in SEQ ID NO: 99 in the sequence listing, and an antisense primer having a base sequence shown in SEQ ID NO: 100 in the sequence listing; a sense primer having a base sequence shown in SEQ ID NO: 101 in the sequence listing, and an antisense primer having a base sequence shown in SEQ ID NO: 102 in the sequence listing; a sense primer having a base sequence shown in SEQ ID NO: 103 in the sequence listing, and an antisense primer having a base sequence shown in SEQ ID NO: 104 in the sequence listing; a sense primer having a base sequence shown in SEQ ID NO: 105 in the sequence listing, and an antisense primer having a base sequence shown in SEQ ID NO: 106 in the sequence listing; a sense primer having a base sequence shown in SEQ ID NO: 107 in the sequence listing, and an antisense primer having a base sequence shown in SEQ ID NO: 108 in the sequence listing; a sense primer having a base sequence shown in SEQ ID NO: 109 in the sequence listing, and an antisense primer having a base sequence shown in SEQ ID NO: 110 in the sequence listing; a sense primer having a base sequence shown in SEQ ID NO: 111 in the sequence listing, and an antisense primer having a base sequence shown in SEQ ID NO: 112 in the sequence listing; a sense primer having a base sequence shown in SEQ ID NO: 113 in the sequence listing, and an antisense primer having a base sequence shown in SEQ ID NO: 114 in the sequence listing; or a sense primer having a base sequence shown in SEQ ID NO: 115 in the sequence listing, and an antisense primer having a base sequence shown in SEQ ID NO: 116 in the sequence listing.

In order to distinguish mesenchymal stem cells in the present invention, a gene in a test cell is amplified by using at least one pair of primers comprising the sense primer and the antisense primer mentioned above, and the amplified gene is detected by using the probe for detecting mesenchymal stem cell marker genes of the present invention.

With regard to the marker genes for detecting mesenchymal stem cells of the present invention, some genes exhibit specifically high expression and some genes exhibit specifically low expression in mesenchymal stem cells. By detecting the expressions of these genes in combination, accuracy in detecting mesenchymal stem cells can be improved. Examples of genes exhibiting specifically high expression in mesenchymal stem cells include a mesenchymal stem cell marker gene comprising SEQ ID NOs: 1, 11 and 19 in the sequence listing, and those of genes exhibiting specifically low expression in mesenchymal stem cells include a mesenchymal stem cell marker gene comprising SEQ ID NOs: 2, 4, 6, 7, 9, 10, 12, 14, 15, 17 and 46 in the sequence listing. The mesenchymal stem cell marker gene confirmed in the present invention is also exemplified. Therefore, by combining the expression of one or more genes from each gene group, mesenchymal stem cells can be distinguished with more improved detection accuracy.

In order to detect the expression of a mesenchymal stem cell marker polypeptide in a test cell by a fluorescent antibody method using the antibody of the present invention, and to distinguish and separate mesenchymal stem cells, mesenchymal stem cells can be labeled and separated by known methods. As for the labeling method, for instance, a fluorescent antibody method is exemplified. The labeling of undifferentiated hemopoietic cells by the fluorescent antibody method can be conducted by: fluorescently labeling an antibody that specifically binds to the polypeptide marker for detecting mesenchymal stem cells of the present invention, and labeling mesenchymal stem cells by binding the antibody to mesenchymal stem cells expressing an antigen (direct fluorescent antibody method); or binding the unlabeled specific antibody of the present invention to a mesenchymal stem cell expressing an antigen, and then, by binding a labeled secondary antibody (anti-immunoglobulin antibody), labeling mesenchymal stem cells (indirect fluorescent antibody method). Subsequently, the labeled mesenchymal stem cells are separated and collected.

The probe for detecting mesenchymal stem cell marker genes used for detecting and distinguishing mesenchymal stem cells of the present invention, the microarray or the DNA chip for detecting mesenchymal stem cell marker genes in which the probe is immobilized, and the antibody for detecting mesenchymal stem cells of the present invention can be commercialized as a kit for distinguishing mesenchymal stem cells in which they are included.

The present invention is described below more specifically with reference to Examples, however, the technical scope of the present invention is not limited to these exemplification.

### Example 1 [Materials and methods]

### (Cells)

Human mesenchymal stem cells and human fibroblasts were cultured at 37°C in a Dalbecco's modified Eagle's medium (low glucose) (Sigma) supplemented with 10% fetal calf serum and 1 ng/ml bFGF.

### (DNA microarray)

Total RNA was extracted from each cell in one 10 cm-dish by using TRIZOL reagent (Invitrogen), and then mRNA was purified by using micro poly (A) purist (Ambion) to conduct DNA microarray (Incyte Genomics: Kurabo Life Array analysis service; Lot. No. KL01081). The DNA microarray analysis was commissioned to Kurabo.

### (Semiquantitative RT-PCR)

Total RNA was extracted from each cell by using TRIZOL reagent (Invitrogen), and then cDNA was synthesized by conducting a reverse transcription reaction at 42°C for 50 minutes with SuperScript first-strand synthesis system for RT-PCR (Invitrogen) using 1 mg each of total RNA as a template. Each gene was amplified with Advantage 2 PCR enzyme system (Clontech) using the amplified cDNA as a template. The reaction was conducted in 30 cycles, and each cycle included denaturation at 94°C for 30 seconds and annealing at 68°C for 1 minute. The primer sequences of each gene used for amplification are tabulated in Table 1. The amplified genes were separated by electrophoresis using 1% agarose gel, and detected by ethidium bromide staining.

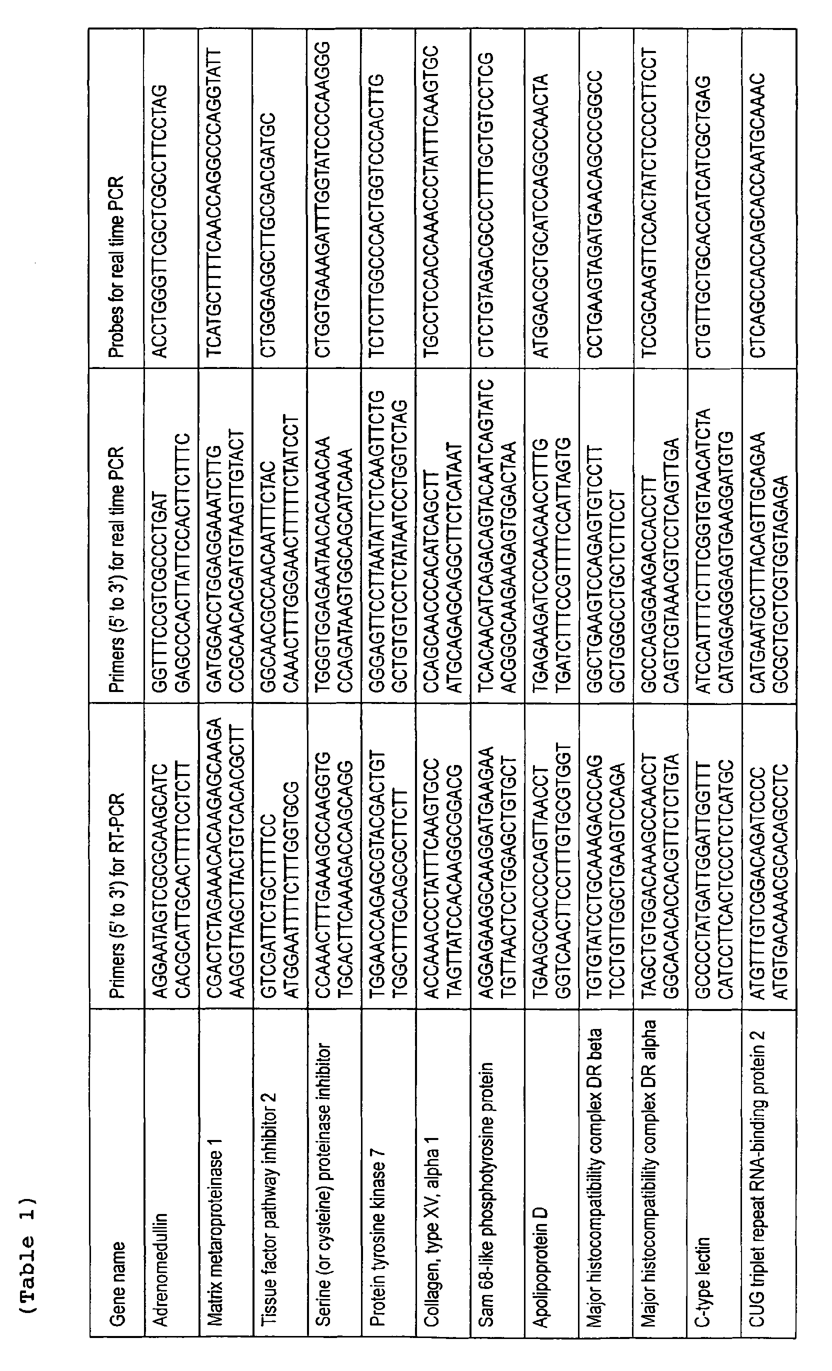

### Example 2 [Detection results]

### (DNA microarray)

As a result of DNA microarray, it was revealed that 63 out of 9400 genes exhibited significantly higher expression, and 141 out of 9400 genes exhibited significantly lower expression in mesenchymal stem cells than in fibroblasts. Significant differences were not observed in other genes. Among 87 genes which exhibited a difference of three-fold or more, 29 genes exhibited higher expression and 58 genes exhibited lower expression in mesenchymal stem cells.

### (Semiquantitative RT-PCR)

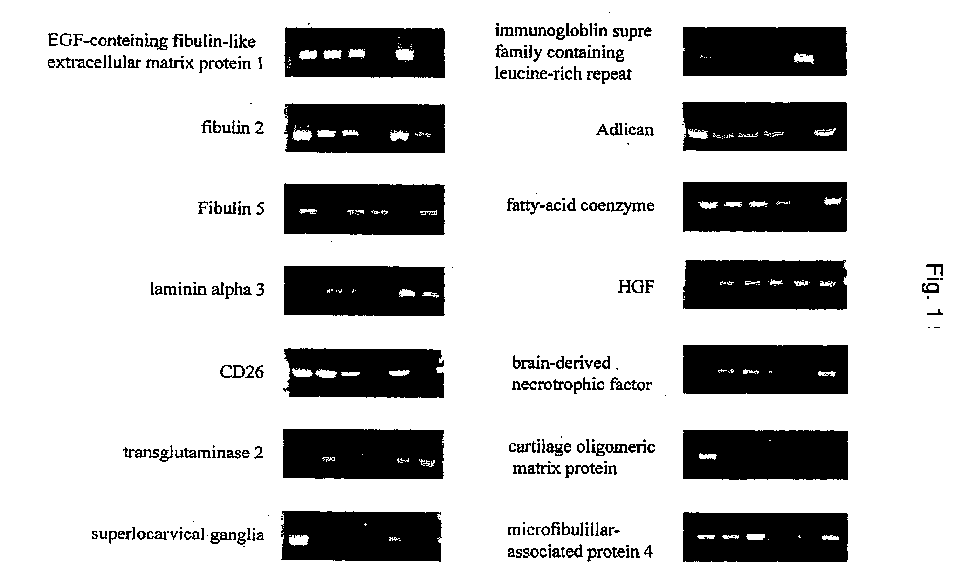

Total RNAs were extracted from mesenchymal stem cells derived from 3 individuals and fibroblasts derived from 3 individuals, and by using them as a template, the expression levels of the above-mentioned 87 genes were examined by semiquantitative RT-PCR. As a result, most genes exhibited no difference in the expression levels. In addition, there were many cases wherein differences in the expression were considered to be caused by individual differences, because differences in the expression levels were observed between mesenchymal stem cells, or fibroblasts, from different individuals (Fig. 1).

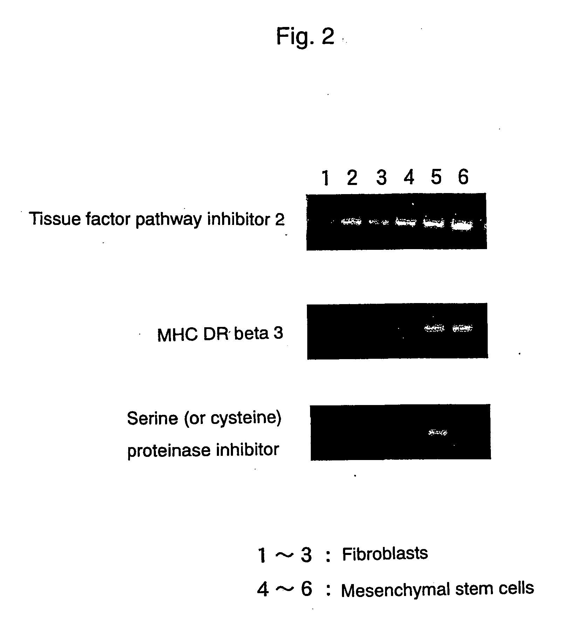

The following 3 genes were confirmed to exhibit high expression in mesenchymal stem cells by RT-PCR: serine (or cysteine) proteinase inhibitor (SEQ ID NO: 1 in the sequence listing), major histocompatibility complex class2 DR beta 3 (SEQ ID NO: 11), and tissue factor pathway inhibitor 2 (SEQ ID NO: 19) (Fig. 2).

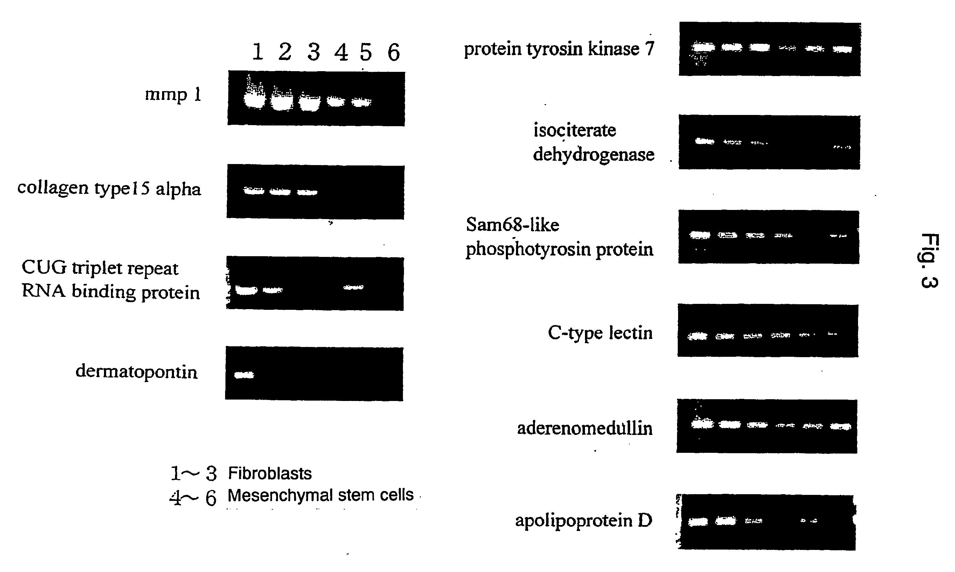

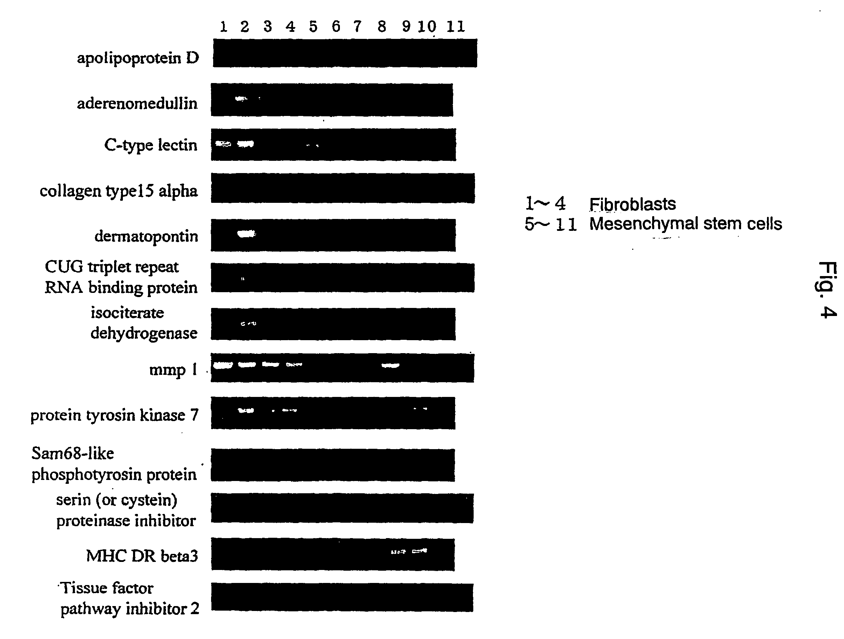

In addition, the following 10 genes were confirmed to exhibit low expression in mesenchymal stem cells by RT-PCR: adrenomedullin (SEQ ID NO: 2), apolipoprotein D (SEQ ID NO: 4), collagenase type XV alpha 1 (SEQ ID NO: 6), CUG triplet repeat RNA binding protein (SEQ ID NO: 7), dermatopontin protein (SEQ ID NO: 9), isocitrate dehydrogenase 2 (SEQ ID NO: 10), tyrosine kinase 7 (SEQ ID NO: 12), Sam68-like phosphotyrosine protein (SEQ ID NO: 14), C-type lectin superfamily member 2 (SEQ ID NO: 15), and matrix metalloprotease 1 (SEQ ID NO: 17) (Fig. 3). Further, with regard to the 13 genes, the expression levels were confirmed by semiquantitative RT-PCR using total RNA extracted from mesenchymal stem cells derived from 7 individuals and fibroblasts derived from 4 individuals as a template (Fig. 4).

### Example 3 [Measurement of the expression of each gene in mesenchymal stem cells I]

The expressions of the gene marker of the present invention in human mesenchymal stem cells and human fibroblasts were measured in the same manner as in Example 1. The primers for RT-PCR and for real time PCR, and probes for real time PCR used are shown in Table 2.

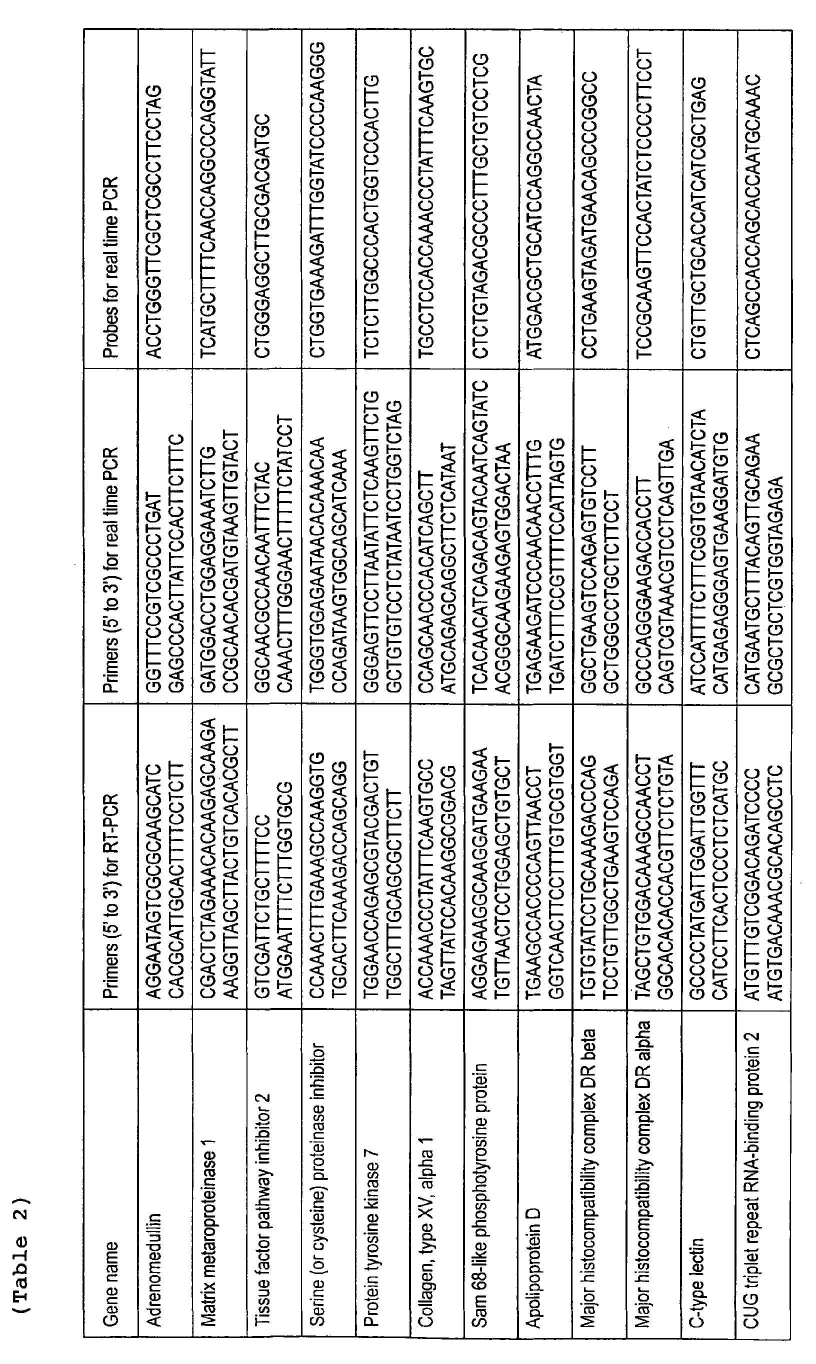

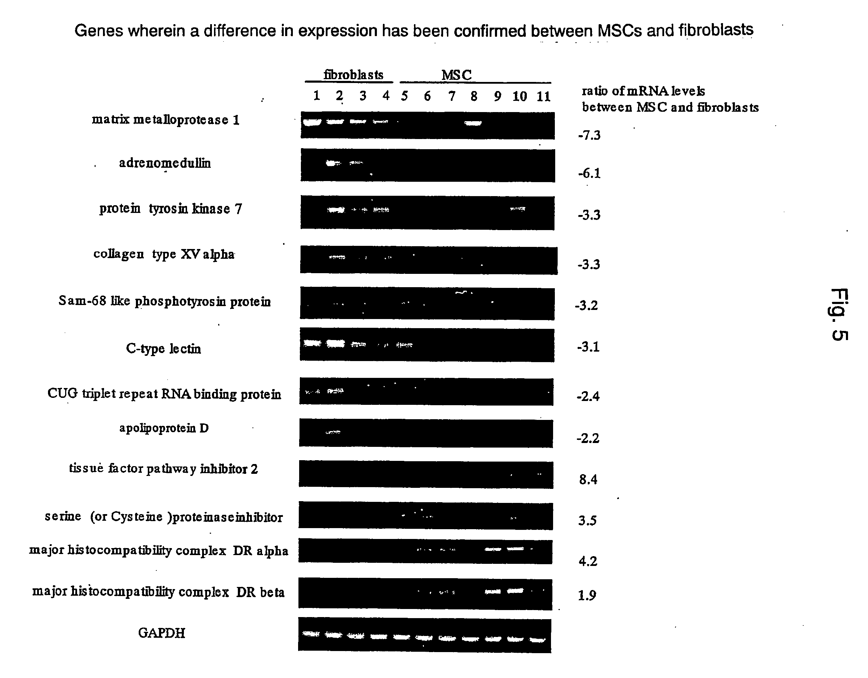

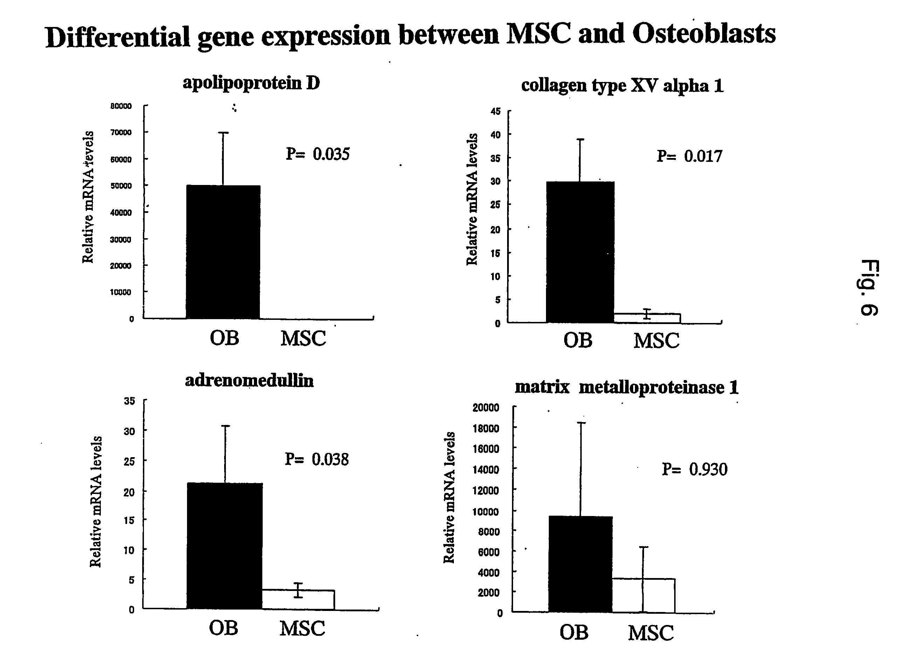

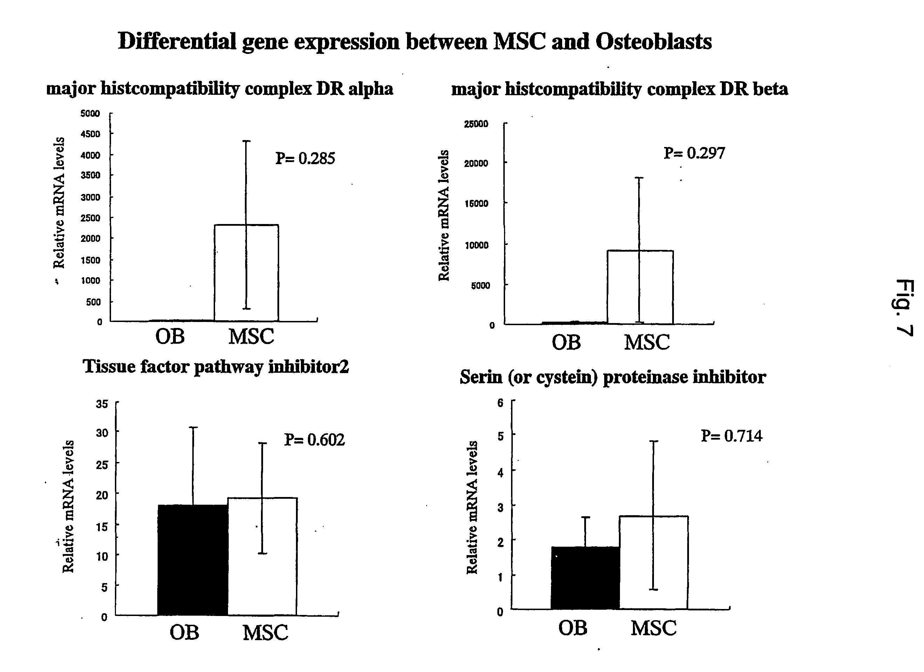

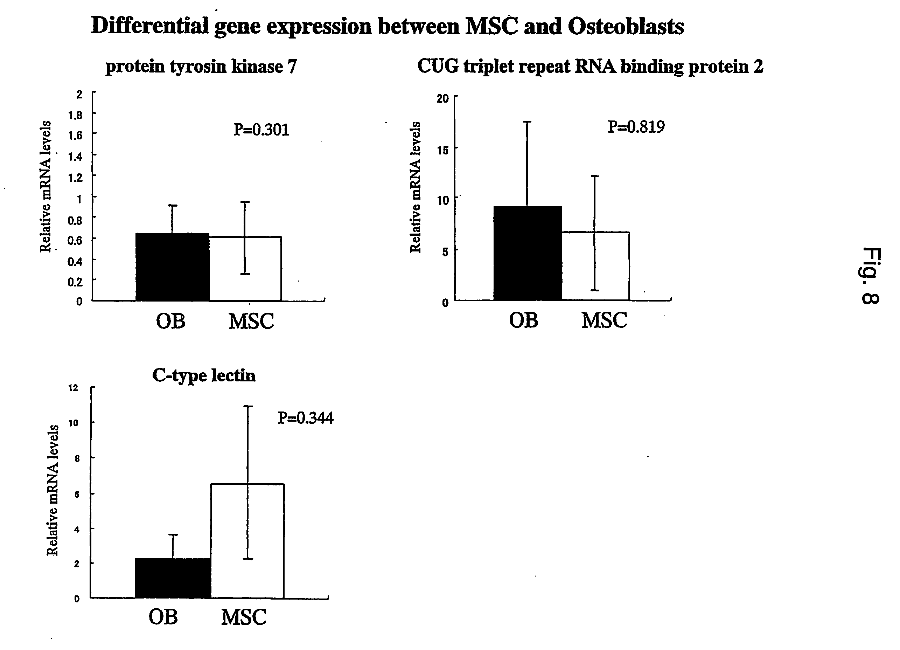

The expression state of mRNA level in mesenchymal stem cells and human fibroblasts are shown in Fig. 5. The relative expression state of mRNA level in mesenchymal stem cells and human fibroblasts are shown in Figs. 6, 7, and 8.

### Example 4 [Measurement of the expression of each gene in mesenchymal stem cells II]

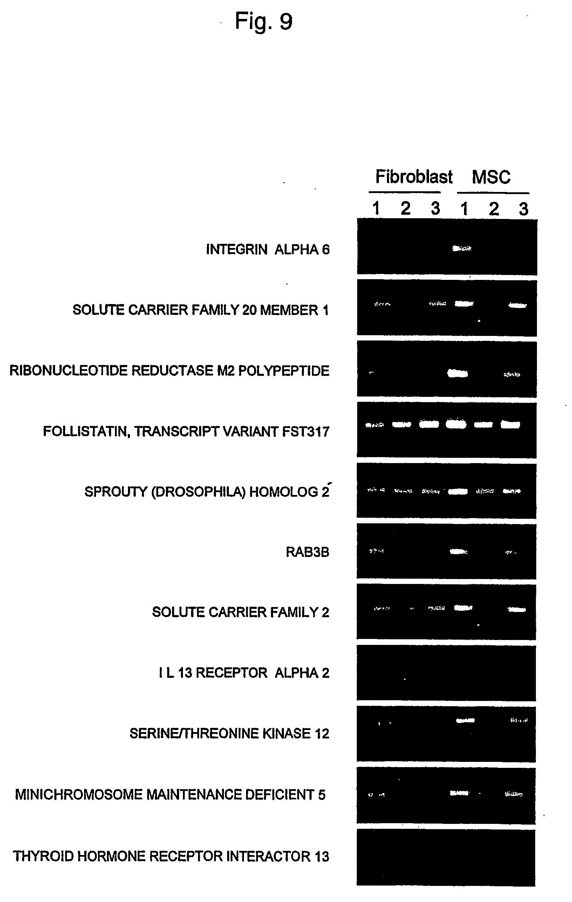

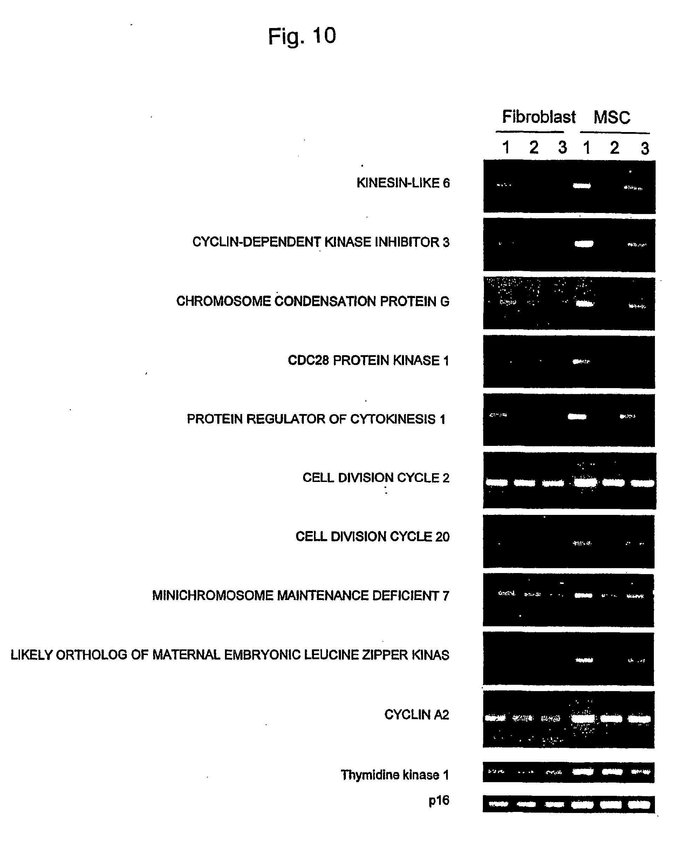

The expressions of each gene marker of the present invention in human mesenchymal stem cells and human fibroblasts were measured in the same manner as in Example 1. The sense and antisense primers for RT-PCR used are shown in SEQ ID NOs: 71 to 116 in the sequence listing. The expression state of genes in mesenchymal stem cells and human fibroblasts are shown in Figs. 9 and 10. The expression ratio, mesenchymal stem cell/fibroblast, is shown in Table 3.

**(Table 3)**

| MSC/fibroblast | Gene name |
|---|---|
| 11.6 | NTEGRIN, ALPHA 6 (ITGA6), MRNA |
| 8.7 | SOLUTE CARRIER FAMILY 20 (PHOSPHATE TRANSPORTER), MEMBER 1 |
| 7.8 | RIBONUCLEOTIDE REDUCTASE M2 POLYPEPTIDE (RRM2), MRNA |
| 7.6 | FOLLISTATIN (FST), TRANSCRIPT VARIANT FST317, MRNA |
| 7.3 | SPROUTY (DROSOPHILA) HOMOLOG 2 (SPRY2), MRNA |
| 6.9 | RAB3B, MEMBER RAS ONCOGENE FAMILY (RAB3B), MRNA |
| 6.5 | SOLUTE CARRIER FAMILY 2 (FACILITATED GLUCOSE TRANSPORTER) |
| 6.1 | INTERLEUKIN 13 RECEPTOR, ALPHA 2 (IL13RA2), MRNA |
| 5.9 | SERINE/THREONINE KINASE 12 (STK12), MRNA |
| 5.7 | MINICHROMOSOME MAINTENANCE DEFICIENT (S. CEREVISIAE) |
| 5.6 | THYROID HORMONE RECEPTOR INTERACTOR 13 (TRIP13), MRNA |
| 5.5 | KINESIN-LIKE 6 (MITOTIC CENTROMERE-ASSOCIATED KINESIN) |
| 5.5 | CYCLIN-DEPENDENT KINASE INHIBITOR 3 |
| 5.4 | CHROMOSOME CONDENSATION PROTEIN G (HCAP-G), MRNA |
| 5.2 | CDC28 PROTEIN KINASE 1 (CKS1), MRNA |
| 5.2 | PROTEIN REGULATOR OF CYTOKINESIS 1 (PRC1), MRNA |
| 5.2 | CELL DIVISION CYCLE 2, G1 TO S AND G2 TO M (CDC2), MRNA |
| 5.2 | CDC20 (CELL DIVISION CYCLE 20, S. CEREVISIAE, HOMOLOG) (CD |
| 5.1 | LIKELY ORTHOLOG OF MATERNAL EMBRYONIC LEUCINE ZIPPER KINAS |
| 5.1 | MINICHROMOSOME MAINTENANCE DEFICIENT (S. CEREVISIAE) 7 |
| 4.9 | YCLIN A2 (CCNA2), MRNA |
| 3.6 | THYMIDINE KINASE 1, SOLUBLE (TK1), MRNA |
| 0.6 | cyclin-dependent kinase inhibitor 2A (melanoma, p16, inhibits CDK4) (CDKN2A), mRNA |

### Example 5 [Measurement of the expression of each gene in mesenchymal stem cells III]

The expressions of the gene marker of the present invention in human mesenchymal stem cells and human fibroblasts were measured in the same manner as in Example 1. The expression ratio, mesenchymal stem cell/fibroblast, is shown in Table 4.

**(Table 4)**

| (Mesenchymal stem cells/fibroblasts) | (Gene name) |
|---|---|
| 8.9 | EGF-containing fibulin-like extracellular matrix protein 1 |
| 8.4 | Human insulin-like growth factor binding protein 5 (IGFBP5) mRNA |
| 7.7 | Homo sapiens clone 24775 mRNA sequence |
| 7 | "proteoglycan 1, secretory granule" |
| 6.8 | insulin-like growth factor binding protein 5 |
| 6.7 | "solute carrier family 21 (organic anion transporter), member 3" |
| 6.5 | "transglutaminase 2 (C polypeptide,proteinglutamine-gamma-glutamyl transferase)" |
| 5.8 | coagulation factor II (thrombin) receptor |
| 5.6 | "plasminogen activator, urokinase" |
| 5.5 | "tissue inhibitor of metalloproteinase 3 (Sorsby fundus dystrophy, pseudoinflammatory)" |
| 5.1 | matrix Gla protein |
| 4.6 | B-cell CLL/lymphoma 1 |
| 4.6 | "CD74 antigen (invariant polypeptide of major histocompatibility complex, class II antigen-associated)" |
| 4.2 | adducin 3 (gamma) |
| 4.2 | "solute carrier family 16 (monocarboxylic acid transporters), member 4" |
| 3.4 | protein S (alpha) |
| 3.3 | "phosphatidylinositol (4,5) bisphosphate 5-phosphatase homolog; phosphatidylinositol |
| | polyphosphate 5-phosphatase type IV" |
| 3.2 | progesterone membrane binding protein |
| 3.2 | brain-derived neurotrophic factor |
| 3 | apolipoprotein E |
| 38.5 | "GI\|6005949\|REF\|NM_007191.1\| HOMO SAPIENS WNT INHIBITORY FACTOR-1 (WIF-1), MRNA" |
| 12.7 | "GI\|8922916\|REF\|NM_018349.1\| HOMO SAPIENS HYPOTHETICAL PROTEIN FLJ11175 (FLJ11175), MRNA" . |
| 11.2 | "GI\|10835001\|REF\|NM_001175.1\| HOMO SAPIENS RHO GDP DISSOCIATION INHIBITOR (GDI) BETA (ARHGDIB), MRNA" |
| 10.3 | "GI\|11055973\|REF\|NM_021614.1\| HOMO SAPIENS POTASSIUM INTERMEDIATE/SMALL CONDUCTANCE CALCIUM-ACTIVATE" |
| 8.7 | "GI\|5731346\|REF\|NM_004289.3\| HOMO SAPIENS NUCLEAR FACTOR (ERYTHROID-DERIVED 2)-LIKE 3 (NFE2L3), MRNA" |
| 7.6 | "GI\|11056053\|REF\|NM_021643.1\| HOMO SAPIENS GS3955 PROTEIN (GS3955), MRNA" |
| 7.5 | "EST10324 ADIPOSE TISSUE, WHITE I HOMO SAPIENS CDNA 3' END SIMILAR TO SIMILAR TO GLYCOPROTEIN MUC18," |
| 6.6 | "GI\|13375818\|REF\|NM_024609.1\| HOMO SAPIENS HYPOTHETICAL PROTEIN FLJ21841 (FLJ21841), MRNA" |
| 6.5 | "GI\|6005971\|REF\|NM_007150.1\| HOMO SAPIENS ZINC FINGER PROTEIN 185 (LIM DOMAIN) (ZNF185), MRNA" |
| 6.5 | "GNL\|UG\|HS#S2334464 SC1=PUTATIVE TRANS-ACTING FACTOR INVOLVED IN CELL CYCLE CONTROL [HUMAN, MRNA, 24" |
| 6.4 | "GI\|7657368\|REF\|NM_014222.1\| HOMO SAPIENS NADH DEHYDROGENASE (UBIQUINONE) 1 ALPHA SUBCOMPLEX, 8 (19K" |
| 6.3 | "GI\|8924142\|REF\|NM_018518.1\| HOMO SAPIENS HOMOLOG OF YEAST MCM10; HYPOTHETICAL PROTEIN PRO2249 (PRO2" |
| 6.2 | "GI\|4502960\|REF\|NM_000094.1\| HOMO SAPIENS COLLAGEN, TYPE VII, ALPHA 1 (EPIDERMOLYSIS BULLOSA, DYSTRO" |
| 6.2 | "GI\|4502074\|REF\|NM_001144.1\| HOMO SAPIENS AUTOCRINE MOTILITY FACTOR RECEPTOR (AMFR), MRNA" |
| 6.2 | GNL\|UG\|HS#S1090551 HOMO SAPIENS CLONE 24421 MRNA SEQUENCE /CDS=UNKNOWN /GB=AF070641 /GI=3283914 /UG= |
| 6.2 | GNL\|UG\|HS#S1570341 HOMO SAPIENS MRNA; CDNA DKFZP586E1621 (FROM CLONE DKFZP586E1621) /CDS=UNKNOWN /GB |
| 6.1 | "GI\|11545760\|REF\|NM_022055.1\| HOMO SAPIENS RESERVED (KCNK12), MRNA" |
| 6.0 | "GI\|5902145\|REF\|NM_007019.1\| HOMO SAPIENS UBIQUITIN-CONJUGATING ENZYME E2C (UBE2C), MRNA" |
| 5.9 | "GNL\|UG\|HS#S1970614 HOMO SAPIENS CDNA FLJ10517 FIS, CLONE |
| | NT2RP2000812 /CDS=(61,918) /GB=AK001379 /G" |
| 5.8 | "GI\|4503052\|REF\|NM_001884.1\| HOMO SAPIENS CARTILAGE LINKING PROTEIN 1 (CRTL1), MRNA" |
| 5.8 | GNL\|UG\|HS#S1972520 HOMO SAPIENS MRNA; CDNA DKFZP434F2322 (FROM CLONE DKFZP434F2322) /CDS=UNKNOWN /GB |
| 5.7 | "GNL\|UG\|HS#S3837475 HOMO SAPIENS, CLONE MGC:9549 IMAGE:3857382, MRNA, COMPLETE CDS /CDS=(92,1285) /G" |
| 5.4 | "GI\|14150008\|REF\|NM_032270.1\| HOMO SAPIENS HYPOTHETICAL PROTEIN DKFZP586J1119 (DKFZP586J1119), MRNA" |
| 5.4 | "GI\|4758439\|REF\|NM_004877.1\| HOMO SAPIENS GLIA MATURATION FACTOR, GAMMA (GMFG), MRNA" |
| 5.4 | "GNL\|UG\|HS#S2654530 HOMO SAPIENS CDNA: FLJ23095 FIS, CLONE LNG07413 /CDS=UNKNOWN /GB=AK026748 /GI=10" |
| 5.3 | "GI\|8922180\|REF\|NM-018410.1\| HOMO SAPIENS HYPOTHETICAL PROTEIN DKFZP762E1312 (DKFZP762E1312), MRNA" |
| 5.3 | "GI\|5031806\|REF\|NM_005853.1\| HOMO SAPIENS IROQUOIS-CLASS HOMEODOMAIN PROTEIN (IRX-2A), MRNA" |
| 5.2 | "GI\|8922501\|REF\|NM_018131.1\| HOMO SAPIENS HYPOTHETICAL PROTEIN FLJ10540 (FLJ10540), MRNA" |
| 5.1 | "GNL\|UG\|HS#S1972896 HOMO SAPIENS MRNA; CDNA DKFZP434C1915 (FROM CLONE DKFZP434C1915); PARTIAL CDS /C" |
| 5.1 | "GI\|14149622\|REF\|NM_005916.1\| HOMO SAPIENS MINICHROMOSOME MAINTENANCE DEFICIENT (S. CEREVISIAE) 7 (M" |
| 5.0 | "GI\|4758177\|REF\|NM_004411.1\| HOMO SAPIENS DYNEIN, CYTOPLASMIC, INTERMEDIATE POLYPEPTIDE 1 (DNCI1), M" |
| 5.0 | HUMAN MRNA FOR PROTEIN GENE PRODUCT (PGP) 9.5. |
| 5.0 | "GI\|5453903\|REF\|NM_006479.1\| HOMO SAPIENS RAD51-INTERACTING PROTEIN (PIR51), MRNA" |
| 4.9 | "GI\|4502144\|REF\|NM_001168.1\| HOMO SAPIENS BACULOVIRAL IAP REPEAT-CONTAINING 5 (SURVIVIN) (BIRC5), MR" |
| 4.9 | "GI\|9945386\|REF\|NM_004482.2\| HOMO SAPIENS UDP-N-ACETYL-ALPHA -D-GALACTOSAMINE:POLYPEPTIDE N-ACETYLGAL" |
| 4.9 | "GI\|14327895\|REF\|NM_031966.1\| HOMO SAPIENS CYCLIN B1 (CCNB1), MRNA" |
| 4.8 | "GI\|6325465\|REF\|NM_004111.3\| HOMO SAPIENS FLAP STRUCTURE-SPECIFIC ENDONUCLEASE 1 (FEN1), MRNA" |
| 4.8 | "GI\|4507274\|REF\|NM_003600.1\| HOMO SAPIENS SERINE/THREONINE KINASE 15 (STK15), MRNA" |
| 4.8 | "GI\|6466452\|REF\|NM_002358.2\| HOMO SAPIENS MAD2 (MITOTIC ARREST DEFICIENT, YEAST, HOMOLOG)-LIKE 1 (MA" |
| 4.7 | "GI\|15421859\|REF\|NM_018454.2\| HOMO SAPIENS NUCLEOLAR PROTEIN ANKT (ANKT), MRNA" |
| 4.7 | "GI\|7661807\|REF\|NM_014176.1\| HOMO SAPIENS HSPC150 PROTEIN SIMILAR TO UBIQUITIN-CONJUGATING ENZYME (H" |
| 4.7 | "GI\|9845284\|REF\|NM_019885.1\| HOMO SAPIENS CYTOCHROME P450 RETINOID METABOLIZING PROTEIN (P450RAI-2)," |
| 4.7 | "GI\|4505372\|REF\|NM_002497.1\| HOMO SAPIENS NIMA (NEVER IN MITOSIS GENE A)-RELATED KINASE 2 (NEK2), MR" |
| 4.7 | "GI\|4502422\|REF\|NM_001202.1\| HOMO SAPIENS BONE MORPHOGENETIC PROTEIN 4 (BMP4), MRNA" |
| 4.7 | "GNL\|UG\|HS#S1970775 HOMO SAPIENS CDNA FLJ10674 FIS, CLONE NT2RP2006436 /CDS=UNKNOWN /GB=AK001536/GI" |
| 4.7 | "GI\|4585861\|REF\|NM_001809.2\| HOMO SAPIENS CENTROMERE PROTEIN A (17KD) (CENPA), MRNA" |
| 4.6 | GNL\|UG\|HS#S1368141 HOMO SAPIENS MRNA; CDNA DKFZP564P116 (FROM CLONE DKFZP564P116) /CDS=UNKNOWN /GB=A |
| 4.6 | "GI\|4507718\|REF\|NM_003318.1\| HOMO SAPIENS TTK PROTEIN KINASE (TTK), MRNA" |
| 4.6 | "GI\|4504896\|REF\|NM_002266.1\| HOMO SAPIENS KARYOPHERIN ALPHA 2 (RAG COHORT 1, IMPORTIN ALPHA 1) (KPNA" |
| 4.6 | "GI\|5729983\|REF\|NM_006596.1\| HOMO SAPIENS POLYMERASE (DNA |
| | DIRECTED), THETA (POLQ), MRNA" |
| 4.5 | "GI\|4758315\|REF\|NM_004454.1\| HOMO SAPIENS ETS VARIANT GENE 5 (ETS-RELATED MOLECULE) (ETV5), MRNA" |
| 4.5 | "HOMO SAPIENS TRANSFORMING, ACIDIC COILED-COIL CONTAINING PROTEIN 3 (TAC" |
| 4.5 | "HOMO SAPIENS KINESIN-LIKE 1 (KNSL1), MRNA" |
| 4.5 | "HOMO SAPIENS CENTROMERE PROTEIN E (312KD) (CENPE), MRNA" |
| 4.5 | "HOMO SAPIENS CDNA, 3' END /CLONE=IMAGE:1651289 /CLONE_END=3' /GB=AI12" |
| 4.4 | "HOMO SAPIENS DISTAL-LESS HOMEO BOX 5 (DLX5), MRNA" |
| 4.4 | "HOMO SAPIENS V-MYB AVIAN MYELOBLASTOSIS VIRAL ONCOGENE HOMOLOG-LIKE 2 " |
| 4.4 | "HOMO SAPIENS SERUM DEPRIVATION RESPONSE (PHOSPHATIDYLSERINE -BINDING PRO" |
| 4.4 | "HOMO SAPIENS HYPOTHETICAL PROTEIN FLJ10604 (FLJ10604), MRNA" |
| 4.4 | "HOMO SAPIENS RIBONUCLEASE HI, LARGE SUBUNIT (RNASEHI), MRNA |
| 4.4 | "HOMO SAPIENS ANILLIN (DROSOPHILA SCRAPS HOMOLOG), ACTIN BINDING PROTEIN" |
| 4.4 | "HOMO SAPIENS H4 HISTONE FAMILY, MEMBER G (H4FG), MRNA" |
| 4.3 | "HOMO SAPIENS G PROTEIN-COUPLED RECEPTOR 37 (ENDOTHELIN RECEPTOR TYPE B-" |
| 4.3 | "HOMO SAPIENS UBIQUITIN CARRIER PROTEIN (E2-EPF), MRNA" |
| 4.3 | "HOMO SAPIENS, SIMILAR TO TUMOR DIFFERENTIALLY EXPRESSED 1, CLONE IMAGE:3639252, " |
| 4.2 | "HOMO SAPIENS HYPOTHETICAL PROTEIN MGC2577 (MGC2577), MRNA" |
| 4.2 | "HOMO SAPIENS HYPOTHETICAL PROTEIN FLJ13912 (FLJ13912), MRNA" |
| 4.2 | "HOMO SAPIENS ANNEXIN A3 (ANXA3), MRNA" |
| 4.2 | "HOMO SAPIENS STATHMIN 1/ONCOPROTEIN 18 (STMN1), MRNA" |
| 4.2 | "GNL\|UG\|HS#S3837587 HOMO SAPIENS, SIMILAR TO RIBOSOMAL PROTEIN L39, CLONE MGC:20168 IMAGE:4555759, M" |
| 4.2 | "HOMO SAPIENS POLO (DROSOPHIA)-LIKE KINASE (PLK), MRNA" |

### Example 6 [Measurement of the expression of each gene in mesenchymal stem cells VI]

The expression of the gene marker of the present invention in human mesenchymal stem cells and human fibroblasts was measured in the same manner as in Example 1. The expression ratio, mesenchymal stem cell/fibroblast, is shown in Table 5. In the table, "-" indicates that the gene exhibits no or extremely low expression in mesenchymal stem cells.

**(Table 5)**

| (Mesenchymal stem cells/fibroblasts) | (Gene name) |
|---|---|
| -53.576 | "HOMO SAPIENS PROTEASOME (PROSOME, MACROPAIN) 26S SUBUNIT, ATPASE, 5 (PS" |
| -45.692 | "HOMO SAPIENS EUKARYOTIC TRANSLATION INITIATION FACTOR 4 GAMMA, 2 (EIF4G" |
| -27.6354 | "HOMO SAPIENS DIHYDROPYRIMIDINASE-LIKE 3 (DPYSL3), MRNA" |
| -20.5446 | "HOMO SAPIENS ADAPTOR-RELATED PROTEIN COMPLEX 1, SIGMA 2 SUBUNIT (AP1S2)" |
| -19.5809 | "HOMO SAPIENS PLECTIN 1, INTERMEDIATE FILAMENT BINDING PROTEIN, 500KD P" |
| -16.9025 | "HOMO SAPIENS HETEROGENEOUS NUCLEAR RIBONUCLEOPROTEIN U (SCAFFOLD ATTAC" |
| -14.97 | "HOMO SAPIENS HYPOTHETICAL PROTEIN MGC5306 (MGC5306), MRNA" |
| -14.6106 | "HOMO SAPIENS MHC CLASS I POLYPEPTIDE-RELATED SEQUENCE A (MICA), MRNA" |
| -13.8886 | "HOMO SAPIENS PROTEIN ASSOCIATED WITH PRK1 (AWP1), MRNA" |
| -13.6578 | "HOMO SAPIENS PROGESTERONE RECEPTOR MEMBRANE COMPONENT 2 (PGRMC2), MRNA" |
| -11.4783 | "HOMO SAPIENS POLYGLUTAMINE BINDING PROTEIN 1 (PQBP1), MRNA" |
| -10.2392 | "HOMO SAPIENS S-ADENOSYLMETHIONINE DECARBOXYLASE 1 (AMD1), MRNA" |
| -9.9506 | "HOMO SAPIENS INTERFERON-INDUCED, HEPATITIS C-ASSOCIATED MICROTUBULARAG" |
| -9.2594 | "HOMO SAPIENS PROTEIN KINASE, AMP-ACTIVATED, GAMMA 1 NON-CATALYTIC SUBUN" |
| -7.518 | "HOMO SAPIENS PROCOLLAGEN-PROLINE, 2-OXOGLUTARATE 4-DIOXYGENASE (PROLINE" |
| -6.0365 | "HOMO SAPIENS ENDOTHELIAL DIFFERENTIATION, LYSOPHOSPHATIDIC ACID G-PROTE" |
| -5.4442 | "HOMO SAPIENS KIAA0127 GENE PRODUCT (KIAA0127), MRNA"*" |

### Example 7 [Measurement of the expression of mesenchymal stem cell DR in human cells]

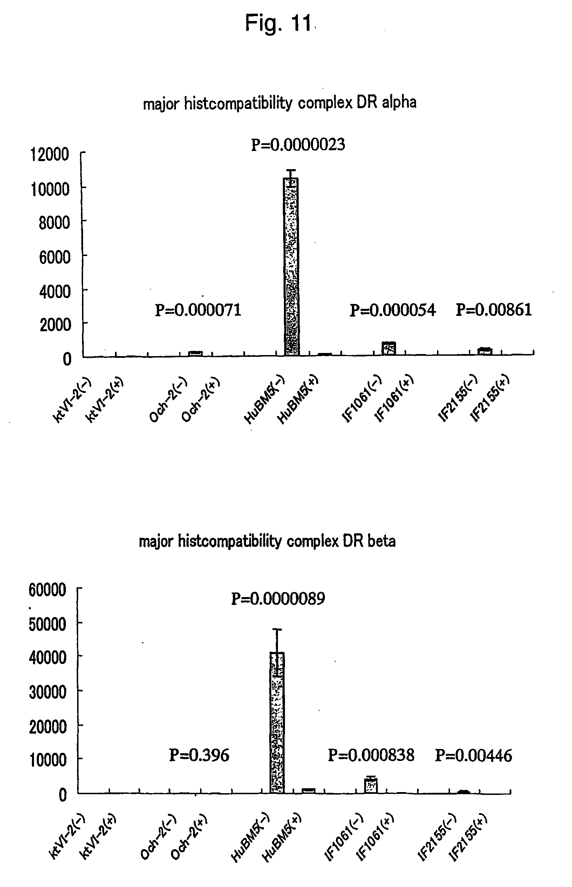

The expression of mesenchymal stem cell DR in human cells was measured with flow cytometry. The results are shown in Fig. 11.

### Example 8 [Changes in the expression of each gene in differentiation induction of mesenchymal stem cells]

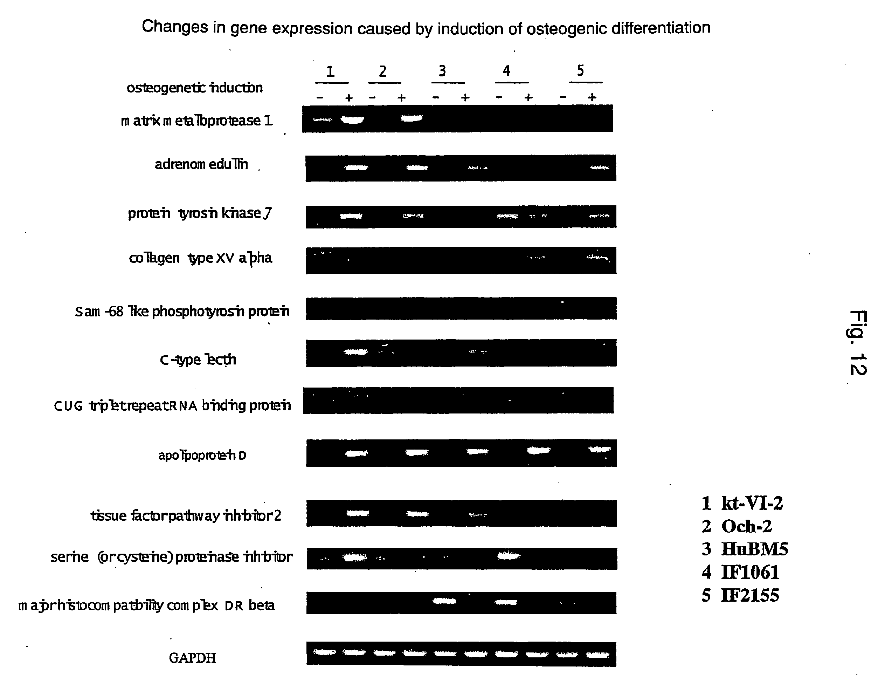

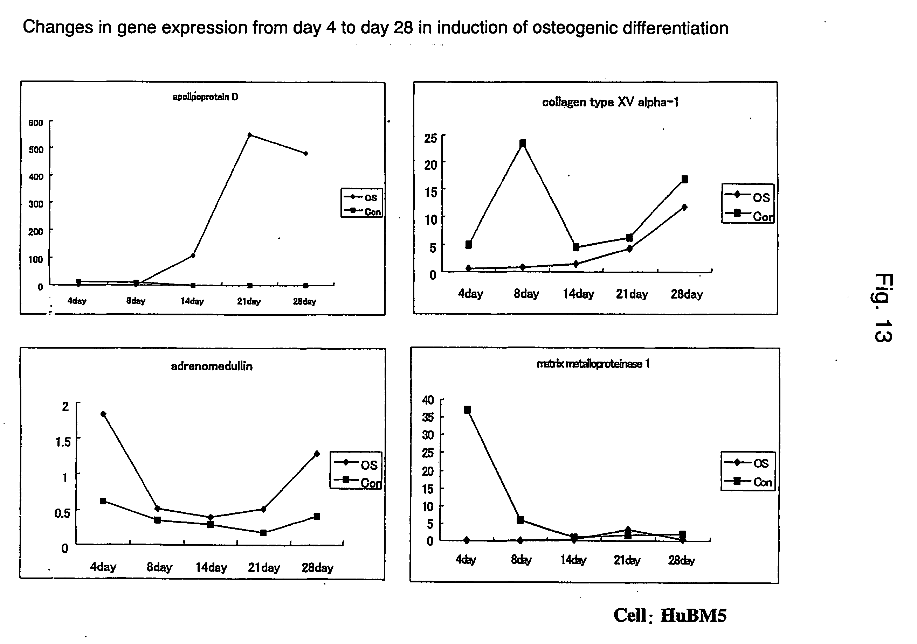

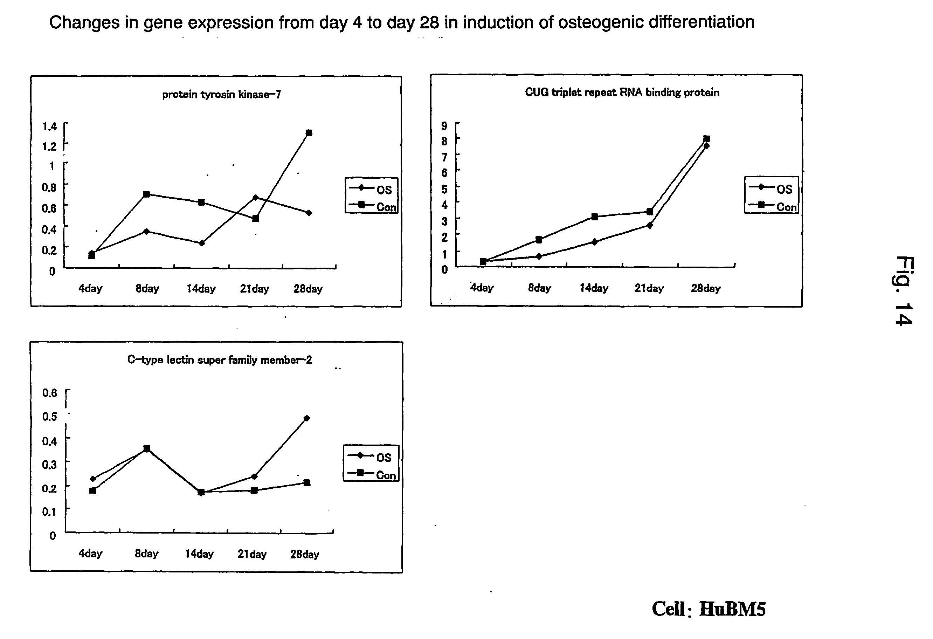

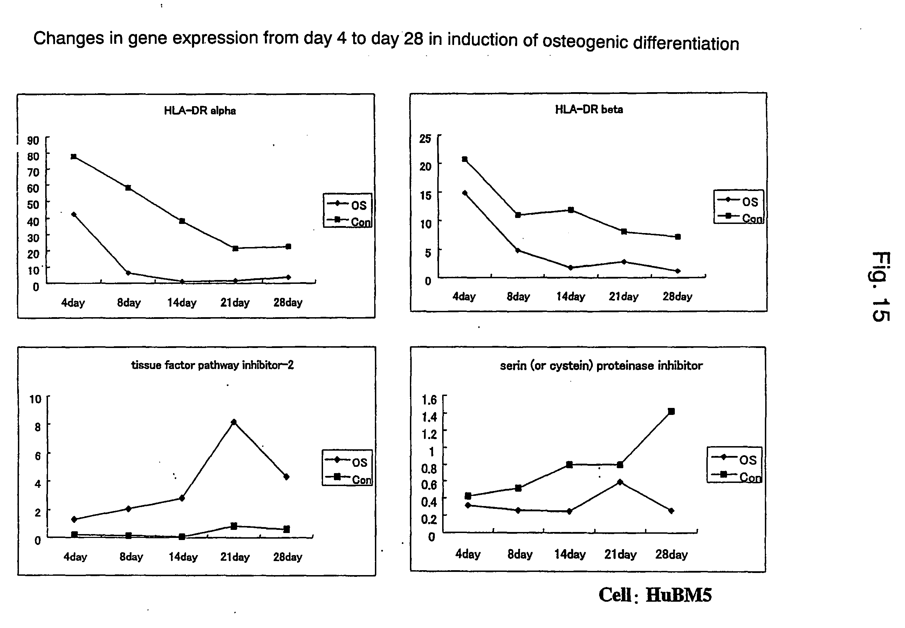

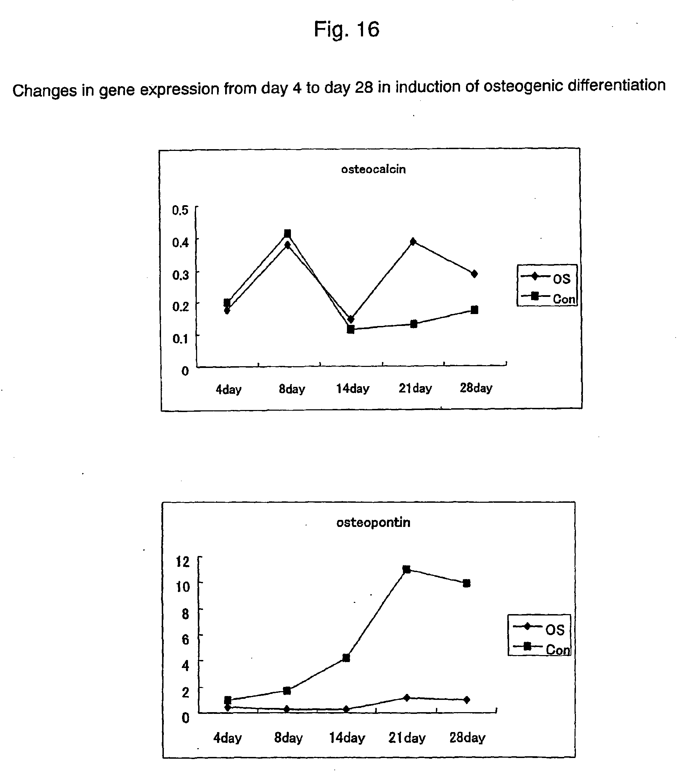

The changes in the expression of each gene in differentiation induction of mesenchymal stem cells were measured by using RT-PCR in the same manner as in Example 1. The expressions of each gene in differentiation induction of osteoblasts are shown in Figs. 12 to 16.

### Industrial Applicability

Recently, with the development of regenerative medicine, mesenchymal stem cells draw attention as xenograft, homograft and autograft materials for regenerative medicine of many tissues . In order to use mesenchymal stem cells for regenerative medicine of tissues, it is necessary to develop techniques for securing a sufficient amount of mesenchymal stem cells, and to confirm that the cultured cells are mesenchymal stem cells, and a method for detecting and distinguishing the mesenchymal stem cells becomes important. It is also helpful for separating mesenchymal stem cells from a group of many kinds of cells by using antibodies, etc. Marker genes which characterize mesenchymal stem cells are identified in the present invention, and as a result, it becomes possible to detect and distinguish mesenchymal stem cells easily and assuredly, and thereby substantial contribution to the practical use of mesenchymal stem cells in regenerative medicine is expected.

## Claims

1. A gene marker for detecting a mesenchymal stem cell which is a gene having a base sequence shown in SEQ ID NO: 1, 2, 4, 6, 7, 9, 10, 11, 12, 14, 15, 17, 19 or 46 in the sequence listing.

2. A gene marker for detecting a mesenchymal stem cell which is a gene of INTEGRIN, ALPHA 6 (ITGA6), MRNA (NM_000210); a gene of SOLUTE CARRIER FAMILY 20 (PHOSPHATE TRANSPORTER), MEMBER 1 (NM_005415); a gene of RIBONUCLEOTIDE REDUCTASE M2 POLYPEPTIDE (RRM2), MRNA(NM_001034); a gene of FOLLISTATIN(FST), TRANSCRIPT VARIANT FST317, MRNA (NM_006350); a gene of SPROUTY (DROSOPHILA) HOMOLOG 2 (SPRY2), MRNA (NM_005842); a gene of RAB3B, MEMBER RAS ONCOGENE FAMILY (RAB3B), MRNA (NM_002867); a gene of SOLUTE CARRIER FAMILY 2 (FACILITATED GLUCOSE TRANSPORTER) (NM_006516); a gene of INTERLEUKIN 13 RECEPTOR, ALPHA 2 (IL13RA2), MRNA (NM_000640); a gene of SERINE/THREONINE KINASE 12 (STK12), MRNA (NM_004217); a gene of MINICHROMOSOME MAINTENANCE DEFICIENT (S. CEREVISIAE) 5 (CE) (NM_006739); a gene of THYROID HORMONE RECEPTOR INTERACTOR 13 (TRIP13), MRNA (NM_004237); a gene of KINESIN-LIKE 6 (MITOTIC CENTROMERE-ASSOCIATED KINESIN) (K) (NM_006845); a gene of CYCLIN-DEPENDENT KINASE INHIBITOR 3 (CDK2-ASSOCIATED DUAL (NM_005192); a gene of CHROMOSOME CONDENSATION PROTEIN G (HCAP-G), MRNA (NM_022346); a gene of CDC28 PROTEIN KINASE 1 (CKS1), MRNA (NM_001826); a gene of PROTEIN REGULATOR OF CYTOKINESIS 1 (PRC1), MRNA (NM_003981); a gene of CELL DIVISION CYCLE 2, G1 TO S AND G2 TOM (CDC2), MRNA (NM_001786); a gene of CDC20 (CELL DIVISION CYCLE 20, S. CEREVISIAE, HOMOLOG) (CD) (NM_001255); a gene of LIKELY ORTHOLOG OF MATERNAL EMBRYONIC LEUCINE ZIPPER KINAS (NM_014791); a gene of MINICHROMOSOME MAINTENANCE DEFICIENT (S. CEREVISIAE) 7 (M) (NM_005916); a gene of CYCLINA2 (CCNA2), MRNA (NM_001237); a gene of THYMIDINE KINASE 1, SOLUBLE (TK1), MRNA (NM_003258); or a gene of cyclin-dependent kinase inhibitor 2A (melanoma, p16, inhibits CDK4) (CDKN2A), mRNA (NM_000077).

3. A gene marker for detecting a mesenchymal stem cell which is a gene of EGF-containing fibulin-like extracellular matrix protein 1 (NM_018894); a gene of Human insulin-like growth factor binding protein 5 (IGFBP5) mRNA (AU132011); a gene of Homo sapiens clone 24775 mRNA sequence (AA402981); proteoglycan 1, secretory granule (AV734015); a gene of insulin-like growth factor binding protein 5 (AA374325); a gene of solute carrier family 21 (organic anion transporter), member 3 (AF085224); a gene of transglutaminase 2 (C polypeptide, protein-glutamine-gamma-glutamyl transferase) (AL552373); a gene of coagulation factor II (thrombin) receptor (M62424); a gene of plasminogen activator, urokinase (NM_002658); a gene of tissue inhibitor of metalloproteinase 3 (Sorsby fundus dystrophy, pseudoinflammatory) (W96324); a gene of matrix Gla protein (BF668572); a gene of B-cell CLL/lymphoma 1 (Z23022); a gene of CD74 antigen (invariant polypeptide of major histocompatibility complex, class II antigen-associated) (BG333618); a gene of adducin 3 (gamma) (AL135243); a gene of solute carrier family 16 (monocarboxylic acid transporters), member 4 (NM_004696); a gene of protein S (alpha) (NM_000313); a gene of phosphatidylinositol (4,5) bisphosphate 5-phosphatase homolog; phosphatidylinositol polyphosphate 5-phosphatase type IV (AF187891); a gene of progesterone membrane binding protein (BE858855); a gene of brain-derived neurotrophic factor (X60201); or a gene of apolipoprotein E (BF967316).

4. A gene marker for detecting a mesenchymal stem cell which is a gene of HOMO SAPIENS WNT INHIBITORY FACTOR-1 (WIF-1), MRNA(NM_007191); a gene of HOMO SAPIENS HYPOTHETICAL PROTEIN FLJ11175 (FLJ11175), MRNA (NM_018349): a gene of HOMO SAPIENS RHO GDP DISSOCIATION INHIBITOR (GDI) BETA (ARHGDIB), MRNA (NM_001175); a gene of HOMO SAPIENS POTASSIUM INTERMEDIATE/SMALL CONDUCTANCE CALCIUM-ACTIVATE (NM_021614); a gene of HOMO SAPIENS NUCLEAR FACTOR (ERYTHROID-DERIVED 2)-LIKE 3 (NFE2L3), MRNA (NM_004289); a gene of HOMO SAPIENS GS3955 PROTEIN (GS3955), MRNA (NM_021643); a gene of HOMO SAPIENS CDNA 3' END SIMILAR TO SIMILAR TO GLYCOPROTEIN MUC18 (AA302605); a gene of HOMO SAPIENS HYPOTHETICAL PROTEIN FLJ21841 (FLJ21841), MRNA (NM_024609); a gene of HOMO SAPIENS ZINC FINGER PROTEIN 185 (LIM DOMAIN) (ZNF185), MRNA (NK_007150); a gene of SC1 = PUTATIVE TRANS-ACTING FACTOR INVOLVED IN CELL CYCLE CONTROL [HUMAN, MRNA, 24] (S53374); a gene of HOMO SAPIENS NADH DEHYDROGENASE (UBIQUINONE) 1 ALPHA SUBCOMPLEX, 8 (19K) (NM_014222); a gene of HOMO SAPIENS HOMOLOG OF YEAST MCM10; HYPOTHETICAL PROTEIN PRO2249 (PRO2) (NM_018518); a gene of HOMO SAPIENS COLLAGEN, TYPE VII, ALPHA 1 (EPIDERMOLYSIS BULLOSA, DYSTRO (NM_000094); a gene of HOMO SAPIENS AUTOCRINE MOTILITY FACTOR RECEPTOR (AMFR), MRNA (NM_001144); a gene of HOMO SAPIENS CLONE 24421 MRNA SEQUENCE /CDS = UNKNOWN /GB = AF070641 /GI = 3283914 /UG = (AF070641); a gene of HOMO SAPIENS MRNA; a gene of CDNA DKFZP586E1621 (FROM CLONE DKFZP586E1621) /CDS = UNKNOWN /GB (AL080235).

5. A gene marker for detecting a mesenchymal stem cell which is a gene of HOMO SAPIENS RESERVED (KCNK12), MRNA (NM_022055); a gene of HOMO SAPIENS UBIQUITIN-CONJUGATING ENZYME E2C (UBE2C), MRNA (NM_007019); a gene of HOMO SAPIENS CDNA FLJ10517 FIS, CLONE NT2RP2000812 /CDS = (61,918) /GB = AK001379 /G (AK001379); a gene of HOMO SAPIENS CARTILAGE LINKING PROTEIN 1 (CRTL1), MRNA (NM_001884); a gene of HOMO SAPIENS MRNA; CDNA DKFZP434F2322 (FROM CLONE DKFZP434F2322) /CDS = UNKNOWN /GB (AL133105); a gene of HOMO SAPIENS, CLONE MGC:9549 IMAGE:3857382, MRNA, COMPLETE CDS /CDS = (92,1285) /G (BC012453); a gene of HOMO SAPIENS HYPOTHETICAL PROTEIN DKFZP586J1119 (DKFZP586J1119), MRNA (NM_032270); a gene of HOMO SAPIENS GLIA MATURATION FACTOR, GAMMA (GMFG), MRNA (NM_004877); a gene of HOMO SAPIENS CDNA: FLJ23095 FIS, CLONE LNG07413 /CDS = UNKNOWN /GB = AK026748 /GI = 10 (AK026748); a gene of HOMO SAPIENS HYPOTHETICAL PROTEIN DKFZP762E1312 (DKFZP762E1312), MRNA (NM_018410); a gene of HOMO SAPIENS IROQUOIS-CLASS HOMEODOMAIN PROTEIN (IRX-2A), MRNA (NM_005853); or a gene of HOMO SAPIENS HYPOTHETICAL PROTEIN FLJ10540 (FLJ10540), MRNA (NM_018131).

6. A gene marker for detecting a mesenchymal stem cell which is a gene of HOMO SAPIENS MRNA; CDNA DKFZP434C1915 (FROM CLONE DKFZP434C1915); PARTIAL CDS /C (AL137698); a gene of HOMO SAPIENS MINICHROMOSOME MAINTENANCE DEFICIENT (S. CEREVISIAE) 7 (M) (NM_005916); a gene of HOMO SAPIENS DYNEIN, CYTOPLASMIC, INTERMEDIATE POLYPEPTIDE 1 (DNCI1), M (NM_004411); a gene of HUMAN MRNA FOR PROTEIN GENE PRODUCT (PGP) 9.5. (X04741); a gene of HOMO SAPIENS RAD51-INTERACTING PROTEIN (PIR51), MRNA (NM_006479); a gene of HOMO SAPIENS BACULOVIRAL IAP REPEAT-CONTAINING 5 (SURVIVIN) (BIRC5), MR (NM_001168); a gene of HOMO SAPIENS UDP-N-ACETYL-ALPHA-D-GALACTOSAMINE:POLYPEPTIDE N-ACETYLGAL (NM_004482); a gene of HOMO SAPIENS CYCLIN B1 (CCNB1), MRNA (NM_031966); a gene of HOMO SAPIENS FLAP STRUCTURE-SPECIFIC ENDONUCLEASE 1 (FEN1), MRNA (NM_004111); a gene of HOMO SAPIENS SERINE/THREONINE KINASE 15 (STK15), MRNA (NM_003600); a gene of HOMO SAPIENS MAD2 (MITOTIC ARREST DEFICIENT, YEAST, HOMOLOG)-LIKE 1 (MA) (NM_002358); a gene of HOMO SAPIENS NUCLEOLAR PROTEIN ANKT (ANKT), MRNA (NM_018454); a gene of HOMO SAPIENS HSPC150 PROTEIN SIMILAR TO UBIQUITIN-CONJUGATING ENZYME (H) (NM_014176); a gene of HOMO SAPIENS CYTOCHROME P450 RETINOID METABOLIZING PROTEIN (P450RAI-2), (NM_019885); a gene of HOMO SAPIENS NIMA (NEVER IN MITOSIS GENE A)-RELATED KINASE 2 (NEK2), MR (NM_002497); a gene of HOMO SAPIENS BONE MORPHOGENETIC PROTEIN 4 (BMP4), MRNA (NM_001202); a gene of HOMO SAPIENS CDNA FLJ10674 FIS, CLONE NT2RP2006436 /CDS = UNKNOWN /GB = AK001536 /GI (AK001536); or a gene of HOMO SAPIENS CENTROMERE PROTEIN A (17 KD) (CENPA), MRNA (NM_001809).

7. A gene marker for detecting a mesenchymal stem cell which is a gene of HOMO SAPIENS MRNA; CDNA DKFZP564P116 (FROM CLONE DKFZP564P116) /CDS = UNKNOWN /GB = A (AL049338); a gene of HOMO SAPIENS TTK PROTEIN KINASE (TTK), MRNA (NM_003318); a gene of HOMO SAPIENS KARYOPHERIN ALPHA 2 (RAG COHORT 1, IMPORTIN ALPHA 1) (KPNA (NM_002266); a gene of HOMO SAPIENS POLYMERASE (DNA DIRECTED), THETA (POLQ), MRNA (NM_006596); a gene of HOMO SAPIENS ETS VARIANT GENE 5 (ETS-RELATED MOLECULE) (ETV5), MRNA (NM_004454); a gene of HOMO SAPIENS TRANSFORMING, ACIDIC COILED-COIL CONTAINING PROTEIN 3 (TAC) (NM_006342); a gene of HOMO SAPIENS KINESIN-LIKE 1 (KNSL1), MRNA (NM_004523); a gene of HOMO SAPIENS CENTROMERE PROTEIN E (312 KD) (CENPE), MRNA (NM_001813); a gene of HOMO SAPIENS CDNA, 3' END /CLONE = IMAGE: 1651289 /CLONE_END = 3' /GB = AI12 (AI123815); a gene of HOMO SAPIENS DISTAL-LESS HOMEO BOX 5 (DLX5), MRNA (NM_005221); a gene of HOMO SAPIENS V-MYB AVIAN MYELOBLASTOSIS VIRAL ONCOGENE HOMOLOG-LIKE 2 (NM_002466); or a gene of HOMO SAPIENS SERUM DEPRIVATION RESPONSE (PHOSPHATIDYLSERINE-BINDING PRO (NM_004657).

8. A gene marker for detecting a mesenchymal stem cell which is a gene of HOMO SAPIENS HYPOTHETICAL PROTEIN FLJ10604 (FLJ10604), MRNA(NM_018154); ageneof HOMO SAPIENS RIBONUCLEASE HI, LARGE SUBUNIT (RNASEHI), MRNA (NM_006397); a gene of HOMO SAPIENS ANILLIN (DROSOPHILA SCRAPS HOMOLOG), ACTIN BINDING PROTEIN (NM_018685); a gene of HOMO SAPIENS H4 HISTONE FAMILY, MEMBER G (H4FG), MRNA (NM_003542); a gene of HOMO SAPIENS G PROTEIN-COUPLED RECEPTOR 37 (ENDOTHELIN RECEPTOR TYPE B-(NM_005302) HOMO SAPIENS UBIQUITIN CARRIER PROTEIN (E2-EPF), MRNA (NM_014501); a gene of HOMO SAPIENS, SIMILAR TO TUMOR DIFFERENTIALLY EXPRESSED 1, CLONE IMAGE:3639252, (BC007375); a gene of HOMO SAPIENS HYPOTHETICAL PROTEIN MGC2577 (MGC2577), MRNA (NM_031299); a gene of HOMO SAPIENS HYPOTHETICAL PROTEIN FLJ13912 (FLJ13912), MRNA (NM_022770); a gene of HOMO SAPIENS ANNEXIN A3 (ANXA3), MRNA (NM_005139); a gene of HOMO SAPIENS STATHMIN 1/ONCOPROTEIN 18 (STMN1), MRNA (NM_005563) HOMO SAPIENS, SIMILAR TO RIBOSOMAL PROTEINL39, CLONEMGC: 20168 IMAGE: 4555759, M (BC012328); or a gene of HOMO SAPIENS POLO (DROSOPHIA)-LIKE KINASE (PLK), MRNA (NM_005030).

9. A gene marker for detecting a mesenchymal stem cell which is a gene of HOMO SAPIENS PROTEASOME (PROSOME, MACROPAIN) 26S SUBUNIT, ATPASE, 5 (PS) (NM_002805); a gene of HOMO SAPIENS EUKARYOTIC TRANSLATION INITIATION FACTOR 4 GAMMA, 2 (EIF4G) (NM_001418); a gene of HOMO SAPIENS DIHYDROPYRIMIDINASE-LIKE 3 (DPYSL3), MRNA) (NM_001387); a gene of HOMO SAPIENS ADAPTOR-RELATED PROTEIN COMPLEX 1, SIGMA 2 SUBUNIT (AP1S2) (NM_003916); a gene of HOMO SAPIENS PLECTIN 1, INTERMEDIATE FILAMENT BINDING PROTEIN, 500 KD (P) (NM_000445); a gene of HOMO SAPIENS HETEROGENEOUS NUCLEAR RIBONUCLEOPROTEIN U (SCAFFOLD ATTAC (NM_031844); a gene of HOMO SAPIENS HYPOTHETICAL PROTEIN MGC5306 (MGC5306), MRNA (NM_024116); a gene of HOMO SAPIENS MHC CLASS I POLYPEPTIDE-RELATED SEQUENCE A (MICA), MRNA (NM_000247); a gene of HOMO SAPIENS PROTEIN ASSOCIATED WITH PRK1 (AWP1), MRNA (NM_019006); a gene of HOMO SAPIENS PROGESTERONE RECEPTOR MEMBRANE COMPONENT 2 (PGRMC2), MRNA (NM_006320); a gene of HOMO SAPIENS POLYGLUTAMINE BINDING PROTEIN 1 (PQBP1), MRNA (NM_005710); a gene of HOMO SAPIENS S-ADENOSYLMETHIONINE DECARBOXYLASE 1 (AMD1), MRNA (NM_001634); HOMO SAPIENS INTERFERON-INDUCED, HEPATITIS C-ASSOCIATED MICROTUBULAR AG (NM_006417); a gene of HOMO SAPIENS PROTEINKINASE, AMP-ACTIVATED, GAMMA 1 NON-CATALYTIC SUBUN (NM_002733); HOMO SAPIENS PROCOLLAGEN-PROLINE, 2-OXOGLUTARATE 4-DIOXYGENASE (PROLINE (NM_004199); a gene of HOMO SAPIENS ENDOTHELIAL DIFFERENTIATION, LYSOPHOSPHATIDIC ACID G-PROTE (NM_012152); or a gene of HOMO SAPIENS KIAA0127 GENE PRODUCT (KIAA0127), MRNA (NM_014755).

10. A probe for detecting a mesenchymal stem cell marker gene having a DNA sequence which hybridizes with the marker gene for detecting a mesenchymal stem cell according to any one of claims 1 to 9 under a stringent condition.

11. The probe for detecting a mesenchymal stem cell marker gene according to claim 10, which comprises whole or part of an antisense strand of the base sequence according to any one of claims 1 to 9.

12. A microarray or a DNA chip for detecting a mesenchymal stem cell marker gene, wherein at least one of the DNA according to claim 10 or 11 is immobilized.

13. The microarray or the DNA chip for detecting a mesenchymal stem cell marker gene according to claim 12, wherein a probe for detecting two or more genes in a group of genes having a base sequence shown in SEQ ID NO: 1, 2, 4, 6, 7, 9, 10, 11, 12, 14, 15, 17, 19 or 46 in the sequence listing and a group of the genes according to any one of claims 2 to 9 is immobilized.

14. A polypeptide marker for detecting a mesenchymal stem cell which is a polypeptide having an amino acid sequence shown in SEQ ID NO: 3, 5, 8, 13, 16 or 18 in the sequence listing.

15. An antibody which is induced by using the polypeptide according to claim 14 and which specifically binds to the polypeptide.

16. The antibody according to claim 15, which is a monoclonal antibody.

17. The antibody according to claim 15, which is a polyclonal antibody.

18. A method for distinguishing a mesenchymal stem cell wherein expression of the mesenchymal stem cell marker gene according to any one of claims 1 to 9 in a test cell is detected.

19. The method for distinguishing a mesenchymal stem cell according to claim 18, wherein the expression of the mesenchymal stem cell marker gene is detected by using Northern blotting.

20. The method for distinguishing a mesenchymal stem cell according to claim 18, wherein the expression of the mesenchymal stem cell marker gene is detected by using the probe for detecting a mesenchymal stem cell marker gene according to claim 10 or 11.

21. The method for distinguishing a mesenchymal stem cell according to claim 18, wherein the expression of the mesenchymal stem cell marker gene is detected by using the microarray or the DNA chip for detecting a mesenchymal stem cell marker gene according to claim 12 or 13.

22. A method for distinguishing a mesenchymal stem cell, wherein the detection of the expression of the mesenchymal stem cell marker gene according to any one of claims 18 to 21 comprises use of quantitative or semiquantitative PCR.

23. The method for distinguishing a mesenchymal stem cell according to claim 22, wherein the use of quantitative or semiquantitative PCR is RT-PCR method.

24. An RT-PCR primer for amplifying a mesenchymal stem cell marker gene which comprises a sense primer having a base sequence shown in SEQ ID NO: 20 in the sequence listing, and an antisense primer having a base sequence shown in SEQ ID NO: 21 in the sequence listing; a sense primer having a base sequence shown in SEQ ID NO: 22 in the sequence listing, and an antisense primer having a base sequence shown in SEQ ID NO: 23 in the sequence listing; a sense primer having a base sequence shown in SEQ ID NO: 24 in the sequence listing, and an antisense primer having a base sequence shown in SEQ ID NO: 25 in the sequence listing; a sense primer having a base sequence shown in SEQ ID NO: 26 in the sequence listing, and an antisense primer having a base sequence shown in SEQ ID NO: 27 in the sequence listing; a sense primer having a base sequence shown in SEQ ID NO: 28 in the sequence listing, and an antisense primer having a base sequence shown in SEQ ID NO: 29 in the sequence listing; a sense primer having a base sequence shown in SEQ ID NO: 30 in the sequence listing, and an antisense primer having a base sequence shown in SEQ ID NO: 31 in the sequence listing; a sense primer having a base sequence shown in SEQ ID NO: 32 in the sequence listing, and an antisense primer having a base sequence shown in SEQ ID NO: 33 in the sequence listing; a sense primer having a base sequence shown in SEQ ID NO: 34 in the sequence listing, and an antisense primer having a base sequence shown in SEQ ID NO: 35 in the sequence listing; a sense primer having a base sequence shown in SEQ ID NO: 36 in the sequence listing, and an antisense primer having a base sequence shown in SEQ ID NO: 37 in the sequence listing; a sense primer having a base sequence shown in SEQ ID NO: 38 in the sequence listing, and an antisense primer having a base sequence shown in SEQ ID NO: 39 in the sequence listing; a sense primer having a base sequence shown in SEQ ID NO: 40 in the sequence listing, and an antisense primer having a base sequence shown in SEQ ID NO: 41 in the sequence listing; a sense primer having a base sequence shown in SEQ ID NO: 42 in the sequence listing, and an antisense primer having a base sequence shown in SEQ ID NO: 43 in the sequence listing; or a sense primerhaving a base sequence shown in SEQ ID NO: 44 in the sequence listing, and an antisense primer having a base sequence shown in SEQ ID NO: 45 in the sequence listing.

25. A real time PCR primer for amplifyirig a mesenchymal stem cell marker gene which comprises a sense primer having a base sequence shown in SEQ ID NO: 47 in the sequence listing, and an antisense primer having a base sequence shown in SEQ ID NO: 48 in the sequence listing; a sense primer having a base sequence shown in SEQ ID NO: 49 in the sequence listing, and an antisense primer having a base sequence shown in SEQ ID NO: 50 in the sequence listing; a sense primer having a base sequence shown in SEQ ID NO: 51 in the sequence listing, and an antisense primer having a base sequence shown in SEQ ID NO: 52 in the sequence listing; a sense primer having a base sequence shown in SEQ ID NO: 53 in the sequence listing, and an antisense primer having a base sequence shown in SEQ ID NO: 54 in the sequence listing; a sense primer having a base sequence shown in SEQ ID NO: 55 in the sequence listing, and an antisense primer having a base sequence shown in SEQ ID NO: 56 in the sequence listing; a sense primer having a base sequence shown in SEQ ID NO: 57 in the sequence listing, and an antisense primer having a base sequence shown in SEQ ID NO: 58 in the sequence listing; a sense primer having a base sequence shown in SEQ ID NO: 59 in the sequence listing, and an antisense primer having a base sequence shown in SEQ ID NO: 60 in the sequence listing; a sense primer having a base sequence shown in SEQ ID NO: 61 in the sequence listing, and an antisense primer having a base sequence shown in SEQ ID NO: 62 in the sequence listing; a sense primer having a base sequence shown in SEQ ID NO: 63 in the sequence listing, and an antisense primer having a base sequence shown in SEQ ID NO: 64 in the sequence listing; a sense primer having a base sequence shown in SEQ ID NO: 65 in the sequence listing, and an antisense primer having a base sequence shown in SEQ ID NO: 66 in the sequence listing; a sense primer having a base sequence shown in SEQ ID NO: 67 in the sequence listing, and an antisense primer having a base sequence shown in SEQ ID NO: 68 in the sequence listing; or a sense primer having a base sequence shown in SEQ ID NO: 69 in the sequence listing, and an antisense primer having a base sequence shown in SEQ ID NO: 70 in the sequence listing.

26. An RT-PCR primer for amplifying a mesenchymal stem cell marker gene which comprises a sense primer having a base sequence shown in SEQ ID NO: 71 in the sequence listing, and an antisense primer having a base sequence shown in SEQ ID NO: 72 in the sequence listing; a sense primer having a base sequence shown in SEQ ID NO: 73 in the sequence listing, and an antisense primer having a base sequence shown in SEQ ID NO: 74 in the sequence listing; a sense primer having a base sequence shown in SEQ ID NO: 75 in the sequence listing, and an antisense primer having a base sequence shown in SEQ ID NO: 76 in the sequence listing; a sense primer having a base sequence shown in SEQ ID NO: 77 in the sequence listing, and an antisense primer having a base sequence shown in SEQ ID NO: 78 in the sequence listing; a sense primer having a base sequence shown in SEQ ID NO: 79 in the sequence listing, and an antisense primer having a base sequence shown in SEQ ID NO: 80 in the sequence listing; a sense primer having a base sequence shown in SEQ ID NO: 81 in the sequence listing, and an antisense primer having a base sequence shown in SEQ ID NO: 82 in the sequence listing; a sense primer having a base sequence shown in SEQ ID NO: 83 in the sequence listing, and an antisense primer having a base sequence shown in SEQ ID NO: 84 in the sequence listing; a sense primer having a base sequence shown in SEQ ID NO: 85 in the sequence listing, and an antisense primer having a base sequence shown in SEQ ID NO: 86 in the sequence listing; a sense primer having a base sequence shown in SEQ ID NO: 87 in the sequence listing, and an antisense primer having a base sequence shown in SEQ ID NO: 88 in the sequence listing; a sense primer having a base sequence shown in SEQ ID NO: 89 in the sequence listing, and an antisense primer having a base sequence shown in SEQ ID NO: 90 in the sequence listing; a sense primer having a base sequence shown in SEQ ID NO: 91 in the sequence listing, and an antisense primer having a base sequence shown in SEQ ID NO: 92 in the sequence listing; a sense primer having a base sequence shown in SEQ ID NO: 93 in the sequence listing, and an antisense primer having a base sequence shown in SEQ ID NO: 94 in the sequence listing; a sense primer having a base sequence shown in SEQ ID NO: 95 in the sequence listing, and an antisense primer having a base sequence shown in SEQ ID NO: 96 in the sequence listing; a sense primer having a base sequence shown in SEQ ID NO: 97 in the sequence listing, and an antisense primer having a base sequence shown in SEQ ID NO: 98 in the sequence listing; a sense primer having a base sequence shown in SEQ ID NO: 99 in the sequence listing, and an antisense primer having a base sequence shown in SEQ ID NO: 100 in the sequence listing; a sense primer having a base sequence shown in SEQ ID NO: 101 in the sequence listing, and an antisense primer having a base sequence shown in SEQ ID NO: 102 in the sequence listing; a sense primer having a base sequence shown in SEQ ID NO: 103 in the sequence listing, and an antisense primer having a base sequence shown in SEQ ID NO: 104 in the sequence listing; a sense primer having a base sequence shown in SEQ ID NO: 105 in the sequence listing, and an antisense primer having a base sequence shown in SEQ ID NO: 106 in the sequence listing; a sense primer having a base sequence shown in SEQ ID NO: 107 in the sequence listing, and an antisense primer having a base sequence shown in SEQ ID NO: 108 in the sequence listing; a sense primer having a base sequence shown in SEQ ID NO: 109 in the sequence listing, and an antisense primer having a base sequence shown in SEQ ID NO: 110 in the sequence listing; a sense primer having a base sequence shown in SEQ ID NO: 111 in the sequence listing, and an antisense primer having a base sequence shown in SEQ ID NO: 112 in the sequence listing; a sense primer having a base sequence shown in SEQ ID NO: 113 in the sequence listing, and an antisense primer having a base sequence shown in SEQ ID NO: 114 in the sequence listing; or a sense primer having a base sequence shown in SEQ ID NO: 115 in the sequence listing, and an antisense primer having a base sequence shown in SEQ ID NO: 116 in the sequence listing.

27. The method for distinguishing a mesenchymal stem cell according to claim 18, wherein a gene in a test cell is amplified by using at least one pair of primers comprising the sense primer and the antisense primer according to any one of claims 24 to 26, and the amplified gene is detected by using the probe for detecting a mesenchymal stem cell marker gene according to claim 10 or 11.

28. A method for distinguishing a mesenchymal stem cell, wherein expression of a mesenchymal stem cell marker polypeptide in a test cell is detected by using the antibody according to any one of claims 15 to 17.

29. A method for distinguishing a mesenchymal stem cell, wherein expression of one or more genes in a group of mesenchymal stem cell marker genes comprising SEQ ID NOs: 1, 11 and 19 in the sequence listing, and expression of one or more genes in a group of mesenchymal stem cell marker genes comprising SEQ ID NOs: 2, 4, 6, 7, 9, 10, 12, 14, 15 and 17 in the sequence listing, in a test cell, are evaluated in combination.

30. A method for distinguishing and separating a mesenchymal stem cell, wherein expression of a marker gene for detecting a mesenchymal stem cell and/or a marker polypeptide for detecting a mesenchymal stem cell in a test cell is detected by using the probe for detecting a mesenchymal stem cell marker gene according to claim 10 or 11 and/or the antibody according to any one of claims 15 to 17, and the distinguished mesenchymal stem cell is separated.

31. The method for distinguishing and separating a mesenchymal stem cell according to claim 30, wherein a mesenchymal stem cell in a test cell is labeled by a fluorescent antibody method using the antibody according to any one of claims 15 to 17, and the labeled mesenchymal stem cell is separated.

32. A kit for distinguishing a mesenchymal stem cell which comprises at least one of the probe for detecting a mesenchymal stem cell marker gene according to claim 10 or 11, the microarray or the DNA chip for detecting a mesenchymal stem cell marker gene according to claim 12 or 13 in which the probe is immobilized, and the antibody according to any one of claims 15 to 17.
